# EUROPEAN PATENT SPECIFICATION

(11) **EP 4 627 113 B1**
(45) Date of publication and mention of the grant of the patent: **12.08.2026**
(21) Application number: 23836688.4
(22) Date of filing: 30.11.2023
(51) Int. Cl.: C12Q 1/6827

(54) **CHEMOENZYMATIC CORRECTION OF FALSE POSITIVE URACIL TRANSFORMATIONS**
CHEMOENZYMATISCHE KORREKTUR VON FALSCH-POSITIVEN URACIL-TRANSFORMATIONEN
CORRECTION CHIMIO-ENZYMATIQUE DE TRANSFORMATIONS D'URACILE FAUX POSITIVES

(30) Priority: 30.11.2022 US 202263428797 P; 30.11.2022 US 202263428812 P
(43) Date of publication of application: 08.10.2025
(73) Proprietor: Illumina, Inc., San Diego, CA 92122 (US)
(72) Inventor: BUSBY, Kayla, San Diego, California 92122 (US); KARADEEMA, Rebekah, San Diego, California 92122 (US); GROSS, Stephen, San Diego, California 92122 (US); CRESSINA, Elena, Cambridge Cambridgeshire CB21 6DF (GB)
(74) Representative: Williams, Andrea
(86) International application number: PCT/US2023/081796
(87) International publication number: WO 2024/118903

(56) References cited:
- WO-A2-2013/138644
- WANG YAFEN ET AL: "Base-Resolution Analysis of Deoxyuridine at the Genome Scale Based on the Artificial Incorporation Modified Nucleobase", ACS CENTRAL SCIENCE, vol. 7, no. 6, 23 June 2021 (2021-06-23), pages 973 - 979, XP093121772, ISSN: 2374-7943, Retrieved from the Internet <URL:https://pubs.acs.org/doi/pdf/10.1021/acscentsci.0c01504> DOI: 10.1021/acscentsci.0c01504
- LIU ZHENG J ET AL: "Sequencing abasic sites in DNA at single-nucleotide resolution", NATURE CHEMISTRY, NATURE PUBLISHING GROUP UK, LONDON, vol. 11, no. 7, 17 June 2019 (2019-06-17), pages 629 - 637, XP036815924, ISSN: 1755-4330, [retrieved on 20190617], DOI: 10.1038/S41557-019-0279-9
- A. W. BRIGGS ET AL: "Removal of deaminated cytosines and detection of in vivo methylation in ancient DNA", NUCLEIC ACIDS RESEARCH, vol. 38, no. 6, 1 April 2010 (2010-04-01), pages e87 - e87, XP055204567, ISSN: 0305-1048, DOI: 10.1093/nar/gkp1163
- SHU XIAOTING ET AL: "Genome-wide mapping reveals that deoxyuridine is enriched in the human centromeric DNA", NATURE CHEMICAL BIOLOGY, NATURE PUBLISHING GROUP US, NEW YORK, vol. 14, no. 7, 21 May 2018 (2018-05-21), pages 680 - 687, XP036525007, ISSN: 1552-4450, [retrieved on 20180521], DOI: 10.1038/S41589-018-0065-9

## Description

### CONTINUING APPLICATION DATA

This application claims the benefit of U.S. Provisional Application Serial No. 63/428,797, filed November 30, 2022, and U.S. Provisional Application No. 63/428,812, filed November 30, 2022.

### FIELD OF INVENTION

Embodiments of the present disclosure relate to the prevention of false positive detection of 5-methylcytosine (5mC) and/or 5-hydroxymethylcytosine (5hmC) due to the deamination of unmethylated cytosines in assays using cytosine deaminases to selectively deaminate methylated cytosines. In particular, embodiments of the methods, compositions, and kits provided herein relate to the chemoenzymatic correction of false positive uracil transformations back to cytosines.

### BACKGROUND

Modified DNA cytosines, including 5-methylcytosine (5mC), are a well-studied epigenetic modification that play fundamental roles in human development and disease. Its genome-wide distribution differs between tissue types, and between healthy and diseased states. In recent years, 5mC has also gained prominence as a tool for clinical diagnostics. For example, its distribution in cell-free DNA (cfDNA) obtained from a liquid biopsy can be used for the tissue-specific prediction of early-stage cancer. As a result, there has been an intense focus on developing methods for mapping 5mC at single base resolution, with minimal loss of sample DNA quantity, quality, and complexity.

5mC bases treated with a cytosine deaminase result in thymine bases, providing a signal for assessing sequence-specific methylation state of cytosines when sequenced. APOBEC3A is a cytidine deaminase that recognizes single-stranded DNA and catalyzes the deamination of cytosine (C) to uracil (U), 5-methylcytosine (5mC) to thymine (T), and 5-hydroxymethylcytosine to 5-hydroxymethyluracil. Protein engineering of APOBEC3A has resulted in mutant APOBEC proteins with selectivity towards deamination of 5mC with reduced activity towards deamination of C, however residual activity for deamination of C remains. This undesirable deamination of unmethylated cytosines results in the false positive detection of 5mC (and 5hmC), with uracil bases being read as thymine bases in the assay.

Wang Yafen et al.: "Base-Resolution Analysis of Deoxyuridine at the Genome Scale Based on the Artificial Incorporation Modified Nucleobase", ACS CENTRAL SCIENCE, vol. 7, no. 6, 23 June 2021 (2021-06-23), pages 973-979, describes the AI-seq (artificial incorporation modified nucleobase for sequencing), a method for "base substitution" which is capable of profiling uracil at single-nucleotide resolution and showing its centromeric enrichment. In particular, an abasic site is generated using UDG followed by addition of a cytosine analogue.

WO 2013/138644 discloses "altered" variants of cytidine deaminase (APOBEC3A) for determining DNA methylation status, and in particular discriminating between 5mC and 5hmC deaminations and the deamination of cytosines 'wherein 5-mC is converted to a thymine (T) and C is converted to a uracil'.

### SUMMARY OF THE INVENTION

In one aspect, this disclosure describes a method of preventing false positive detection of 5-methylcytosine (5mC) and/or 5-hydroxymethylcytosine (5hmC) due to deamination of unmethylated cytosines in an assay using a cytosine deaminase to selectively deaminate methylated cytosines, the method including providing a sample comprising single stranded DNA library fragments in which a cytosine deaminase has deaminated methylated cytosines, contacting the sample with an uracil DNA glycosylase (UDG) to deglycosylate uracil residues to form abasic sites having a hemiacetal formation within the single stranded DNA library fragments, and contacting the sample with a reactive cytosine nucleobase analog to install a cytosine at abasic sites thru a noncanonical linkage resulting in double stranded DNA corrected library fragments. In some aspects, the method further includes subjecting the sample to polymerase chain reaction (PCR) amplification. In some aspects, the DNA library fragments are about 100bp to about 200bp in length. In some aspects, the DNA library fragments comprise 5' and/or 3' adapter sequences. In some aspects, the method further includes sequencing the corrected library fragments.

In another aspect, not encompassed by the wording of the claims this disclosure describes a method of replacing uracil residues (deaminated cytosine residues) with cytosine residues, the method including providing a sample comprising single stranded DNA fragments, contacting the sample with an uracil DNA glycosylase (UDG) to deglycosylate uracil residues to form abasic sites having a hemiacetal formation within the single stranded DNA fragments, and contacting the sample with a reactive cytosine nucleobase analog to install a cytosine at the abasic sites thru a noncanonical linkage resulting in double stranded DNA fragments. In some aspects, not encompassed by the wording of the claims the method further includes subjecting the sample to polymerase chain reaction (PCR) amplification. In some aspects, not encompassed by the wording of the claims the sample includes single stranded DNA fragments, includes long fragments of at least about 5 kbp to at least about 6 kbp, is obtained from formalin-fixed paraffin embedded (FFPE) tissue, is obtained from ancient DNA sample, and/or is obtained from a forensic DNA sample.

With a method as described herein, the cytosine deaminase comprises an altered cytosine deaminase.

In some aspects, the altered cytosine deaminase is a member of the AID subfamily, the APOBEC1 subfamily, the APOBEC2 subfamily, the APOBEC3A subfamily, the APOBEC3B subfamily, the APOBEC3C subfamily, the APOBEC3D subfamily, the APOBEC3F subfamily, the APOBEC3G subfamily, the APOBEC3G subfamily, the APOBEC3H subfamily, or the APOBEC4 subfamily, or an alteration thereof. In some aspects, the altered cytosine deaminase comprises an altered APOBEC3A.

In some aspects, the altered cytidine deaminase comprises an amino acid substitution mutation at a position functionally equivalent to (Tyr/Phe)130 in a wild-type APOBEC3A protein and/or an amino acid substitution mutation at a position functionally equivalent to Tyr132 in a wild-type APOBEC3A protein. In some aspects, the altered cytidine deaminase comprises amino acid substitution mutations at positions functionally equivalent to (Tyr/Phe)130 and Tyr132 in a wild-type APOBEC3A protein.

In some aspects, the substitution mutation at the position functionally equivalent to Tyr130 comprises a mutation to alanine, glycine, phenylalanine, histidine, glutamine, methionine, asparagine, lysine, valine, aspartic acid, glutamic acid, serine, cysteine, proline, arginine, or threonine.

In some aspects, the substitution mutation at the position functionally equivalent to Tyr130 comprises a mutation to Ala, Val, or Trp.

In some aspects, the substitution mutation at the position functionally equivalent to Tyr132 comprises a mutation to His, Arg, Gln, or Lys.

In some aspects, the altered cytidine deaminase comprises an amino acid substitution mutation at a position functionally equivalent to (Tyr/Phe)130 in a wild-type APOBEC3A protein, wherein the substitution mutation is (Tyr/Phe)130Trp.

In some aspects, the (Tyr/Phe)130 is Tyr130, and the wild-type APOBEC3A protein is SEQ ID NO: 12.

In some aspects, the altered cytidine deaminase converts 5-methyl cytosine (5mC) to thymidine (T) by deamination at a greater rate than conversion of cytosine (C) to uracil (U) by deamination. In some aspects, the rate is at least 100-fold greater.

In some aspects, the altered cytidine deaminase converts cytosine (C) to uracil (U) by deamination and 5-methyl cytosine (5mC) to thymidine (T) by deamination at a greater rate than conversion of 5-hydroxymethyl cytosine (5hmC) to 5-hydroxymethyl uracil (5hmU) by deamination. In some aspects, conversion of 5hmC to 5hmU by deamination is undetectable.

In some aspects, the altered cytidine deaminase comprises a ZDD motif H- [P/A/V]-EX[23-28]-P-C-X[2-4]-C (SEQ ID NO: 1).

In some aspects, the altered cytidine deaminase is a member of the APOBEC3A subfamily and comprises a ZDD motif HXEX24SW(S/T)PCX[2-4]CX6FX8LX5R(L/I)YX[8-11]LX2LX[10]M (SEQ ID NO: 2), wherein the amino acid substitution mutation at the position functionally equivalent to (Tyr/Phe)130 of the wild-type APOBEC3A protein is the Tyr (Y) amino acid of the ZDD motif.

In some aspects, the altered cytidine deaminase is a member of the APOBEC3A subfamily and comprises X[16-26]-GRXXTXLCYXV-X15-GXXXN-X12-HAEXXF-X14-YXXTWXXSWSPC- X[2-4]-CA-X5-FL-X7-LXIXXXR(L/I)Y-X8-GLXXLXXXG-X5-M-X4-FXXCWXXFV-X6-FXPW-X13-LXXI-X[2-6] (SEQ ID NO: 3).

In some aspects, the altered cytidine deaminase is a member of the APOBEC3A family and comprises SEQ ID NO: 5, SEQ ID NO: 6, SEQ ID NO: 7, SEQ ID NO: 8, SEQ ID NO: 9, SEQ ID NO: 10, or SEQ ID NO: 11.

In some aspect of the methods described herein, the reactive cytosine nucleobase analog includes a hydroxylamine-cytosine derivative, a hydrazine-cytosine derivative, or a hydrazide-cytosine derivative.

In some aspects of the methods described herein, amplification includes a standard PCR polymerase or a U-intolerant polymerase.

In another aspect, this disclosure describes a kit including a cytosine deaminase, an uracil DNA glycosylase (UDG), and a reactive cytosine nucleobase analog. In some aspects, the cytosine deaminase is an altered APOBEC.

In one aspect not encompassed by the wording of the claims, this disclosure describes a method of preventing false positive detection of 5-methylcytosine (5mC) and/or 5-hydroxymethylcytosine (5hmC) due to deamination of unmethylated cytosines in an assay using a cytosine deaminase to selectively deaminate methylated cytosines, the method including:
providing a sample including first strand single stranded DNA fragments in which a cytosine deaminase has deaminated methylated cytosines, wherein the first strand single stranded DNA fragments include 5' end and 3' end library adapters;
contacting the sample including first strand single stranded DNA fragments with a polymerase, dNTPs, and a primers complementary to the 3' end library adapter, wherein the primer complementary to the 3' end library adapter includes a 5' phosphate group, under conditions to provide for second strand synthesis resulting in double stranded DNA fragments each including the first strand and a complementary second strand, wherein the resulting complementary second strand includes a 5' phosphate group;
contacting the sample including double stranded DNA fragments with an uracil DNA glycosylase (UDG), wherein the UDG removes uracil residues from the first strand, resulting in double stranded DNA fragments with uracils removed;
contacting the sample including double stranded DNA fragments with uracils removed with an endonuclease, wherein the endonuclease creates single nucleotide gaps at the sites of the removed uracil residues, resulting in double stranded DNA fragments including single nucleotide gaps at the sites of the removed uracil residues;
contacting the sample including double stranded DNA fragments including single nucleotide gaps at the sites of the removed uracil residues with an exonuclease-deficient polymerase, a nucleotide selected from dCTP, dITP, or other universal base, and a DNA ligase to insert cytosines residues at the single nucleotide gaps at the sites of the removed uracil residues in the first strand, resulting in repaired double stranded DNA fragments; and
contacting the sample including repaired double stranded DNA fragments with a lambda exonuclease to selectively digest the complementary second strand including a 5' phosphate group, resulting in first strand single stranded repaired library fragments. In some aspects, the method further includes subjecting the sample including the first strand single stranded repaired library fragments to polymerase chain reaction (PCR) amplification resulting in double stranded DNA corrected library fragments.

In another aspect not encompassed by the wording of the claims, this disclosure describes a method of preventing false positive detection of 5-methylcytosine (5mC) and/or 5-hydroxymethylcytosine (5hmC) due to deamination of unmethylated cytosines in an assay using a cytosine deaminase to selectively deaminate methylated cytosines, the method including:
providing a sample including first strand single stranded DNA library fragments in which a cytosine deaminase has deaminated methylated cytosines, wherein the first strand single stranded DNA library fragments include 5' end and 3' end library adapters;
contacting the sample including first strand single stranded DNA library fragments with a polymerase, dNTPs, a ligase, and primers complementary to the 5' end and 3' end library adapters under conditions to provide for second strand synthesis resulting in double stranded DNA fragments each including the first strand and a complementary second strand;
wherein the primers complementary to the 5' and 3' end library adapters include 8-oxoguanine and/or inosine residues;
wherein the resultant complementary second strands includes 5' and 3' end library adapters including 8-oxoguanine and/or inosine residues; and
wherein the ligase ligates the primer complementary to the 5' end library adapter to the complementary second strand;
contacting the sample including double stranded DNA fragments with an uracil DNA glycosylase (UDG), wherein the UDG removes uracil residues from the first strand, resulting in double stranded DNA fragments with uracils removed;
contacting the sample including double stranded DNA fragments with uracils removed with an endonuclease, wherein the endonuclease creates single nucleotide gaps at the sites of the removed uracil residues, resulting in double stranded DNA fragments including single nucleotide gaps at the sites of the removed uracil residues;
contacting the sample including double stranded DNA fragments including single nucleotide gaps at the sites of removed uracil residues with an exo-nuclease deficient polymerase, a nucleotide selected from dCTP, dITP, or other universal base, and a DNA ligase to insert a cytosine residue at the single nucleotide gaps at the sites of the removed uracil sites in the first strand, resulting in repaired double stranded DNA fragments; and
contacting the sample repaired double stranded DNA fragments with formamidopyrimidine glycosylase (FPG), 8-oxoguanine DNA glycosylase (OGG) and/or Endonuclease V (Endo V) to degrade the 5' end and 3' end library adapters including 8-oxoguanine and/or inosine residues in the complementary second strand, resulting in repaired double stranded DNA fragments including first strand repaired library fragments including adapters and complementary second strands lacking adapters. In further aspects, the method further includes subjecting the sample including repaired double stranded DNA fragments to polymerase chain reaction (PCR) amplification resulting in double stranded DNA corrected library fragments, wherein only the first strand repaired library fragments are amplifiable during PCR.

In another aspect not encompassed within the wording of the claims, this disclosure describes a method of replacing uracil residues with cytosine residues, the method including:
providing a sample including first strand single stranded DNA fragments, wherein the first strand single stranded DNA fragments include 5' end and 3' end library adapters;
contacting the sample including first strand single stranded DNA fragments with a polymerase, dNTPs, and a primers complementary to the 3' end library adapter, wherein the primer complementary to the 3' end library adapter includes a 5' phosphate group, under conditions to provide for second strand synthesis resulting in double stranded DNA fragments each including the first strand and a complementary second strand, wherein the resulting complementary second strand includes a 5' phosphate group;
contacting the sample including double stranded DNA fragments with an uracil DNA glycosylase (UDG), wherein the UDG removes uracil residues from the first strand, resulting in double stranded DNA fragments with uracil residues removed;
contacting the sample including double stranded DNA fragments with uracil residues removed with an endonuclease, wherein the endonuclease creates single nucleotide gaps at the sites of the removed uracil residues, resulting in double stranded DNA fragments including single nucleotide gaps at the sites of the removed uracil residues;
contacting the sample including double stranded DNA fragments including single nucleotide gaps at the sites of the removed uracil residues with an exonuclease-deficient polymerase, a nucleotide selected from dCTP, dITP, or other universal base, and a DNA ligase to insert cytosines residues at the single nucleotide gaps at the sites of the removed uracil residues in the first strand, resulting in repaired double stranded DNA fragments; and
contacting the sample including repaired double stranded DNA fragments with a lambda exonuclease to selectively digest the complementary second strand including a 5' phosphate group, resulting in repaired first strand single stranded DNA fragments. In some aspects, the method further includes subjecting the sample including repaired first strand single stranded DNA fragments to polymerase chain reaction (PCR) amplification resulting in corrected double stranded DNA fragments. In some aspects, the sample includes long fragments of at least about 5 kbp to at least about 6 kbp; is obtained from formalin-fixed paraffin embedded (FFPE) tissue; is obtained from ancient DNA sample; and/or is obtained from a forensic DNA sample.

In another aspect not encompassed by the wording of the claims, this disclosure describes a method of replacing uracil residues with cytosine residues, the method including:
providing a sample including first strand single stranded DNA fragments, wherein the first strand single stranded DNA fragments include 5' end and 3' end library adapters;
contacting the sample including first strand single stranded DNA library fragments with a polymerase, dNTPs, a ligase, and primers complementary to the 5' end and 3' end library adapters under conditions to provide for second strand synthesis resulting in double stranded DNA fragments each including the first strand and a complementary second strand;
wherein the primers complementary to the 5' and 3' end library adapters includes 8-oxoguanine and/or inosine residues;
wherein the resultant complementary second strands include 5' and 3' end library adapters including 8-oxoguanine and/or inosine residues; and
wherein the ligase ligates the primer complementary to the 5' end library adapter to the complementary second strand;
contacting the sample including double stranded DNA fragments with an uracil DNA glycosylase (UDG), wherein the UDG removes uracil residues from the first strand, resulting in double stranded DNA fragments with uracil residues removed;
contacting the sample including double stranded DNA fragments with uracil residues removed with an endonuclease, wherein the endonuclease creates single nucleotide gaps at the sites of the removed uracil residues, resulting in double stranded DNA fragments including single nucleotide gaps at the sites of the removed uracil residues;
contacting the sample including double stranded DNA fragments including single nucleotide gaps at the sites of the removed uracil residues with an exonuclease-deficient polymerase, a nucleotide selected from dCTP, dITP, or other universal base, and a DNA ligase to insert cytosines residues at the single nucleotide gaps at the sites of the removed uracil residues in the first strand, resulting in repaired double stranded DNA fragments; and
contacting the sample including repaired double stranded DNA fragments with formamidopyrimidine glycosylase (FPG), 8-oxoguanine DNA glycosylase (OGG) and/or Endonuclease V (Endo V) to degrade the 5' end and 3' end library adapters including 8-oxoguanine and/or inosine residues in the complementary second strand, resulting in repaired double stranded DNA fragments including first strand repaired library fragments including adapters and complementary second strands lacking adapters. In some aspects, the method further includes subjecting the sample including repaired first strand single stranded DNA fragments to polymerase chain reaction (PCR) amplification resulting in corrected double stranded DNA fragments. In some aspects, the sample includes long fragments of at least about 5 kbp to at least about 6 kbp; is obtained from formalin-fixed paraffin embedded (FFPE) tissue; is obtained from ancient DNA sample; and/or is obtained from a forensic DNA sample.

In some aspects of the methods described herein not encompassed by the wording of the claims, contacting the sample with the uracil DNA glycosylase (UDG), and contacting the sample with the endonuclease are simultaneous.

In some aspects of the methods described herein not encompassed by the wording of the claims, the endonuclease includes endonuclease IV (Endo IV) and/or apurinic/pyrimidinic Endonuclease IV (APE1).

In some aspects of the methods described herein not encompassed by the wording of the claims, the exonuclease-deficient polymerase includes Klenow exo- DNA polymerase, T7 DNA polymerase, T4 DNA polymerase, and/or *Sulfolobus* DNA polymerases IV.

In some aspects of the methods described herein not encompassed by the wording of the claims,the ligase includes T4 ligase.

In some aspects of the methods described herein not encompassed by the wording of the claims, the polymerase is an uracil-intolerant polymerase.

In some aspects of the methods described herein not encompassed by the wording of the claims,, the method further includes processing the double stranded DNA corrected library fragments to produce a sequencing library. In some aspects, the method further includes sequencing the sequencing library.

In another aspect not encompassed by the wording of the claims, this disclosure describes a kit including one or more of a cytosine deaminase; primers including a 5' phosphate group; a polymerase; dNTPs; an uracil DNA glycosylase (UDG); an endonuclease; a ligase; dCTP (or dITP or other universal base); and/or a lambda exonuclease. In some aspects, the cytosine deaminase is an altered APOBEC

In another aspect not encompassed by the wording of the claims,, this disclosure describes a kit including one or more of a cytosine deaminase; primers including 8-oxoguanine and/or inosine residues; a polymerase; dNTPs; an uracil DNA glycosylase (UDG); an endonuclease; a ligase; dCTP (or dITP or other universal base); formamidopyrimidine glycosylase (FPG); 8-oxoguanine DNA glycosylase (OGG); and/or Endonuclease V (Endo V). In some aspects, the cytosine deaminase is an altered APOBEC.

### Definitions

Terms used herein will be understood to take on their ordinary meaning in the relevant art unless specified otherwise. Several terms used herein and their meanings are set forth below.

As used herein, the term "nucleic acid" is intended to be consistent with its use in the art and includes naturally occurring nucleic acids or functional analogs thereof. Particularly useful functional analogs are capable of hybridizing to a nucleic acid in a sequence specific fashion or capable of being used as a template for replication of a particular nucleotide sequence. Naturally occurring nucleic acids generally have a backbone containing phosphodiester bonds. An analog structure can have an alternate backbone linkage including any of a variety of those known in the art. Naturally occurring nucleic acids generally have a deoxyribose sugar (for example, found in deoxyribonucleic acid (DNA)) or a ribose sugar (for example, found in ribonucleic acid (RNA)). A nucleic acid can contain any of a variety of analogs of these sugar moieties that are known in the art. A nucleic acid can include native or non-native bases. In this regard, a native deoxyribonucleic acid can have one or more bases selected from the group consisting of adenine, thymine, cytosine, or guanine and a ribonucleic acid can have one or more bases selected from the group consisting of uracil, adenine, cytosine, or guanine. Useful non-native bases that can be included in a nucleic acid are known in the art. The term "template" and "target," when used in reference to a nucleic acid, is intended as a semantic identifier for the nucleic acid in the context of a method or composition set forth herein and does not necessarily limit the structure or function of the nucleic acid beyond what is otherwise explicitly indicated.

As used herein, the term "target nucleic acid," is intended as a semantic identifier for the nucleic acid in the context of a method or composition or kit set forth herein and does not necessarily limit the structure or function of the nucleic acid beyond what is otherwise explicitly indicated. Reference to a nucleic acid such as a target nucleic acid includes both single-stranded and double-stranded nucleic acids, and both DNA and RNA, unless indicated otherwise.

The terms "polynucleotide" and "oligonucleotide" are used interchangeably herein to refer to a polymeric form of nucleotides of any length, and may include ribonucleotides, deoxyribonucleotides, analogs thereof, or mixtures thereof. The terms should be understood to include, as equivalents, analogs of either DNA, RNA, cDNA, or antibody-oligo conjugates made from nucleotide analogs and to be applicable to single stranded (such as sense or antisense) and double stranded polynucleotides. The term as used herein also encompasses cDNA that is complementary or copy DNA produced from an RNA template, for example by the action of reverse transcriptase.

As used herein, the term "primer" and its derivatives refer generally to any polynucleotide that can hybridize to a target sequence of interest. Typically, the primer functions as a substrate onto which nucleotides can be polymerized by a polymerase; in some embodiments, however, the primer can become incorporated into the synthesized nucleic acid strand and provide a site to which another primer can hybridize to prime synthesis of a new strand that is complementary to the synthesized nucleic acid molecule. The primer can include any combination of nucleotides or analogs thereof. In some embodiments, the primer is a single-stranded oligonucleotide or polynucleotide. The terms "polynucleotide" and "oligonucleotide" are used interchangeably herein to refer to a polymeric form of nucleotides of any length, and may comprise ribonucleotides, deoxyribonucleotides, analogs thereof, or mixtures thereof. The terms should be understood to include, as equivalents, analogs of either DNA or RNA made from nucleotide analogs and to be applicable to single stranded (such as sense or antisense) and double-stranded polynucleotides. The term as used herein also encompasses cDNA that is complementary or copy DNA produced from an RNA template, for example by the action of reverse transcriptase. This term refers only to the primary structure of the molecule. Thus, the term includes triple-, double- and single-stranded deoxyribonucleic acid ("DNA"), as well as triple-, double- and single-stranded ribonucleic acid ("RNA").

The term "flowcell" as used herein refers to a chamber comprising a solid surface across which one or more fluid reagents can be flowed. Examples of flowcells and related fluidic systems and detection platforms that can be readily used in the methods of the present disclosure are described, for example, in Bentley et al., 2008, Nature 456:53-59, WO 04/018497; US 7,057,026; WO 91/06678; WO 07/123744; US 7,329,492; US 7,211,414; US 7,315,019; US 7,405,281, and US 2008/0108082. Example flow cells and substrates for manufacture of flow cells that may be used in methods and compositions as set forth herein include, but are not limited to, those commercially available from Illumina, Inc. (San Diego, CA).

As used herein, the term "amplicon," when used in reference to a nucleic acid, means the product of copying the nucleic acid, wherein the product has a nucleotide sequence that is the same as or complementary to at least a portion of the nucleotide sequence of the nucleic acid. An amplicon can be produced by any of a variety of amplification methods that use the nucleic acid, or an amplicon thereof, as a template including, for example, PCR, rolling circle amplification (RCA), ligation extension, or ligation chain reaction. An amplicon can be a nucleic acid molecule having a single copy of a particular nucleotide sequence (for example, a PCR product) or multiple copies of the nucleotide sequence (for example, a concatameric product of RCA). A first amplicon of a target nucleic acid is typically a complimentary copy. Subsequent amplicons are copies that are created, after generation of the first amplicon, from the target nucleic acid or from the first amplicon. A subsequent amplicon can have a sequence that is substantially complementary to the target nucleic acid or substantially identical to the target nucleic acid.

As defined herein "multiplex amplification" refers to selective and non-random amplification of two or more target sequences within a sample using at least one target-specific primer. In some embodiments, multiplex amplification is performed such that some or all of the target sequences are amplified within a single reaction vessel. The "plexy" or "plex" of a given multiplex amplification refers generally to the number of different target-specific sequences that are amplified during that single multiplex amplification. In some embodiments, the plexy can be about 12-plex, 24-plex, 48-plex, 96-plex, 192-plex, 384-plex, 768-plex, 1536-plex, 3072-plex, 6144-plex or higher. It is also possible to detect the amplified target sequences by several different methodologies (e.g., gel electrophoresis followed by densitometry, quantitation with a bioanalyzer or quantitative PCR, hybridization with a labeled probe; incorporation of biotinylated primers followed by avidin-enzyme conjugate detection; incorporation of 32P-labeled deoxynucleotide triphosphates into the amplified target sequence).

As used herein, the term "amplification site" refers to a site in or on an array where one or more amplicons can be generated. An amplification site can be further configured to contain, hold, or attach at least one amplicon that is generated at the site.

As used herein, the term "array" refers to a population of sites that can be differentiated from each other according to relative location. Different molecules that are at different sites of an array can be differentiated from each other according to the locations of the sites in the array. An individual site of an array can include one or more molecules of a particular type. For example, a site can include a single target nucleic acid molecule having a particular sequence or a site can include several nucleic acid molecules having the same sequence (and/or complementary sequence, thereof). The sites of an array can be different features located on the same substrate. Exemplary features include without limitation, droplets, wells in a substrate, beads (or other particles) in or on a substrate, projections from a substrate, ridges on a substrate or channels in a substrate. The sites of an array can be separate substrates each bearing a different molecule. Different molecules attached to separate substrates can be identified according to the locations of the substrates on a surface to which the substrates are associated or according to the locations of the substrates in a liquid or gel. Exemplary arrays in which separate substrates are located on a surface include, without limitation, those having beads in wells.

As used herein, the term "clonal population" refers to a population of nucleic acids that is homogeneous with respect to a particular nucleotide sequence. The homogenous sequence is typically at least 10 nucleotides long, but can be even longer including for example, at least 50, 100, 250, 500 or 1000 nucleotides long. A clonal population can be derived from a single target nucleic acid or template nucleic acid. Typically, all of the nucleic acids in a clonal population will have the same nucleotide sequence. It will be understood that a small number of mutations (e.g., due to amplification artifacts) can occur in a clonal population without departing from clonality.

The term "sensitivity" as used herein is equal to the number of true positives divided by the sum of true positives and false negatives.

The term "specificity" as used herein is equal to the number of true negatives divided by the sum of true negatives and false positives.

As used herein, "providing" in the context of a protein, sample of DNA or RNA, or composition means making the protein, sample of DNA or RNA, or composition, purchasing the protein, sample of DNA or RNA, or composition, or otherwise obtaining the protein, sample of DNA or RNA, or composition.

As used herein, "isolated" refers to material removed from its original environment (e.g., the natural environment if it is naturally occurring), and thus is altered "by the hand of man" from its natural state.

As used herein, the term "each," when used in reference to a collection of items, is intended to identify an individual item in the collection but does not necessarily refer to every item in the collection unless the context clearly dictates otherwise.

As used in this specification and the appended claims, the term "or" is generally employed in its sense including "and/or" unless the content clearly dictates otherwise. The term "and/or" means one or all of the listed elements or a combination of any two or more of the listed elements. The use of "and/or" in some instances does not imply that the use of "or" in other instances may not mean "and/or."

Unless otherwise specified, "a," "an," "the," and "at least one" are used interchangeably and mean one or more than one.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure.

As used herein, "have," "has," "having," "include," "includes," "including," "comprise," "comprises," "comprising," or the like are used in their open ended inclusive sense, and generally mean "include, but not limited to, "includes, but not limited to," or "including, but not limited to."

It is understood that wherever embodiments are described herein with the language "have," "has," "having," "include," "includes," "including," "comprise," "comprises," "comprising," and the like, otherwise analogous embodiments described in terms of "consisting of" and/or "consisting essentially of" are also provided. The term "consisting of" means including, and limited to, whatever follows the phrase "consisting of." That is, "consisting of" indicates that the listed elements are required or mandatory, and that no other elements may be present. The term "consisting essentially of" indicates that any elements listed after the phrase are included, and that other elements than those listed may be included provided that those elements do not interfere with or contribute to the activity or action specified in the disclosure for the listed elements.

Conditions that are "suitable" for an event to occur or "suitable" conditions are conditions that do not prevent such events from occurring. Thus, these conditions permit, enhance, facilitate, and/or are conducive to the event.

Reference throughout this specification to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout this specification are not necessarily referring to the same embodiment of the disclosure. Furthermore, the particular features, configurations, compositions, or characteristics may be combined in any suitable manner in one or more embodiments.

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 4.5, 5, 5.3, and 6. This applies regardless of the breadth of the range.

In the description herein particular embodiments may be described in isolation for clarity. Unless otherwise expressly specified that the features of a particular embodiment are incompatible with the features of another embodiment, certain embodiments can include a combination of compatible features described herein in connection with one or more embodiments.

For any method disclosed herein that includes discrete steps, the steps may be conducted in any feasible order. And, as appropriate, any combination of two or more steps may be conducted simultaneously.

Unless otherwise indicated, all numbers expressing quantities of components, molecular weights, and so forth used in the specification and claims are to be understood as being modified in all instances by the term "about." Accordingly, unless otherwise indicated to the contrary, the numerical parameters set forth in the specification and claims are approximations that may vary depending upon the desired properties sought to be obtained by the present invention. At the very least, and not as an attempt to limit the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. All numerical values, however, inherently contain a range necessarily resulting from the standard deviation found in their respective testing measurements.

In several places throughout the application, guidance is provided through lists of examples, which examples can be used in various combinations. In each instance, the recited list serves only as a representative group and should not be interpreted as an exclusive list.

All headings throughout are for the convenience of the reader and should not be used to limit the meaning of the text that follows the heading, unless so specified.

The above summary of the present disclosure provided above is not intended to describe each disclosed embodiment or every implementation of the present disclosure. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### BRIEF DESCRIPTIONS OF THE FIGURES

FIG. 1. Overview of the Chemoenzymatic Uracil Replacement of Nucleobases (ChURN) method.
FIG. 2. Correction of deaminated cytosines.
FIG. 3. Synthetic scheme for hydroxylamine-cytosine.
FIG. 4. Synthetic schemes for reactive cytosines. Synthesis of a hydroxylamine aldehyde reactive cytosine analog is shown as #1. Synthesis of a hydrazide reactive cytosine analog is shown as #2. Synthesis of a hydrazine reactive cytosine analog is shown as cytosine #3.

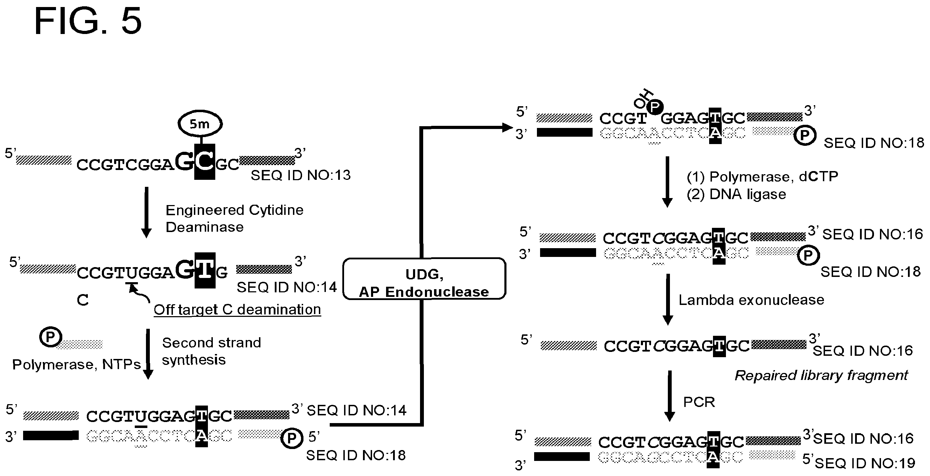

FIG. 5. Overview of the Uracil Enzymatic Removal and Substitution at Errors (U-ERASE) method. First, single stranded DNA libraries are subjected to a cytidine deaminase, resulting in mC>T and some off-target C>U deamination. Then, a second strand of DNA is synthesized. Subsequently, libraries are treated with UDG and an AP endonuclease, resulting in the removal of uracil bases and single nucleotide (nt) gaps at those sites. These gaps are repaired through treatment with a polymerase, dCTP, and a ligase, resulting in a repaired DNA strand. Subsequently, the second strand is selectively degraded, allowing for selective amplification of the original DNA strand via PCR.

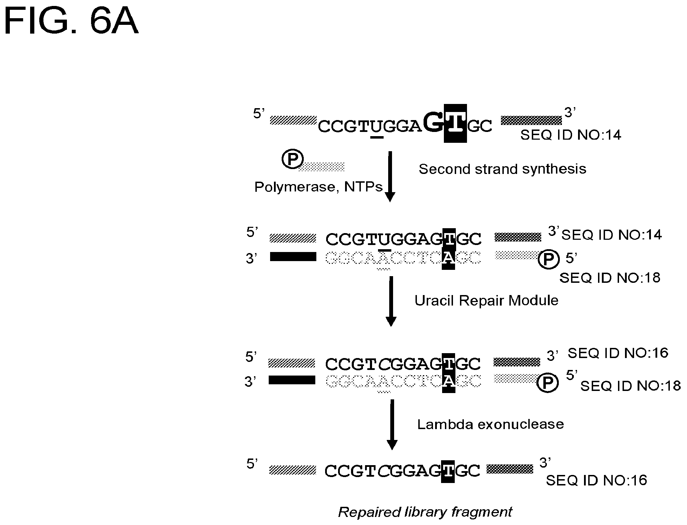

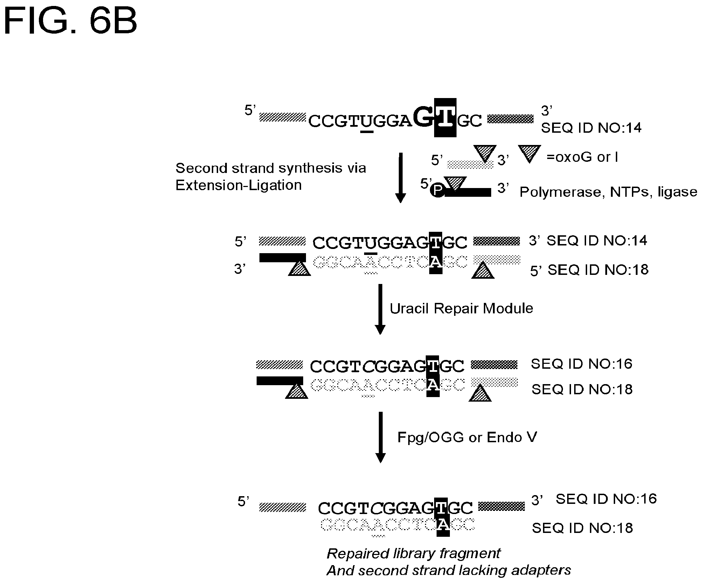

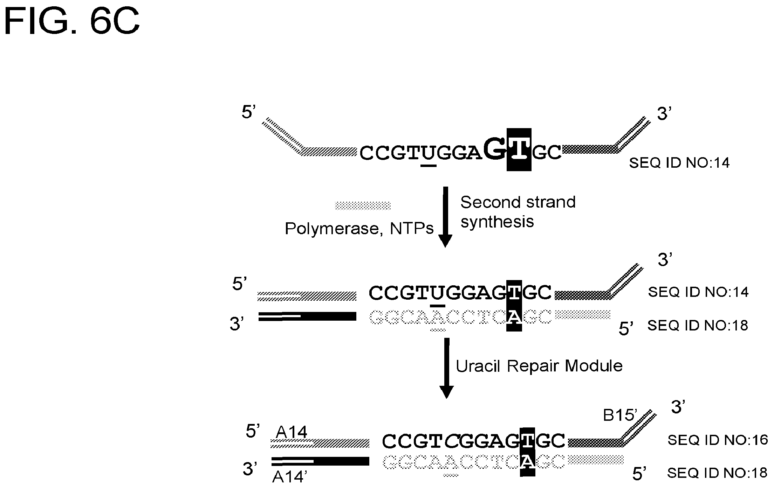

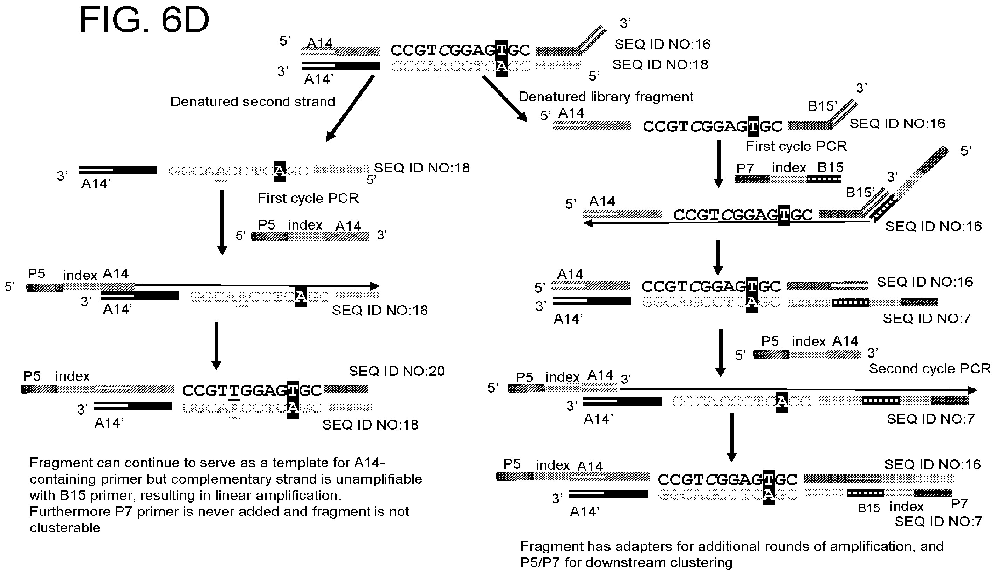

FIGS. 6A-6D. Strategies for second strand synthesis. In Fig. 6A, the second strand is synthesized using a 5'phosphorylated primer. After the uracil repair module, lambda exonuclease, which is specific for DNA strands with a 5' phosphate, is used to selectively degrade the second strand. Fig. 6B shows an alternative strategy for second strand synthesis that leverages extension-ligation and adapter sequences with modified bases (either 8-oxoguanine or inosine). After the uracil repair module, FPG, OGG or Endo V, for 8-oxoG and inosine, respectively, is used to cleave the adapter sequences, rendering the second strand unamplifiable during PCR. Fig. 6C shows an alternative strategy for second strand synthesis in which nested primers are provided for synthesis of the second complementary strand of DNA. Fig. 6D shows linear amplification and generation of non-clusterable product of the second strand compared to exponential amplification and generation of clusterable product from the original library fragment.
FIG. 7. Overview of the uracil repair module. To excise uracil residues from library fragments, Uracil DNA glycosylase (UDG) first removes the uracil base from the DNA polynucleotide. Then, an endonuclease cleaves the phosphodiester backbone, resulting in a 3'hydroxyl group, 1 nt gap, and 5' phosphate. Endonucleases that may be used include AP Endonuclease 1 or Endonuclease IV.
FIG. 8. Representative workflow showing nucleobase replacement at the site of uracil excision. First, Klenow exo- incorporates dCTP into the 1 nucleotide gap. The Klenow-treated product includes a top strand with a discontinuous backbone. Then, T4 DNA ligase ligates across the mismatch to complete repair of the library fragment.

### DETAILED DESCRIPTION OF ILLUSTRATIVE EMBODIMENTS

Described herein are various methods of removing uracils due to the deamination of unmethylated cytosines in an assay using engineered cytosine deaminases to deaminate methylated cytosines. The Chemoenzymatic Uracil Replacement of Nucleobases (ChURN) method includes providing a sample comprising single stranded DNA library fragments in which a cytosine deaminase has deaminated methylated cytosines; contacting the sample with an uracil DNA glycosylase (UDG) to deglycosylate uracil residues to form abasic sites having a hemiacetal formation within the single stranded DNA library fragments; contacting the sample with a reactive cytosine nucleobase analog to install a cytosine at abasic sites thru a noncanonical linkage; and subjecting the sample to polymerase chain reaction (PCR) amplification resulting in double stranded DNA corrected library fragments. The Uracil Enzymatic Removal and Substitution at Errors (U-ERASE) method not encompassed by the wording of the claims includes providing a sample comprising single stranded DNA library fragments in which a cytosine deaminase has deaminated methylated cytosines, synthesizing double stranded DNA library fragments in which the second strand is tagged to facilitate its downstream degradation, treating the double stranded DNA library fragments with an uracil DNA glycosylase and an endonuclease resulting in the removal of uracil bases and single nucleotide gaps at those sites, and repairing the single nucleotide gaps through treatment with a polymerase, dCTP, and a ligase, resulting in replacement of false positive uracil bases with a mismatched base, such as cytosine. Subsequently, the second strand is selectively degraded, allowing for selective amplification of the original DNA strand via PCR.

### Chemoenzymatic Uracil Replacement of Nucleobases (ChURN)

With the Chemoenzymatic Uracil Replacement of Nucleobases (ChURN) method described herein the problem of false positive conversions of cytosines to uracils in cytosine deaminase based methylation detection assays is solved by the selective chemoenzymatic correction of false positive uracils to cytosines. The enzymatic de-glycosylation of uracil residues is followed by chemical treatment to install cytosine nucleobases.

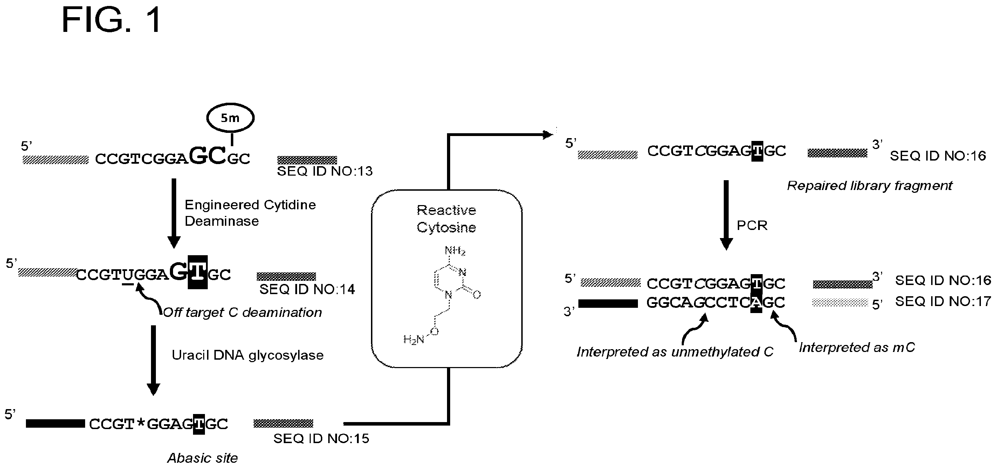

A schematic illustrating this Chemoenzymatic Uracil Replacement of Nucleobases (ChURN) method is shown in FIG. 1. Briefly, a preparation of DNA fragments from an input sample that has been treated with a cytidine deaminase to deaminate 5-methylcytosine (5mC) and/or 5-hydroxymethylcytosine (5hmC) residues and possibly including one or more off-target conversions of a cytosine to an uracil is first treated with an uracil DNA glycosylase (UDG). UDG enzymatically catalyzes the hydrolysis of the N-glycosidic bond from deoxyuridine to release uracil, resulting in an abasic site. The preparation of DNA fragments is then treated with one or more reactive cytosine analogs that react with the abasic site, to repair the abasic site by the insertion of a cytosine, resulting in the repair or correction of the off-target conversions of a cytosine to an uracil. The preparation of DNA fragments from the input sample may then be amplified, propagating the repaired/corrected DNA fragments, and sequenced.

With the methods described herein, the target nucleic acids (also referred to herein as "DNA fragments" or "a preparation of DNA fragments from an input sample") may be essentially any nucleic acid of known or unknown sequence.

Such target nucleic acids are typically derived from primary nucleic acids present in a sample, such as a biological sample. The primary nucleic acids may originate as DNA or RNA. DNA primary nucleic acids may originate in double-stranded DNA (dsDNA) form (e.g., genomic DNA, genomic DNA fragments, cell-free DNA, and the like) from a sample or may originate in single-stranded form from a sample. RNA primary nucleic acids may be mRNA or non-coding RNA, e.g., microRNA or small interfering RNA. A preparation of DNA fragments from an input sample may be single or double stranded DNA. In some preferred embodiments, DNA fragments are single stranded.

The primary nucleic acid molecules may represent the entire genetic complement of an organism, e.g., genomic DNA molecules which include both intron and exon sequences, as well as non-coding regulatory sequences such as promoter and enhancer sequences. The primary nucleic acid molecules may represent the entire genetic complement of specific cells of an organism, e.g., from tumor cells, where the genomic DNA molecules which include both intron and exon sequences, as well as non-coding regulatory sequences such as promoter and enhancer sequences. In one embodiment, particular subsets of genomic DNA can be used, such as, for example, particular chromosomes, DNA associated with open chromatin, DNA associated with closed chromatin, or one or more specific sequences such as a region of a specific gene (e.g., targeted sequencing). In one or more embodiments, the primary nucleic acid molecules may represent a particular subset of DNA, e.g., DNA having a specific sequence that anneals with a primer such as one used for targeted sequencing or target enrichment. In one embodiment, a particular subset of DNA can be used, such as cell-free DNA, which can include DNA of the subject including DNA from normal cells, DNA from diseased cells such as tumor cells, and/or DNA from fetal cells.

The primary nucleic acid molecules may represent the entire transcriptome of cells of an organism, e.g., mRNA molecules. The primary nucleic acid molecules may represent the entire transcriptome of specific cells of an organism, e.g., from tumor cells or for instance the cells of a tissue. In one embodiment, the primary nucleic acid molecules may represent a particular subset of mRNA, e.g., mRNA having a specific sequence that anneals with a primer such as one used for targeted sequencing or target enrichment.

A sample, such as a biological sample, can include nucleic acid molecules obtained from biopsies, tumors, scrapings, swabs, blood, mucus, urine, plasma, semen, hair, laser capture micro-dissections, surgical resections, and other clinical or laboratory obtained samples. **In** some embodiments, the sample can be an epidemiological, agricultural, forensic, or pathogenic sample. **In** some embodiments, the sample can include cultured cells. **In** some embodiments, the sample can include nucleic acid molecules obtained from an animal such as a human or mammalian source. **In** another embodiment, the sample can include nucleic acid molecules obtained from a non-mammalian source such as a plant, bacteria, virus, or fungus. **In** some embodiments, the source of the nucleic acid molecules may be an archived or extinct sample or species.

Additional non-limiting examples of sources of biological samples can include whole organisms as well as a sample obtained from a subject or a patient. The biological sample can be obtained from any biological fluid or tissue and can be in a variety of forms, including liquid fluid and tissue, solid tissue, and preserved forms such as dried, frozen, and fixed forms. The sample may be of any biological tissue, cells, or fluid. Such samples include, but are not limited to, sputum, blood, serum, plasma, blood cells (e.g., white cells), ascitic fluid, urine, saliva, tears, sputum, vaginal fluid (discharge), washings obtained during a medical procedure (e.g., pelvic or other washings obtained during biopsy, endoscopy or surgery), tissue, nipple aspirate, core or fine needle biopsy samples, cell-containing body fluids, peritoneal fluid, and pleural fluid, or cells therefrom, and free floating nucleic acids such as cell-free circulating DNA. Biological samples may also include sections of tissues such as frozen or fixed sections taken for histological purposes or micro-dissected cells or extracellular parts thereof. **In** some embodiments, the sample can be a blood sample, such as, for example, a whole blood sample. **In** another example, the sample is an unprocessed dried blood spot (DBS) sample. **In** yet another example, the sample is a formalin-fixed paraffin-embedded (FFPE) sample. **In** yet another example, the sample is a saliva sample. **In** yet another example, the sample is a dried saliva spot (DSS) sample.

Exemplary biological samples from which target nucleic acids can be derived include, for example, those from a eukaryote, for instance a mammal, such as a rodent, mouse, rat, rabbit, guinea pig, ungulate, horse, sheep, pig, goat, cow, cat, dog, primate, human or non-human primate; a plant, such as *Arabidopsis thaliana,* corn, sorghum, oat, wheat, rice, canola, or soybean; an algae, such as *Chlamydomonas reinhardtii;* a nematode such as *Caenorhabditis elegans;* an insect, such as *Drosophila melanogaster,* mosquito, fruit fly, honey bee or spider; a fish, such as zebrafish; a reptile; an amphibian, such as a frog or *Xenopus laevis; a Dictyostelium discoideum;* a fungi, such as *Pneumocystis carinii, Takifugu rubripes,* yeast, *Saccharamoyces cerevisiae,* or *Schizosaccharomyces pombe;* or a protozoan such as *Plasmodium falciparum.* Target nucleic acids can also be derived from a prokaryote such as a bacterium, *Escherichia coli, Staphylococcus* or *Mycoplasma pneumoniae;* an archaeon; a virus such as Hepatitis C virus or human immunodeficiency virus; or a viroid. Target nucleic acids can be derived from a homogeneous culture or population of organisms described herein or alternatively from a collection of several different organisms, for example, in a community or ecosystem.

In some embodiments, a biological sample includes tissue that is processed to obtain the desired primary nucleic acids. In some embodiments, cells are used obtain the desired primary nucleic acids. In some embodiments, nuclei are used to obtain the desired primary nucleic acids. The method can further include dissociating cells, and/or isolating nuclei from cells. Methods for isolating cells and nuclei from tissue are available (WO 2019/236599).

In some embodiments, nucleic acids present in tissue, in cells, or in isolated nuclei can be processed depending on the desired read-out. For instance, nucleic acids can be fixed during processing, and useful fixation methods are available (WO 2019/236599). Fixation can be useful to preserve a sample or maintain contiguity of analytes from a sample, a cell, or a nucleus. Fixation methods preserve and stabilize tissue, cell, and nucleus morphology and architecture, inactivates proteolytic enzymes, strengthens samples, cells, and nuclei so they can withstand further processing and staining, and protects against contamination. Examples of methods where fixation can be useful include, but are not limited to, whole genome sequencing of isolated nuclei and chromosome conformation capture methods such as Hi-C. Common methods of fixation include perfusion, immersion, freezing, and drying (Srinivasan et al., Am J Pathol. 2002 Dec; 161(6): 1961-1971.doi: 10.1016/S0002-9440(10)64472-0). In some embodiments such as whole genome sequencing, isolated nuclei can be processed to dissociate nucleosomes from DNA while leaving the nuclei intact, and methods for generating nucleosome-free nuclei are available (WO 2018/018008).

In some embodiments, primary nucleic acids in bulk, e.g., from a plurality of cells, can be used to produce a sequencing library as described herein. In other embodiments, individual cells or nuclei can be used as sources of primary nucleic acids to obtain sequence information from single cells and nuclei. Many different single cell library preparation methods are known in the art, including, but not limited to, Drop-seq, Seq-well, and single cell combinatorial indexing ("sci-") methods. Companies providing single cell products and related technologies include, but are not limited to, Illumina, 10X genomics, Takara Biosciences, BD biosciences, Biorad, 1cellbio, isoplexis, CellSee, nanoselect, and Dolomite bio. Sci-seq is a methodological framework that employs split-pool barcoding to uniquely label the nucleic acid contents of large numbers of single cells or nuclei. Typically, the number of nuclei or cells can be at least two. The upper limit is dependent on the practical limitations of equipment (e.g., multi-well plates, number of indexes) used in other steps of the methods as described herein. The number of nuclei or cells that can be used is not intended to be limiting and can number in the billions.

The target nucleic acids used in the methods and compositions of the present disclosure can be derived by fragmentation. Random fragmentation refers to the fragmentation of a polynucleotide molecule from a primary nucleic acid sample in a non-ordered fashion by enzymatic, chemical, or mechanical methods. Such fragmentation methods are known in the art and use standard methods (Sambrook and Russell, Molecular Cloning, A Laboratory Manual, third edition). Moreover, random fragmentation is designed to produce fragments irrespective of the sequence identity or position of nucleotides comprising and/or surrounding the break. In one or more embodiments, the random fragmentation is by mechanical means such as nebulization or sonication to produce fragments of about 50 base pairs in length to about 1500 base pairs in length, for example, about 50-700 base pairs in length, about 50-400 base pairs in length. In some preferred embodiments, fragments are about 100 to 200 base pairs in length.

In some embodiments, the DNA fragments are DNA library fragments. Any of the many library preparation protocols available are compatible with the methods described herein. A library may be a whole-genome library or a targeted library. A library includes, but is not limited to, a sequencing library. A multitude of sequencing library methods are known to a skilled person (see, for example, Sequencing Methods Review, available on the world wide web at illumina.com/content/dam/illumina-marketing/documents/products/research_ reviews/sequencing-methods-review.pdf). For example, library preparation may be for use with any of a variety of next generation sequencing platforms, such as for example, the sequencing by synthesis platform of ILLUMINA^{®} or the ion semiconductor sequencing platform of ION TORRENT^{™}. For example, established ligase-dependent methods or transposon-based methods may be used (See, for example, Head et al, 2014, Biotechniques; 56(2):61 and Bruinsma et al., 2019, BMC Genomics; 19:722) and numerous kits for making sequencing libraries by these methods are available commercially from a variety of vendors.

DNA fragments, including DNA library fragments, may be prepared from input sample material such that adapter sequences are ligated to fragments to facilitate downstream workflow steps, such as for example, amplification and sequencing. For example, universal amplification sequences, e.g., sequences present in a universal adaptor, may be placed at the ends of each nucleotide fragment to facilitate amplification. Methods for attaching adapters to a nucleic acid are known to the person skilled in the art. For example, the attachment can be through tagmentation using transposase complexes (WO 2016/130704), or through standard library preparation techniques using ligation (U.S. Pat. Pub. No. 2018/0305753). Addition of an adapter can occur before or after treatment of the target nucleic acid with a cytidine deaminase and/or an uracil de-glycosylase.

Adapter sequences may include 5' and/or 3' adapter sequences. An adapter may be attached to just one end of the DNA fragment, for example, 5' and/or 3' ends, or to both ends. As used herein, the term "adapter" and its derivatives, e.g., universal adapter, refers generally to any linear oligonucleotide which can be attached to a target nucleic acid. An adapter can be single-stranded or double-stranded DNA or can include both double-stranded and single-stranded regions. An adapter can include a universal sequence that is substantially identical, or substantially complementary, to at least a portion of a primer, for example a universal primer; an index (also referred to herein as a barcode or tag) to assist with downstream error correction, identification, or sequencing; and/or a unique molecular identifier. In some embodiments, the adapter is substantially non-complementary to the 3' end or the 5' end of any target sequence present in the sample. In some embodiments, suitable adapter lengths are in the range of about 6-100 nucleotides, about 12-60 nucleotides, or about 15-50 nucleotides in length. For instance, The terms "adaptor" and "adapter" are used interchangeably. As used herein, the term "universal," when used to describe a nucleotide sequence, refers to a region of sequence that is common to two or more nucleic acid molecules where the molecules also have regions of sequence that differ from each other. Non-limiting examples of universal capture sequences include sequences that are identical to or complementary to P5 and P7 primers. The terms "P5" and "P7" may be used when referring to a universal capture sequence or a capture oligonucleotide. The terms "P5'" (P5 prime) and "P7'" (P7 prime) refer to the reverse complement of P5 and P7, respectively. It will be understood that any suitable universal capture sequence or a capture oligonucleotide can be used in the methods presented herein, and that the use of P5 and P7 are exemplary embodiments only. Uses of capture oligonucleotides such as P5 and P7 or their complements on flowcells are known in the art, as exemplified by the disclosures of WO 2007/010251, WO 2006/064199, WO 2005/065814, WO 2015/106941, WO 1998/044151, and WO 2000/018957. For example, any suitable forward amplification primer, whether immobilized or in solution, can be useful in the methods presented herein for hybridization to a complementary sequence and amplification of a sequence. Similarly, any suitable reverse amplification primer, whether immobilized or in solution, can be useful in the methods presented herein for hybridization to a complementary sequence and amplification of a sequence. One of skill in the art will understand how to design and use primer sequences that are suitable for capture and/or amplification of nucleic acids as presented herein.

DNA fragments, including DNA library fragments, can have an average strand length that is desired or appropriate for a particular application of the methods, compositions, or kits set forth herein. For example, the average strand length can be less than about 100,000 nucleotides, 50,000 nucleotides, 10,000 nucleotides, 5,000 nucleotides, 1,000 nucleotides, 500 nucleotides, 200 nucleotides, 100 nucleotides, or 50 nucleotides. Alternatively, or additionally, the average strand length can be greater than about 10 nucleotides, 50 nucleotides, 100 nucleotides, 200 nucleotides, 500 nucleotides, 1,000 nucleotides, 5,000 nucleotides, 10,000 nucleotides, 50,000 nucleotides, or 100,000 nucleotides. The average strand length for a population of DNA fragments can be in a range between a maximum and minimum value set forth above.

In some embodiments, DNA fragments, including DNA library fragments, may be of a shorter length, for example, about 50 nucleotides to about 500 nucleotides in length, about 50 nucleotides to about 300 nucleotides in length, about 50 nucleotides to about 250 nucleotides in length, about 100 nucleotides to about 200 nucleotides in length, or about 100 nucleotides to about 250 nucleotides in length. In some embodiments, DNA fragments, including DNA library fragments, may be about 100 nucleotides to about 200 nucleotides in length. Shorter fragment length can be employed to maximize the overall performance of the enzymatic error-correction, by minimizing the number of potential false-positive uracils that may be present in any one individual DAN fragment. By minimizing the number of false positives in each fragment, the probability of successfully repairing library fragments and mediating their downstream amplification may be increased, thus preserving library quality and diversity.

With the methods described herein, a sample including single-stranded DNA (ssDNA) fragments is contacted with a cytosine deaminase to deaminated methylated cytosines. In some embodiments, a sample including single-stranded DNA (ssDNA) fragments is a preparation of denatures library fragments. In some embodiments, the library fragments may include 5' and/or 3' adapter sequences.

As used herein, a "cytidine deaminase enzyme" refers to an enzyme that deaminates cytosine and/or one or more cytosine derivatives. The deamination occurs at the amino group at the C4 position of the cytosine or cytosine derivative. For example, a cytidine deaminase enzyme may deaminate cytosine to form U, may deaminate mC to form T, and/or may deaminate hydroxymethylcytosine (hmC) to form hmU. A nonlimiting example of a cytidine deaminase enzyme that may deaminate cytosine to form U, may deaminate mC to form T, and/or may deaminate hmC to form hmU is apolipoprotein B mRNA editing enzyme, catalytic polypeptide-like (APOBEC). Nonlimiting examples of such APOBECs include APOBEC1, APOBEC2, APOBEC3A, APOBEC3B, APOBEC3C, APOBEC3E, APOBEC3F, APOBEC3G, APOBEC3H, and APOBEC4. As used herein, the term "methylcytosine" or "mC" refers to cytosine that includes a methyl group (-CH3 or -Me). The methyl group may be located at the 5 position of the cytosine, in which case the mC may be referred to as 5mC.

In some embodiments, a cytidine deaminase is an altered cytidine deaminase, recombinantly engineered to include a substitution mutation at one or more residues when compared to a reference cytidine deaminase. An altered cytidine deaminase can be based on a member of the AID subfamily, the APOBEC1 subfamily, the APOBEC2 subfamily, the APOBEC3 subfamily (e.g., the 3A subfamily, the 3B subfamily, the 3C subfamily, the 3D subfamily, the 3F subfamily, the 3G subfamily, or the 3H subfamily), or the APOBEC4 subfamily. The skilled person will readily appreciate that such an altered or engineered cytidine deaminase described herein is not naturally occurring. In some embodiments, such an altered or engineered cytidine deaminase demonstrates selective deamination for mC.

An altered cytidine deaminase may be one of three types of altered cytidine deaminases. One type of altered cytidine deaminase preferentially deaminates 5mC instead of C (i.e., converts 5mC to T at a greater rate than converting C to U) and is referred to herein as having "cytosine-defective deaminase activity." A second type of altered cytidine deaminase preferentially deaminates C instead of 5mC (i.e., converts C to U at a greater rate than converting 5mC to T) and is referred to herein as having "5mC-defective deaminase activity." A third type of altered cytidine deaminase preferentially deaminates C and 5mC to U and T, respectively, and has significantly reduced deamination of 5hmC, 5fC, and 5caC. The third type is referred to herein as having "5hmC-defective deaminase activity." Unless the context indicates otherwise, reference to an altered cytidine deaminase includes altered cytidine deaminases having cytosine-defective deaminase activity, altered cytidine deaminases having 5mC-defective deaminase activity, and altered cytidine deaminases having 5mC-defective deaminase activity.

Altered cytidine deaminases include apolipoprotein B mRNA editing enzymes, catalytic polypeptide-like (APOBEC) and activation induced cytidine deaminase (AID). Wild-type APOBEC and AID cytidine deaminases have the activity of deaminating cytidine (C) of DNA and/or RNA to form uridine (U). An altered cytidine deaminase of the present disclosure has an altered rate of deamination of C, 5mC, and/or 5hmC when compared to the wild-type enzyme. A cytidine deaminase of the present disclosure can be referred to herein as an "altered cytidine deaminase," "recombinant cytidine deaminase," "mutant cytosine deaminase," or "modified cytidine deaminases" and refers to any of the altered cytosine deaminases described herein that comprise one or more changes from the reference (i.e., wildtype) amino acid sequence that provide the unexpected property of an altered deamination profile, e.g., alters its ability to preferentially deaminate one form of cytosine over another.

Whether a protein has cytidine deaminase activity may be determined by *in vitro* assays. On example of an *in vitro* assay is based on digestion with the restriction enzyme *Swa*I*. A* protein that can deaminate 5mC to thymidine has cytidine deaminase activity.

An altered cytidine deaminase that preferentially deaminates 5mC instead of C (i.e., has cytosine-defective deaminase activity) can have a catalytic efficiency that is at least 10-fold, at least 50-fold, or at least 100-fold higher on 5mC than C substrates. In one embodiment, an altered cytidine deaminase that preferentially deaminates 5mC instead of C can have a catalytic efficiency that is no greater than 1500-fold higher on 5mC than C substrates.

An altered cytidine deaminase that preferentially deaminates C instead of 5mC (i.e., has 5mC-defective deaminase activity) can have a catalytic efficiency that is at least 10-fold, at least 50-fold, or at least 100-fold higher on C than 5mC substrates. In one embodiment, an altered cytidine deaminase that preferentially deaminates C instead of 5mC can have a catalytic efficiency that is no greater than 1500-fold higher on C than 5mC substrates.

When compared to a wild type cytidine deaminase, an altered cytidine deaminase that deaminates C and 5mC to U and T, respectively, and has significantly reduced deamination of 5hmC (i.e., has 5hmC-defective deaminase activity), the deamination of 5hmC by an altered cytidine deaminase disclosed herein is reduced by at least 80%, at least 90%, or at least 99% compared to the wild type cytidine deaminase. In one embodiment, the deamination of 5hmC by an altered cytidine deaminase disclosed herein is undetectable using an assay such as the *Swa*I-based assay.

In certain embodiments, an altered cytidine deaminase of the present disclosure is based on a member of the APOBEC protein family. An altered cytidine deaminase of the present disclosure that is "based on" a member of the APOBEC protein family means the altered cytidine deaminase is an APOBEC protein that includes one or more of the substitution mutations described herein as compared to a reference APOBEC sequence. An altered cytidine deaminase of the present disclosure that is "based on" a member of the APOBEC protein family can also include conservative and/or nonconservative mutations as described herein.

The APOBEC protein family includes subfamilies AID, APOBEC1, APOBEC2, APOBEC3 (including 3A, 3B, 3C, 3D, 3F, 3G, 3H), and APOBEC4. An altered cytidine deaminase of the present disclosure can be based on a member of the AID subfamily, the APOBEC1 subfamily, the APOBEC2 subfamily, the APOBEC3 subfamily (e.g., the 3A subfamily, the 3B subfamily, the 3C subfamily, the 3D subfamily, the 3F subfamily, the 3G subfamily, or the 3H subfamily), or the APOBEC4 subfamily. An altered cytidine deaminase of the present disclosure can be based on a member of the APOBEC protein family from a vertebrate, such as a mammal. Examples of mammals include, but are not limited to, rodents, primates, rabbit, bovine (e.g., cow), porcine (e.g., pig), and equine (e.g., horse). An example of a primate is a human and a chimpanzee.

The APOBEC protein family is a member of the large cytidine deaminase superfamily that contains a canonical zinc-dependent deaminase (ZDD) signature motif embedded within a core cytidine deaminase fold. This fold includes a five-stranded mixed beta (b)-sheet surrounded by six alpha (a)-helices with the order a1-b1-b2-a2-b3-a3-b4-a4-b5-a5-a6 (Salter et al., 2016, Trends Biochem Sci; 41(7):578-594. doi:10.1016/j.tibs.2016.05.001; Salter et al., 2018, Trends Biochem Sci; 43(8):606-622 doi.org/10.1016/j.tibs.2018.04.013). Each cytidine deaminase domain core structure of APOBEC proteins contains a highly conserved spatial arrangement of the catalytic center residues of a zinc-binding motif H-[P/A/V]-E-X_{[23-28]}-P-C-X_{[2-4]}-C (SEQ ID NO: 1) (referred to herein as the ZDD motif, where X is any amino acid, and the subscript range of numbers after X refers to the number of amino acids) (Salter et al., 2016, Trends Biochem Sci; 41(7):578-594. doi:10.1016/j.tibs.2016.05.001). Without intending to be limited by theory, the H and two C residues coordinate a Zn atom, and the E residue polarizes a water molecule near the Zn-atom for catalysis (Chen et al., 2021, Viruses; 13:497*).*

Some members of the APOBEC protein family, e.g., the AID subfamily, the APOBEC1 subfamily, the APOBEC2 subfamily, the APOBEC3A subfamily, the APOBEC3C subfamily, the APOBEC3H subfamily, and the APOBEC4 subfamily, include one copy of the ZDD motif. Other members of the APOBEC protein family, e.g., the APOBEC3B subfamily, the APOBEC3D subfamily, the APOBEC3F subfamily, and the APOBEC3G subfamily, include two copies of the ZDD motif, but often only the C-terminal copy is active (Salter et al., 2016, Trends Biochem Sci; 41(7):578-594. doi:10.1016/j.tibs.2016.05.001). Thus, an altered cytidine deaminase disclosed herein includes one or two ZDD motifs. In one embodiment, an altered cytidine deaminase based on a member of the APOBEC3A subfamily includes the following ZDD motif: HXEX₂₄SW(S/T)PCX_{[2-4]}CX₆FX₈LX₅R(L/I)YX_{[8-11]}LX₂LX_{[10]}M (SEQ ID NO: 2) (where X is any amino acid, and the subscript number or range of numbers after X refers to the number of amino acids) (Salter et al., 2016, Trends Biochem Sci; 41(7):578-594).

In one embodiment, an altered cytidine deaminase disclosed herein is a member of the following subfamilies, APOBEC3A, APOBEC3B, APOBEC3C, APOBEC3D, APOBEC3F, and APOBEC3G, and can include one or more highly conserved sites that are part of the active site and within the ZDD motif SEQ ID NO: 1. The sites include tryptophan at position 98 and serine or threonine at position 99 (Kouno et al., 2017, Nat. Comm; 8:15024).

In addition to the ZDD motif, a member of the APOBEC protein family also includes other highly conserved residues that are part of the active site but not present as part of the ZDD motif SEQ ID NO: 1. A member the APOBEC3A subfamily, APOBEC3B subfamily, APOBEC3C subfamily, APOBEC3D subfamily, APOBEC3F subfamily, and APOBEC3G subfamily typically includes one or more of the following highly conserved sites that are part of the active site: arginine at position 28; histidine, asparagine, or arginine at position 29; serine or threonine, preferably threonine, at position 31; asparagine or aspartic acid at position 57; tyrosine or phenylalanine at position 130; asparagine or tyrosine at position 131; asparagine, tyrosine, or phenylalanine, preferably tyrosine, at position 132; and arginine or lysine at position 189 (Kouno et al., 2017, Nat. Comm; 8:15024, DOI: 10.1038/ncomms15024).

An altered cytidine deaminase of the present disclosure includes a substitution mutation at one or more residues when compared to a reference cytidine deaminase. A substitution mutation can be at the same position or a functionally equivalent position compared to the reference cytidine deaminase. Reference cytidine deaminases and functionally equivalent positions are described in detail herein. The skilled person will readily appreciate that an altered cytidine deaminase described herein is not naturally occurring.

A reference cytidine deaminase can be a member of the APOBEC protein family. Essentially any known member of the APOBEC protein family can be a reference cytidine deaminase. The skilled person can easily identify members of each of the subfamilies by using a publicly available database such as the Protein database available at the National Center for Biotechnology Information (ncbi.nlm.nih.gov/protein) and searching for APOBEC1, APOBEC2, APOBEC3A, APOBEC3B, APOBEC3C, APOBEC3D, APOBEC3F, APOBEC3G, APOBEC3H, APOBEC4, or, when identifying members of the AID family, Activation-induced cytidine deaminase. A wild type reference cytidine deaminase has the activity of binding single-stranded DNA (ssDNA) and deaminating a cytosine present on the ssDNA to convert it to uracil. In one embodiment, a wild type reference cytidine deaminase has the activity of binding single-stranded RNA (ssRNA) and deaminating a cytosine present on the ssRNA to convert it to uracil. Methods for determining whether a protein binds ssDNA or ssRNA and deaminates a cytosine present are known to the skilled person.

In one embodiment, an altered cytidine deaminase has an amino acid sequence that is based on a reference sequence which is a member of the APOBEC protein family includes a ZDD motif H-[P/A/V]-E-X_{[23-28]}-P-C-X_{[2-4]}-C (SEQ ID NO: 1) and at least one substitution mutation disclosed herein. Optionally, an altered cytidine deaminase includes other active site residues disclosed herein. Non-limiting examples of reference cytidine deaminase proteins are shown in the following table.

**Table 1. Examples of members of the APOBEC protein subfamilies.**

| APOBEC protein family | Non-limiting examples |
|---|---|
| AID | UniProt: Q9GZX7; UniProt: G3QLD2; Uniprot Q9WVE0 |
| APOBEC1 | UniProt: P41238; NCBI XP_030856728.1; Uniprot P51908 |
| APOBEC2 | UniProt: Q9Y235; Uniprot G3SGN8; Uniprot Q9WV35 |
| APOBEC3A | UniProt: P31941 (SEQ ID NO: 12); GenBank: XP_045219544.1 |
| | GenBank: AER45717.1; GenBank: XP_003264816.1; GenBank: PNI48846.1; GenBank: ADO85886.1 |
| APOBEC3B | UniProt: Q9UH17; Uniprot G3QV16; Uniprot F6M3K5 |
| APOBEC3C | UniProt: Q9NRW3; Uniprot Q694B5; Uniprot B0LW74 |
| APOBEC3D | UniProt: Q96AK3; NCBI NP_001332895.1; NCBI NP_001332931.1 |
| APOBEC3F | UniProt: Q8IUX4; Uniprot G3RD21; Uniprot Q1G0Z6 |
| APOBEC3G | UniProt: Q9HC16; Uniprot Q694C1; Uniprot U5NDB3 |
| APOBEC3H | UniProt: Q6NTF7; Uniprot B7T0U7; Uniprot Q19Q52 |
| APOBEC4 | UniProt: Q8WW27; NCBI XP_004028087.1; Uniprot Q497M3 |

| | |
|---|---|
| UniProt, database of protein sequence and functional information, available at uniprot.org; GenBank, collection of nucleotide sequences and their protein translations, available at ncbi.nlm.nih.gov/protein/. | |

In one embodiment, an altered cytidine deaminase has an amino acid sequence that is based on a reference sequence that is a member of the APOBEC3A subfamily, and includes a ZDD motif HXEX₂₄SW(S/T)PCX_{[2-4]}CX₆FX₈LX₅R(L/I)YX_{[8-11]}LX₂LX_{[10]}M (SEQ ID NO: 2) (where X is any amino acid, and the subscript number or range of numbers after X refers to the number of amino acids) and at least one substitution mutation disclosed herein. In one embodiment, the substitution mutation is a substitution mutation at the underlined tyrosine, such as a substitution mutation to alanine (A). Optionally, the altered cytidine deaminase includes other active site residues disclosed herein.

In one embodiment, the amino acid sequence of an altered cytidine deaminase includes the amino acids of a member of the APOBEC3A subfamily: X_{[16-26]}-GRXXTXLCYXV-X₁₅-GXXXN-X₁₂-HAEXXF-X₁₄-YXXTWXXSWSPC- X_{[2-4]}-CA-X₅-FL-X₇-LXIXXXR(L/I)Y-X₈-GLXXLXXXG-X₅-M-X₄-FXXCWXXFV-X₆-FXPW-X₁₃-LXXI- X_{[2-6]} (SEQ ID NO: 3) (where X is any amino acid, and the subscript number or range of numbers after X refers to the number of amino acids), or a subset thereof, and at least one substitution mutation disclosed herein. In one embodiment, the substitution mutation is a substitution mutation at the underlined tyrosine, such as a substitution mutation to alanine (A) or to tryptophan (W).

In one embodiment, the amino acid sequence of an altered cytidine deaminase includes the amino acids of a member of the APOBEC3A subfamily: X₂₆-GRXXTXLCYXV-X₁₅-G-X₁₆-HAEXXF-X₁₄-YXXTWXXSWSPC-X₄-CA-X₅-FL-X₇-LXIFXXR(L/I)Y-X₈-GLXXLXXXG-X₅-M-X₄-FXXCWXXFV-X₆-FXPW-X₁₃-LXXI-X₆ (SEQ ID NO: 4) (where X is any amino acid, and the subscript number after X refers to the number of amino acids present), or a subset thereof, and at least one substitution mutation disclosed herein. In one embodiment, the substitution mutation is a substitution mutation at the underlined tyrosine (Y), such as a substitution mutation to alanine (A) or to tryptophan (W).

A substitution mutation can be at the same position or a functionally equivalent position compared to a reference cytidine deaminase. By "functionally equivalent" it is meant that the altered cytidine deaminase has the amino acid substitution at the amino acid position in a reference cytidine deaminase that has the same functional role in both the reference cytidine deaminase and the altered cytidine deaminase.

In general, functionally equivalent substitution mutations in two or more different cytidine deaminases occur at homologous amino acid positions in the amino acid sequences of the cytidine deaminases. Hence, use herein of the term "functionally equivalent" also encompasses mutations that are "positionally equivalent" or "homologous" to a given mutation, regardless of whether or not the particular function of the mutated amino acid is known. It is possible to identify the locations of functionally equivalent and positionally equivalent amino acid residues in the amino acid sequences of two or more different cytidine deaminases on the basis of sequence alignment and/or molecular modelling. For example, the tyrosine at residue 130 of the APOBEC3A proteins of *Homo sapiens, Pongo pygmaeus, Nomascus leucogenys, Pan troglodytes,* and *Gorilla* and the tyrosine at residue 133 of the APOBEC3A protein from *Macaca fascicularis* are functionally equivalent and positionally equivalent. The skilled person can easily identify functionally equivalent residues in cytidine deaminases.

In one embodiment, an altered cytidine deaminase has an amino acid sequence that is structurally similar to a reference cytidine deaminase disclosed herein. In one embodiment, a reference cytidine deaminase is one that includes the amino acid sequence of a sequence listed in Table 1.

As used herein, an altered cytidine deaminase may be "structurally similar" to a reference cytidine deaminase if the amino acid sequence of the altered cytidine deaminase possesses a specified amount of sequence similarity and/or sequence identity compared to the reference cytidine deaminase.

Structural similarity of two amino acid sequences can be determined by aligning the residues of the two sequences (for example, a candidate altered cytidine deaminase and a reference cytidine deaminase described herein) to optimize the number of identical amino acids along the lengths of their sequences; gaps in either or both sequences are permitted in making the alignment in order to optimize the number of identical amino acids, although the amino acids in each sequence must nonetheless remain in their proper order. A candidate altered cytidine deaminase is the cytidine deaminase being compared to the reference cytidine deaminase. A candidate altered cytidine deaminase that has structural similarity with a reference cytidine deaminase and cytidine deaminase activity is an altered cytidine deaminase.

Unless modified as otherwise described herein, a pair-wise comparison analysis of amino acid sequences can be conducted, for instance, by the local homology algorithm of Smith and Waterman, 1981, Adv. Appl. Math; 2:482, by the homology alignment algorithm of Needleman and Wunsch, 1907, J Mol Biol; 48:443, by the search for similarity method of Pearson and Lipman, 1988, Proc Nat'l Acad Sci USA; 85:2444, by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Current Protocols in Molecular Biology, Ausubel et al., eds., Current Protocols, a joint venture between Greene Publishing Associates, Inc. and John Wiley & Sons, Inc., supplemented through 2004). One example of an algorithm that is suitable for determining structural similarity is the BLAST^{®} algorithm, which is described in Altschul et al., 1990, J Mol Biol; 215:403-410. The BLAST^{®} algorithm can be used to calculate percent sequence identity and percent sequence similarity between two sequences. Software for performing BLAST^{®} analyses is publicly available through the National Center for Biotechnology Information.

In the comparison of two amino acid sequences, structural similarity may be referred to by percent "identity" or may be referred to by percent "similarity." "Identity" refers to the presence of identical amino acids. "Similarity" refers to the presence of not only identical amino acids but also the presence of conservative substitutions. Thus, in one embodiment the amino acid sequence of a cytidine deaminase protein having sequence similarity to a reference sequence may include conservative substitutions of amino acids present in that reference sequence.

A conservative substitution for an amino acid in a protein may be selected from other members of the class to which the amino acid belongs. For example, it is well-known in the art of protein biochemistry that an amino acid belonging to a grouping of amino acids having a particular size or characteristic (such as charge, hydrophobicity, or hydrophilicity) can be substituted for another amino acid without altering the activity of a protein, particularly in regions of the protein that are not directly associated with biological activity. For example, amino acids having a non-polar side chain include alanine, glycine, isoleucine, leucine, methionine, phenylalanine, proline, tryptophan, and valine; amino acids having a hydrophobic side chain include glycine, alanine, valine, leucine, isoleucine, proline, phenylalanine, methionine, and tryptophan; amino acids having a polar side chain include arginine, asparagine, aspartic acid, glutamine, glutamic acid, histidine, lysine, serine, cysteine, tyrosine, and threonine; and amino acids having an uncharged side chain include glycine, serine, cysteine, asparagine, glutamine, tyrosine, and threonine.

Thus, as used herein, reference to a cytidine deaminase as described herein, such as reference to the amino acid sequence of one or more SEQ ID NOs described herein can include a protein with at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% amino acid sequence similarity to the reference cytidine deaminase. Examples of altered cytidine deaminases having similarity with a reference amino acid sequence includes those having, for instance, at least 80%, at least 85%, at least 90%, or at least 95% similarity with SEQ ID NO: 5 and having an alanine at amino acid 130. Other examples of altered cytidine deaminases having similarity with a reference amino acid sequence includes those having, for instance, at least 80%, at least 85%, at least 90%, or at least 95% similarity or identity with SEQ ID NO: 6 and having an alanine at amino acid 130 and a histidine at amino acid 132.

Alternatively, as used herein, reference to a cytidine deaminase as described herein, such as reference to the amino acid sequence of one or more SEQ ID NOs described herein can include a protein with at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% amino acid sequence identity to the reference cytidine deaminase. Examples of altered cytidine deaminases having identity with a reference amino acid sequence includes those having, for instance, at least 80%, at least 85%, at least 90%, or at least 95% similarity with SEQ ID NO: 5 and having an alanine (A) at amino acid 130. Other examples of altered cytidine deaminases having identity with a reference amino acid sequence includes those having, for instance, at least 80%, at least 85%, at least 90%, or at least 95% similarity or identity with SEQ ID NO: 6 and having an alanine (A) at amino acid 130 and a histidine (H) at amino acid 132.

An altered cytidine deaminase of the present disclosure may include a substitution mutation at a position functionally equivalent to tyrosine at position 130 (Y130) in a member of the APOBEC3A subfamily. Accordingly, an alignment can be produced using a member of the APOBEC3A subfamily and another candidate altered cytidine deaminase from the APOBEC3A subfamily or a different APOBEC subfamily. In one embodiment, the candidate is selected from APOPEC subfamilies APOBEC1 or AID. An example of an algorithm that can be used to produce an alignment is Clustal O. In some APOBEC family proteins, the wild type residue at a position functionally equivalent to Y130 is phenylalanine (F).

In another embodiment, an altered cytidine deaminase of the present disclosure includes a substitution mutation at a position functionally equivalent to the tyrosine (Y) of ZDD motif HXEX₂₄SW(S/T)PCX_{[2-4]}CX₆FX₈LX₅R(L/I)YX_{[8-11]}LX₂LX_{[10]}M (SEQ ID NO: 2) in a member of the APOBEC family, such as a member of the APOBEC3A subfamily. The underlined tyrosine (Y) of SEQ ID NO: 2 is the position functionally equivalent to the tyrosine amino acid 130 of the wild type APOBEC3A protein (SEQ ID NO: 12).

In one embodiment, the substitution mutation at a position functionally equivalent to Y130 increases cytidine deaminase activity and preferentially acts on 5mC compared to cytosine (i.e., has cytosine-defective deaminase activity). The substitution mutation can be a mutation to alanine (A), glycine (G), phenylalanine (F), histidine (H), glutamine (Q), methionine (M), asparagine (N), lysine (K), valine (V), aspartic acid (D), glutamic acid (E), serine (S), cysteine (C), proline (P), or threonine (T). For example, the altered cytidine deaminase can comprise SEQ ID NO: 9, wherein X is selected from A, G, F, H, Q, M, N, K, V, D, E, S, C, P or T (and is not Y), or can comprise SEQ ID NO: 10, wherein Z is selected from A, G, F, H, Q, M, N, K, V, D, E, S, C, P or T (and is not Y), preferably, in one embodiment, X or Z is A or L. In an exemplary aspect of this embodiment, the substitution mutation at a position functionally equivalent to Y130 is a mutation to alanine (A), (e.g., SEQ ID NO: 5). Specific examples of altered cytidine deaminases having increased activity and preferentially acting on 5mC compared to cytosine include SEQ ID NO: 5 or a sequence having at least 90%, at least 95%, at least 98%, at least 99% sequence identity to SEQ ID NO: 5 and comprising Y130A.

An altered cytidine deaminase of the present disclosure having cytosine-defective deaminase activity (i.e., converts 5mC to T at a greater rate than converting C to U) optionally includes a second substitution mutation at a position two, three, four, or five amino acids on the C-terminal side of the Y130 position, or functionally equivalent to the Y130 position. In one embodiment, the second mutation is a tyrosine (Y), tryptophan (W), cysteine (C), histidine (H), or phenylalanine (F) at a position two, three, four, or five amino acids on the C-terminal side of the Y130 position, or functionally equivalent to the Y130 position. In one embodiment, the second mutation is at a position functionally equivalent to tyrosine at position 132 (Y132) in a member of the APOBEC3A subfamily. An APOBEC protein, such as an APOBEC3A protein, containing substitution mutations at both the first site, a position functionally equivalent to Y130, and the second site, at a position two, three, four, or five amino acids on the C-terminal side of the Y130 position, increases the preferential activity to act on 5mC compared to the same APOBEC protein, such as an APOBEC3A protein, containing one substitution mutation at Y130. In one embodiment, the substitution mutation at the second position is an amino acid having a positively charged side chain and selected from arginine (R), histidine (H), lysine (L), or a polar side chain selected from glutamine (Q). In one embodiment, the substitution mutation at the second position is histidine (H), such as Y132 to histidine. The double mutant containing both first and second mutations can be any substitution mutation at a position functionally equivalent to Y130 described herein and any second substitution mutation at a position two, three, four, or five amino acids on the C-terminal side of the Y130 position described herein, in any combination. For example, the altered cytidine deaminase can be, for example, SEQ ID NO: 4 and have a substitution at Y130 and Y132, or the position functionally equivalent to Y130 and Y132 as described herein. One example of an altered cytidine deaminase is SEQ ID NO: 11 comprising Y130X and Y132Z, where X is selected from (A), (L), or (W) (preferably (A)), and Z is selected from (R), (H), (L), or (Q), preferably (H). This encompasses examples including, but not limited to, for example Y130A and Y132R, Y130A and Y132H, Y130A and Y132L, Y130A and Y132Q, Y130L and Y132R, Y130L and Y132H, Y130L and Y132L, Y130L and Y132Q, Y130W and Y132R, Y130W and Y132H, Y130W and Y132L, Y130W and Y130Q, or any suitable combinations therein. In one embodiment, the double mutant includes substitution mutations Y130A and Y132H. Specific examples of altered cytidine deaminases having both substitution mutations and preferentially acting on 5mC compared to the APOBEC protein having just the single substitution mutation at cytosine include SEQ ID NO: 6 or a sequence having at least 90%, at least 95%, at least 98%, at least 99% sequence identity to SEQ ID NO: 6 and comprising Y130A and Y132H.

The person of ordinary skill in the art can confirm the 5mC preferential deaminase activity of the arginine, glutamine, histidine, and lysine substitution mutations at the second position in the double mutants described above. For example, double mutants can be constructed to create an altered cytidine deaminase having a first substitution mutation at a position functionally equivalent to Y130 and a second arginine, glutamine, histidine, or lysine substitution mutation at the tyrosine position two amino acids on the C-terminal side of the Y130 position, and then evaluated for deamination of C residues in one assay and deamination of 5mC residues in a second assay. Using an assay such as the SwaI-based assay described herein, the ratio of 5mC deamination and C deamination can be compared to identify those double mutants that preferentially deaminate 5mC compared to C. One of ordinary skill in the art could similarly test double mutants having a tyrosine at a position three, four or five positions C-terminal to the position functionally equivalent to Y130 and confirm that a substitution mutation at that position to arginine, glutamine, histidine, or lysine, in combination with a mutation at the position functionally equivalent to Y130 (such as Y130A), as double mutants that preferentially deaminate 5mC compared to C.

Some embodiments presented herein relate to substitution mutations that result in 5mC-defective deaminase activity (i.e., converts C to U at a greater rate than converting 5mC to T). In one embodiment, the substitution mutation at a position functionally equivalent to Y130 increases cytidine deaminase activity and preferentially acts on cytosine compared to 5mC and is a mutation to an amino acid having a non-polar side chain or a hydrophobic side chain, such as leucine (L) or tryptophan (W). In an exemplary aspect of this embodiment, the substitution mutation at a position functionally equivalent to Y130 is a mutation to leucine. Other examples of mutations that result in increased preferential deamination activity on cytosine compared to 5mC include a single mutant with Y132P, and double mutants with a substitution mutation at Y130V and Y132H, or Y130W and Y132H. Specific examples of altered cytidine deaminases having increased cytidine deaminase activity and preferentially acts on cytosine compared to 5mC include SEQ ID NO: 7 or a sequence having at least 90%, at least 95%, at least 98%, at least 99% sequence identity to SEQ ID NO: 7 and comprising Y130L.

In one embodiment, the substitution mutation is at a position functionally equivalent to Y130 that results in 5hmC-defective deaminase activity (i.e., preferentially deaminates C and 5mC to U and T, respectively, and has significantly reduced deamination of 5hmC). In an exemplary aspect of this embodiment, the substitution mutation at a position functionally equivalent to Y130 is a mutation to an amino acid having a non-polar side chain or a hydrophobic side chain, such as tryptophan (W). Specific examples of altered cytidine deaminases having the ability to deaminate C and 5mC to U and T, respectively, but reduced ability to deaminate 5hmC, preferably no detectable ability to deaminate 5hmC include SEQ ID NO: 8 or a sequence having at least 90%, at least 95%, at least 98%, at least 99% sequence identity to SEQ ID NO: 8 and comprising Y130W.

In some embodiments, an altered cytidine deaminase includes a substitution mutation at a position functionally equivalent to tyrosine at position 130 (Y130) and/or tyrosine position 132 (Y132) in a member of the APOBEC3A subfamily. In some embodiments, such an altered cytidine deaminase demonstrates selective deamination for mC.

In some embodiments, an altered cytidine deaminase is an altered APOBEC3A cytidine deaminase, altered to include a substitution mutation at tyrosine at position 130 (Y130) and/or tyrosine position 132 (Y132). In some embodiments, such an altered APOBEC3A cytidine deaminase demonstrates selective deamination for mC.

In some embodiments, an altered cytidine deaminase is a double mutant of APOBEC3A, with substitution mutations Y130A/Y132H. In some embodiments, such an altered APOBEC3A cytidine deaminase demonstrates selective deamination for mC.

In some embodiments, an altered cytidine deaminase includes an altered cytidine deaminase having an amino acid of SEQ ID NO: 5, SEQ ID NO: 6, SEQ ID NO: 7, SEQ ID NO: 8, SEQ ID NO: 9, SEQ ID NO: 10, or SEQ ID NO: 11. In some embodiments, such an altered APOBEC3A cytidine deaminase demonstrates selective deamination for mC.

An altered cytidine deaminase described herein can include additional mutations. Typically, additional mutations do not unduly alter the activity of the altered cytidine deaminase. One or more additional mutations can be a conservative mutation.

An altered cytidine deaminase described herein can be a truncated protein. A truncated protein is a fragment of an altered cytidine deaminase of the present disclosure that retains the ability to deaminate 5mC to thymidine. A truncated altered cytidine deaminase can include a deletion of 1 to 13 amino acids on the N-terminal end of the protein, a deletion of 1 to 3 amino acids on the C-terminal end of the protein, or a combination thereof.

In some embodiments, an altered cytidine deaminase includes any of those described in International Patent Application No. PCT/US2023/017846 ("Altered Cytidine Deaminases and Methods of Use"), filed April 7, 2023.

In general, methods for using a cytidine deaminase include contacting target nucleic acids, e.g., DNA or RNA, with the enzyme, under conditions suitable for conversion of modified cytidines, such as 5mC, to thymidine, or for conversion of unmodified cytidine to uracil. Because amplification of DNA does not preserve the modification status of cytidine (e.g., the methylation status of 5mC is not retained), use of a cytidine deaminase typically occurs before amplification of target DNA. Target nucleic acids can be contacted with cytidine deaminase at essentially any time. For instance, target nucleic acids can be contacted with cytidine deaminase after isolation of genomic or cell free DNA or mRNA, before or after fragmentation, or before or after tagmentation. The skilled person will recognize that target nucleic acids can be contacted with a cytidine deaminase after addition of a universal sequence and/or an adapter, provided the universal sequence and/or an adapter is not added by amplification.

Reaction conditions suitable for conversion of modified cytidines, such as 5mC, to thymidine by a cytidine deaminase include, but are not limited to, a substrate of target nucleic acid suspected of including at least one modified cytidine, with appropriate pH, temperature of the reaction, time of the reaction, and concentration of the cytidine deaminase and/or DNA or RNA substrate. It is expected that a cytidine deaminase can function in essentially any buffer. Examples of useful buffers include, but are not limited to, a citrate buffer, such as the citrate buffer available from Thermo Fisher Scientific (Cat. No. #005000); sodium acetate buffer, Bis Tris-Propane HCl; and Tris-HCl Tris. Examples of other buffers include, but are not limited to, Bicine, DIPSO, glycylglycine, HEPES, imidazole, malonate, MES, MOPS, PB, phosphate, PIPES, SPG, succinate, TAPS, TAPSO, trincine. Cytidine deaminases typically function at near-neutral pH, e.g., pH 7. In some embodiments a reducing agent such as dithiothreitol (DTT) can be present. In some embodiments a divalent cation is not included. A deamination reaction can occur at a temperature of about 25°C to about 60°C, including but not limited to, at about 37°C, at about 45°C, at about 50°C, and at about 60°C.

Some cytidine deaminases preferentially deaminate a modified cytosine to thymidine at a faster rate than deamination of cytosine to uracil. Thus, in some embodiments the time of reaction can be used to allow the reaction to run to completion, to maximize the difference of deamination of modified cytosine versus deamination of cytosine. In some embodiments, the reaction can proceed for at least 15 minutes, at least 30 minutes, at least 45 minutes, at least 60 minutes, at least 90 minutes, at least 120 minutes, or at least 150 minutes, and for no greater than 15 minutes, no greater than 30 minutes, no greater than 45 minutes, no greater than 60 minutes, no greater than 90 minutes, no greater than 120 minutes, no greater than 150 minutes, or no greater than 180 minutes. In some embodiments, the reaction can run overnight.

In some embodiments, a deamination reaction can include a cytidine deaminase at a concentration from at least about 25 nanomolar (nM) to no greater than about 5 micromolar (µM). For instance, the concentration of the enzyme can be at least about 25 nM, at least about 0.5, at least about 1 µM, at least about 2µM, at least about 3 µM, at least about 4 µM, or at least about 5 µM, and/or no greater than 5 µM, no greater than 4 µM, no greater than 3µM, no greater than 2 µM, no greater than 1 µM, or 0.5 µM. In one embodiment, a deamination reaction can include nucleic acids at a concentration of at least 400 nanomolar (nM) to no greater than 2 µM. For instance, the concentration of nucleic acids can be at least 400 nM, at least 500 nM, at least, 600 nM, at least 700 nM, at least 800 nM, at least 900 nM, or 1µM, and/or no greater than 1 µM, no greater than 900 nM, no greater than 800 nM, no greater than 700 nM, no greater than 600 nM, no greater than 500 nM, or 400 nM.

With the methods described herein, after a preparation of single-stranded DNA (ssDNA) fragments has been treated with a cytidine deaminase, it is then contacted with an Uracil-DNA-glycosylase. Uracil-DNA-glycosylase (UDG), also known as Uracil-N-glycosylase (UNG), is a highly conserved repair enzyme that catalyzes the excision of uracil from uracil-containing single- and double-stranded DNA but is inactive to RNA. It is a monomeric protein with relatively stable physicochemical properties, a small molecular weight of 25KDa, and is widely present in various prokaryotic and eukaryotic organisms. See, for example, Hölz et al., 2019, Scientific Reports; 9:17822; Schormann et al., 2014, Protein Sci; 23:1667-1685; Zharkov et al., 2010, Mutation Research 685, 11-20; Stivers et al., 2001, Arch Biochem Biophys; 396, 1-9; Parikh et al., 2000, Proc Natl Acad Sci USA; 97:5083; Pearl, 2000, Mutat. Res. 460, 165-181; Lindahl, 1982, Annu Rev Biochem; 51:61-87; and Lindahl et al., 1977, J Biol Chem; 252:3286-3294.

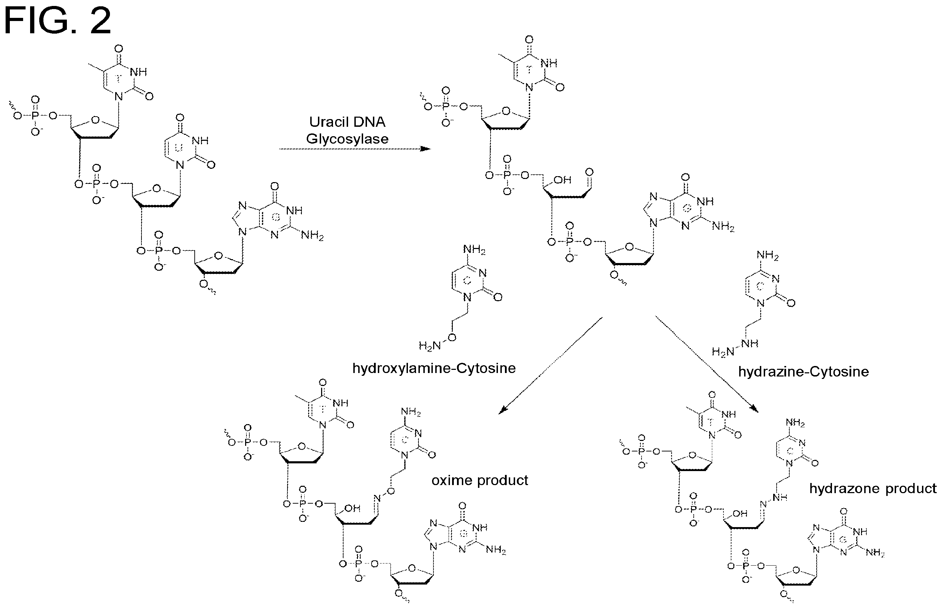

UDG excises uracil from DNA by hydrolyzing the N-glycoside bond between the uracil base and the sugar-phosphate backbone in single- and double-stranded DNA (Bellamy et al., 2007, Nucleic Acids Res; 35:1478-1487; Slupphaug et al., 1996, Nature 384, 87-92; Stivers et al., 1999, Biochemistry; 38:952-963; and Parikh et al., 2000, Mutat Res; 460:183-199), resulting in the formation of an abasic site (AP-site) having a hemiacetal formation. A schematic illustration of the UDG-mediated generation of single nucleotide gaps within single stranded DNA fragments is shown in FIG. 2. Because false positive (cytosine) deamination results in uracil bases, and true positive (methylcytosine) bases result in thymine bases, UDG can be utilized to specifically recognize and remove uracil bases, thus removing the false positive signal and preventing its propagation as a "T" in downstream amplification and sequencing. APOBEC enzymes require ssDNA for recognition, and thus deaminated DNA will be single stranded.

A variety of UDG enzymes are commercially available, including, for example, *E. coli* Uracil-DNA Glycosylase (UDG) (New England Biolabs, Catalog # M0280S, see the worldwide web at neb.com/products/m0280-uracil-dna-glycosylase-udg#Product%20Information) and a heat-labile Uracil DNA Glycosylase (UDG/UNG) isolated from a psychrophilic marine bacteria (Yeasen Biotechnology (Shanghai) Co.,Ltd., Catalog #10707ES, see the worldwide web at yeasenbiotech.com/solutiondetail/79?gelid=EAIaIQobChMI_Oie4unY-gIV3xCtBh0hRwGHEAAYASAAEgKsx_D_BwE). In some embodiments, the UDG is of commercial origin.

Reaction conditions suitable for the UDG-mediated excision of uracil from DNA include, but are not limited to, concentration of the single stranded DNA substrate, pH, temperature of the reaction, time of the reaction, and concentration of the UDG enzyme. It is expected that a UDG can function in essentially any buffer. An example of a useful buffer includes, but is not limited to, 1X UDG Reaction Buffer (New England Biolabs, Catalog # B0280S, see the worldwide web at neb.com/products/m0280-uracil-dna-glycosylase-udg#Product%20Information) which is 20 mM Tris-HCl, 1mM DTT, 1mM EDTA (pH 8 at 25°C). Uracil-DNA Glycosylase is active over a broad pH range, with an optimum at pH 8.0, does not require a divalent cation, and is inhibited by high ionic strength (> 200 µM). Uracil-DNA Glycosylase is active in a temperature of 25°C to 37°C and in some embodiments, the reaction can proceed in a temperature of 25°C to 37°C. In some embodiments, the reaction can proceed at 37°C. In some embodiments, the reaction can proceed for about 5 minutes, about 10 minutes, about 15 minutes, about 20 minutes, about 30 minutes, about 45 minutes, about 60 minutes, about 90 minutes, about 120 minutes, or any range thereof. In some embodiments, a reaction can include about 0.001 U/uL to about 1 U/uL, wherein one unit (U) is defined as the amount of enzyme that catalyzes the release of 60 pmol of uracil per minute from double-stranded, uracil-containing DNA. Activity is measured by release of [³H]-uracil in a 50 µl reaction containing 0.2 µg DNA (10⁴-10⁵ cpm/µg) in 30 minutes at 37°C (see the worldwide web at neb.com/products/m0280-uracil-dna-glycosylase-udg#Product%20Information). In some embodiments, a reaction can include about 0.001 U/uL, about 0.05 U/uL, or about 1 U/uL UDG.

In some embodiments, a reaction can include nucleic acids at a concentration of at least about 10 (picomolar (pM) to about 400 nanomolar (nM). For instance, the concentration of nucleic acids can be at least 200 nm, at least 400 nM, at least 500 nM, at least, 600 nM, at least 700 nM, at least 800 nM, at least 900 nM, or 1µM, and/or no greater than 1 µM, no greater than 900 nM, no greater than 800 nM, no greater than 700 nM, no greater than 600 nM, no greater than 500 nM, or 400 nM.

In some embodiments, reaction conditions include incubating 70 uM of U containing 15-mer or 10-mer with 25 U of UDG (NEB) in NEB buffer at 37°C for one hour.

As shown in FIG. 1, the enzymatic treatment of single stranded DNA fragments with Uracil DNA-glycosylase specifically deglycosylates uracil residues, forming abasic sites. The preparation of single stranded DNA fragments may then be treated with a reactive cytosine nucleobase analog to install the reactive cytosine nucleobase analog at the abasic sites through a noncanonical linkage. The reactive cytosine nucleobase analog then functions during amplification with a polymerase as a cytosine template base, resulting in corrected DNA fragments in which false positive uracil residues have been corrected to cytosine. This is shown schematically in FIG. 1.

As shown in FIG. 2, the abasic sites resulting from UDG enzymatic treatment bear an aldehyde functional group, which can subsequently be treated with a reactive cytosine nucleobase analog bearing a reactive functional group to install a cytosine base. Reactive cytosine nucleobase analogs include, but are not limited to, hydroxylamine-cytosine, which results in a oxime linkage to install the cytosine base, and hydrazine-cytosine, which results in a hydrazone linkage, to install the cytosine base (see the worldwide web at thermofisher. com/us/en/home/references/molecular-probes-the-handbook/reagents-for-modifying-groups-other-thanthiols-or-amines/hydrazines- hydroxylamines-and-aromatic-amines-for-modifying-aldehydes-and-ketones.html; Kalia and Raines, 2008, Angew Chem Int Ed Engl; 47(39):7523-7526; and US 2022/0090179 A1).

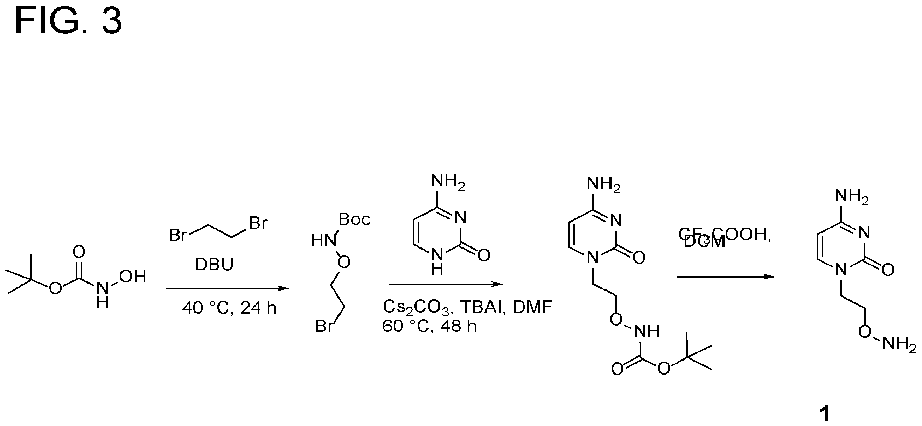

A reactive cytosine nucleobase analog for use in the methods described herein includes, but is not limited to, a hydroxylamine-cytosine derivative, a hydrazine-cytosine derivative, or a hydrazide-cytosine derivative. The pathways for the synthesis of these reactive cytosine nucleobase analog are shown as pathways #1, #3, and #2 of FIG. 3, respectively.

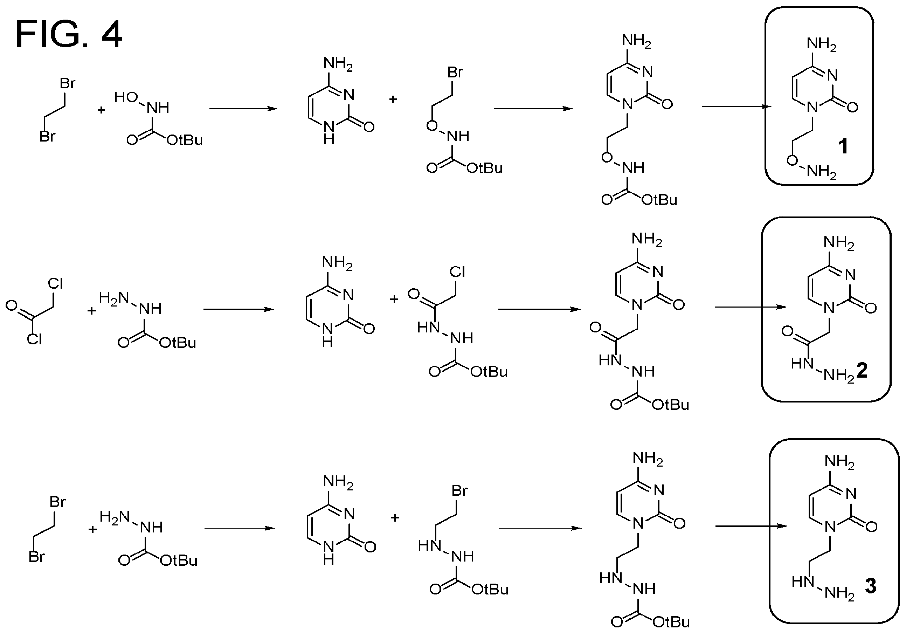

Reactive cytosine nucleobase analogs may be readily synthesized and representative synthetic schemes are shown in FIG. 3 and FIG. 4. Representative chemical reactions for the synthesis of hydroxylamine aldehyde reactive cytosine analogs is shown as cytosine analogue #1 in FIG. 3 and FIG. 4 and described in more detail in, for example, Kubo et al., 1992, Biochemistry; 31(14):3703-3708; Bennett and Kitner, 2006, Nucleosides Nucleotides Nucleic Acids; 25(7):823-42; Wei et al., 2019, DNA Repair (Amst); 27:9-18; and Wilson and Kool, 2019, J Am Chem Soc; 141(49):19379-19388. Representative chemical reactions for the synthesis of a hydrazide reactive cytosine analog is shown as cytosine analogue #2 in FIG. 4 and described in more detail in, for example, Zhang et al., 2019, Mol Cell; 74:1304-1316.e8. Representative chemical reactions for the synthesis of a hydrazine reactive cytosine analog is shown as cytosine analogue #3 in FIG. 4 and described in more detail in, for example, Melton et. al., 2014. Chem Res Toxicol; 27:2113-2118; and Gamboa Varela et. al., 2015, Angew Chem Int Ed Engl; 54(26): 7666-7669.

In some embodiments, 5-10 mM of a hydroxylamine-cytosine derivative (cytosine analogue #1 of FIGS 3 and 4) may be incubated with 50 mM MES at pH 6.0 at 37°C for two hours.

In some embodiments, 5-10 mM of a hydrazide-cytosine derivative (cytosine analogue #2 of FIG. 4) may be incubated with 100 mM MES at pH 4.5 at 55°C for one hour.

In some embodiments, 5-10 mM of a hydroxylamine-cytosine derivative (cytosine analogue #1 of FIGS. 3 and 4) may be incubated with 50-100 mM Tris buffer at pH 7-8 at a variety of temperatures and times.

In some embodiments, the UDG enzymatic step and the chemical step of adding a cytosine analog may be carried out separately and sequentially.

In some embodiments, the UDG enzymatic step and the chemical step of adding a cytosine analog may be carried out step simultaneously in the same reaction mixture (see, for example, Jun et al., 2022, Nat Commun; 13:5043 and Wilson and Kool, 2019, J Am Chem Soc; 141(49):19379-19388).

After the treatment of single stranded DNA fragments with an Uracil DNA Glycosylase (UDG) to selectively de-glycosylate uracil residues and conjugation with a reactive cytosine nucleobase analog, such as for example a hydroxylamine or a hydrazine, the resulting corrected single stranded DNA fragments may be amplified (FIG. 1). DNA molecules with such reactive cytosine nucleobase analogs amplify successfully using standard PCR polymerases (Wang et al., 2021, ACS Central Science; 7(6):973-79). Thus, with amplification, reactive cytosine nucleobase analogs, installed through a noncanonical linkages, act as cytosines, resulting in double stranded fragments where false positive uracil transformations have been effectively corrected back to cytosine, providing double stranded corrected DNA fragments. In some embodiments, U-intolerant polymerases may be employed to provide greater assay specificity.

It will be appreciated that any of the amplification methodologies described herein or generally known in the art may be used with universal or target-specific primers to amplify DNA fragments. Suitable methods for amplification include, but are not limited to, the polymerase chain reaction (PCR), strand displacement amplification (SDA), transcription mediated amplification (TMA) and nucleic acid sequence-based amplification (NASBA), as described in U.S. Pat. No. 8,003,354. The above amplification methods may be employed to amplify one or more nucleic acids of interest. For example, PCR, including multiplex PCR, SDA, TMA, NASBA and the like may be utilized to amplify DNA fragments. In some embodiments, primers directed specifically to the polynucleotide of interest are included in the amplification reaction.

As used herein, "amplify," "amplifying" or "amplification reaction" and their derivatives, refer generally to any action or process whereby at least a portion of a nucleic acid molecule is replicated or copied into at least one additional nucleic acid molecule. The additional nucleic acid molecule optionally includes sequence that is substantially identical or substantially complementary to at least some portion of the target nucleic acid molecule. The target nucleic acid molecule can be single-stranded or double-stranded and the additional nucleic acid molecule can independently be single-stranded or double-stranded. Amplification optionally includes linear or exponential replication of a nucleic acid molecule. In some embodiments, such amplification can be performed using isothermal conditions; in other embodiments, such amplification can include thermocycling. In some embodiments, the amplification is a multiplex amplification that includes the simultaneous amplification of a plurality of target sequences in a single amplification reaction. In some embodiments, "amplification" includes amplification of at least some portion of DNA and RNA based nucleic acids alone, or in combination. The amplification reaction can include any of the amplification processes known to one of ordinary skill in the art. In some embodiments, the amplification reaction includes polymerase chain reaction (PCR).

As used herein, "amplification conditions" and its derivatives, generally refers to conditions suitable for amplifying one or more nucleic acid sequences. Such amplification can be linear or exponential. In some embodiments, the amplification conditions can include isothermal conditions or alternatively can include thermocyling conditions, or a combination of isothermal and thermocycling conditions. In some embodiments, the conditions suitable for amplifying one or more nucleic acid sequences include polymerase chain reaction (PCR) conditions. Typically, the amplification conditions refer to a reaction mixture that is sufficient to amplify nucleic acids such as one or more target sequences, or to amplify an amplified target sequence ligated to one or more adapters, e.g., an adapter-ligated amplified target sequence.

Generally, the amplification conditions include a catalyst for amplification or for nucleic acid synthesis, for example a polymerase; a primer that possesses some degree of complementarity to the nucleic acid to be amplified; and nucleotides, such as deoxyribonucleotide triphosphates (dNTPs) to promote extension of the primer once hybridized to the nucleic acid. The amplification conditions can require hybridization or annealing of a primer to a nucleic acid, extension of the primer and a denaturing step in which the extended primer is separated from the nucleic acid sequence undergoing amplification. Typically, but not necessarily, amplification conditions can include thermocycling; in some embodiments, amplification conditions include a plurality of cycles where the steps of annealing, extending, and separating are repeated. Typically, the amplification conditions include cations such as Mg++ or Mn++ and can also include various modifiers of ionic strength.

As used herein, the term "polymerase chain reaction" (PCR) refers to the method of K. B. Mullis U.S. Pat. Nos. 4,683,195 and 4,683,202, which describes a method for increasing the concentration of a segment of a polynucleotide of interest in a mixture of genomic DNA without cloning or purification. This process for amplifying the polynucleotide of interest consists of introducing a large excess of two oligonucleotide primers to the DNA mixture containing the desired polynucleotide of interest, followed by a series of thermal cycling in the presence of a DNA polymerase. The two primers are complementary to their respective strands of the double-stranded polynucleotide of interest. The mixture is denatured at a higher temperature first and the primers are then annealed to complementary sequences within the polynucleotide of interest molecule. Following annealing, the primers are extended with a polymerase to form a new pair of complementary strands. The steps of denaturation, primer annealing, and polymerase extension can be repeated many times (referred to as thermocycling) to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as the "polymerase chain reaction" (hereinafter "PCR"). Because the desired amplified segments of the polynucleotide of interest become the predominant nucleic acid sequences (in terms of concentration) in the mixture, they are said to be "PCR amplified." In a modification to the method discussed above, the target nucleic acid molecules can be PCR amplified using a plurality of different primer pairs, in some cases, one or more primer pairs per target nucleic acid molecule of interest, thereby forming a multiplex PCR reaction.

In some embodiments, after the treatment of single stranded DNA fragments with an Uracil DNA Glycosylase (UDG) to selectively de-glycosylate uracil residues and conjugation with a reactive cytosine nucleobase analog, the corrected single stranded DNA fragments are not subject to PCR amplification prior to sequencing.

In some embodiments, the double stranded corrected DNA fragments obtained with amplification of corrected single stranded DNA fragments may be sequenced. Sequencing may be by any of a variety of known methodologies, including, but not limited to any of a variety high-throughput, next generation sequencing (NGS) platforms, including, but not limited to, sequencing by synthesis, sequencing by ligation, nanopore sequencing, Sanger sequencing, and the like. In some embodiments, sequencing is performed using the sequencing by synthesis methodologies commercialized by ILLUMINA^{®} as described in U.S. Patent Application Publication No. 2007/0166705, U.S. Patent Application Publication No. 2006/0188901, U.S. Pat. No. 7,057,026, Beijing Genomics Institute (BG) as described in Carnevali et al., 2012, J Comput Biol; 9(3):279-92 (doi: 10.1089/cmb.2011.0201. Epub 2011 Dec 16), or the ion semiconductor sequencing methodologies of ION TORRENT^{™} as described in US 2009/0026082 A1; US 2009/0127589 A1; US 2010/0137143 A1; or US 2010/0282617 A1.

Next Generation Sequencing (NGS) refers to sequencing methods that allow for massively parallel sequencing of clonally amplified molecules and of single nucleic acid molecules. Non-limiting examples of NGS include sequencing-by-synthesis using reversible dye terminators, and sequencing-by-ligation.

In some embodiments, sequencing-by-synthesis (SBS) techniques are utilized. SBS techniques generally involve the enzymatic extension of a nascent nucleic acid strand through the iterative addition of nucleotides against a template strand.

In some embodiments, repaired fragments are cloned, followed by Sanger sequencing of clones to assess methylation.

In addition to preventing false positive detection of 5-methylcytosine (5mC) and/or 5-hydroxymethylcytosine (5hmC) due to the deamination of unmethylated cytosines in an assay using a cytosine deaminase to selectively deaminate methylated cytosines, the Chemoenzymatic Uracil Replacement of Nucleobases (CHURN) methods described herein have additional applications.

The CHURN methods described herein replace uracil and/or abasic sites with cytosine (or another nucleobase functionalized with the correct hydroxylamine or hydrazine linkage) and can be applied to situations where efficient amplification of long DNA targets is required. PCR amplification of long targets (≥ 5 - 6 kbp) can be difficult due to various factors affecting the processivity of thermostable polymerases (Barnes, 1994, Proc Natl Acad Sci U S A; 91:2216-2220). A key impediment to long-range PCR is the prolonged heat exposure of template DNA and dNTPs to heat during thermal cycling conditions required for amplification of kilobase-long targets. Barnes (Barnes, 1994, Proc Natl Acad Sci U S A; 91:2216-2220) proposed single-stranded template DNA present in PCR is highly susceptible to depurination events, resulting in abasic sites which cannot be traversed by many polymerases. Hogrefe et al. (Hogrefe et al., 2022, Proc Natl Acad Sci U S A; 99:596-601) noted spontaneous deamination of dCTP to dUTP during PCR leads to mis-incorporation and accumulation of uracil into amplicons, which will then inhibit proofreading polymerases such as *Pfu* and other B-family polymerases (Greagg et al., 1999, Proc Natl Acad Sci U S A; 96:9045-9050). Additional thermal damage to the template, such as cytosine deamination to uracil, further inhibit PCR efficiency and introduce additional sequence errors (Pienaar et al., 2006, Comput Biol Chem; 30:102-111).

To mitigate the accumulation of numerous cytosine to uracil lesions in template DNA during PCR of long DNA templates, the CHURN methods described herein can be employed to correct errors before resuming PCR and aiding the efficient generation of long and accurate PCR amplicons. For example, a single stranded sample of the template DNA may be contacted with an uracil DNA glycosylase (UDG) to deglycosylate uracil residues to form abasic sites having a hemiacetal formation within the single stranded DNA and then contacted with a reactive cytosine nucleobase analog to install a cytosine at abasic sites thru a noncanonical linkage. Following these treatments, polymerase chain reaction (PCR) amplification of the of the corrected template may be resumed. In some embodiment, long a DNA template is at least about 5 to 6 kilobases (kbp) or greater in length.

The CHURN methods described herein can replace uracil residues with cytosine residues in the absence of double-stranded DNA and can be used to provide for accurate and comprehensive genomic studies of formalin-fixed paraffin embedded (FFPE) material. A major source of sequencing error and noise in the study of FFPE tissues is cytosine deamination (Chen et al., 2014, Mol Diagn Ther; 18:587-593). While there are on-market solutions (including, but not limited to New England Biolabs NEBNext^{®} FFPE DNA Repair Mix and Oxford Gene Technology SureSeq FFPE DNA Repair Mix) and published solutions (Chen et al., 2017, Science; 355:752-756) for repairing cytosine deamination in FFPE, they rely on repair methods dependent on dsDNA duplexes to template correct replacement of U (deaminated cytosine) with C. However, FFPE DNA is commonly partially single-stranded, either due to DNA damage due to fixation and storage or the process by which it is extracted from paraffin (Stiller et al., 2016, Oncotarget; 7:59115-59128). CHURN is distinct from alternative solutions in its ability to replace uracil with cytosine in the absence of double-stranded DNA and will provide for more accurate and comprehensive genomic studies of FFPE material.

The methods described herein may be used to selectively replace uracils due to the deamination of cytosines with cytosines in single-stranded DNA fragments obtained from formalin-fixed paraffin embedded (FFPE) genomic material. To replace uracil residues with cytosine residues, a sample of FFPE material including single stranded DNA may be contacted with an uracil DNA glycosylase (UDG) to deglycosylate uracil residues to form abasic sites having a hemiacetal formation within the single stranded DNA and then contacted with a reactive cytosine nucleobase analog to install a cytosine at abasic sites thru a noncanonical linkage. Following these treatments, subjecting the sample to polymerase chain reaction (PCR) amplification results double stranded corrected fragments DNA.

The CHURN methods described herein can be applied to enable the efficient amplification with proofreading polymerases and accurate sequencing by NGS methods of ancient DNA samples and forensic DNA samples. Cytosine deamination is a contributor to sources of error and inefficiency in the amplification and study of ancient DNA (Gilbert et al., 2007, Nucleic Acids Research; 35: 1-10; and Hofreiter et al., 2001, Nucleic Acids Research; 29:4793-4799) and forensic DNA analysis (Gorden et al., 2018, Forensic Sci Int Genet; 34:257-264). Similar to the application of CHURN to aid efficient amplification of long targets in PCR, CHURN may be applied to replace uracil with cytosine and enable both efficient amplification with proofreading polymerases and accurate sequencing by NGS methods of ancient DNA and/or forensic DNA samples.

The methods described herein may be used to selectively replace uracils due to the deamination of cytosines with cytosines in single-stranded DNA fragments obtained from ancient DNA and/or forensic DNA samples. To replace uracil residues with cytosine residues, a sample of ancient DNA and/or forensic DNA including single stranded DNA may be contacted with an uracil DNA glycosylase (UDG) to deglycosylate uracil residues to form abasic sites having a hemiacetal formation within the single stranded DNA and then contacted with a reactive cytosine nucleobase analog to install a cytosine at abasic sites thru a noncanonical linkage. Following these treatments, subjecting the sample to polymerase chain reaction (PCR) amplification results double stranded corrected fragments DNA.

The present disclosure also provides kits for undertaking a CHURN method as described herein, for the removal of uracils due to deamination of unmethylated cytosines in an assay using a cytosine deaminase to deaminate methylated cytosines. A kit includes at least one or more of a cytosine deaminase, an uracil DNA glycosylase (UDG), and/or a reactive cytosine nucleobase analog in a suitable packaging material in an amount sufficient for at least one reaction. A kit may include one or more other components. Examples of other components include, for example, a cytosine deaminase, a PCR polymerase, PCR master mix, suitable library primers, a DNA denaturation solution (such as for example, NaOH, formamide, or DMSO), a cytosine deaminase buffer, a UDG reaction buffer, a coupling buffer for reactive cytosine analog, DNA purification beads for purification steps, a positive control polynucleotide, such as a double-stranded DNA including one or more known modified cytosines for use in measuring efficiency, or a negative control polynucleotide, such as a double-stranded DNA including unmodified cytosines. Optionally, other reagents such as buffers and solutions are also included. Instructions for use of the packaged components are also typically included.

As used herein, the phrase "packaging material" refers to one or more physical structures used to house the contents of the kit. The packaging material is constructed by known methods, preferably to provide a sterile, contaminant-free environment. The packaging material has a label which indicates that the components can be used for the removal of uracils due to deamination of unmethylated cytosines in an assay using a cytosine deaminase to deaminate methylated cytosines. In addition, the packaging material contains instructions indicating how the materials within the kit are employed to practice a CHURN method as described herein. As used herein, the term "package" refers to a solid matrix or material such as glass, plastic, paper, foil, and the like, capable of holding within fixed limits the polypeptides. "Instructions for use" typically include a tangible expression describing the reagent concentration or at least one assay method parameter, such as the relative amounts of reagent and sample to be admixed, maintenance time periods for reagent/sample admixtures, temperature, buffer conditions, and the like.

### Uracil Enzymatic Removal and Substitution at Errors (U-ERASE)

The U-ERASE method described in the following passages is not encompassed by the wording of the claims.

With the Uracil Enzymatic Removal and Substitution at Errors (U-ERASE) method described herein, but not encompassed in the wording of the claims, the problem of false positive conversions of cytosines to uracils in cytosine deaminase based methylation detection assays is solved by the enzymatic correction of false positive uracils to cytosines. A schematic illustrating the Uracil Enzymatic Removal and Substitution at Errors (U-ERASE) method is shown in FIG. 5. Briefly, the U-ERASE methods described herein include the synthesis of a second strand tagged in a fashion that will facilitate its downstream degradation, the enzymatic removal of false positive uracil bases with uracil DNA glycosylase and enzymatic replacement with a mismatched base, such as cytosine, followed by the selective degradation or cleavage of the second strand used to facilitate enzymatic repair.

After treatment of a preparation of DNA fragments from an input sample with a cytosine deaminase to deaminate 5-methylcytosine (5mC) and/or 5-hydroxymethylcytosine (5hmC) residues and possibly including one or more off-target conversions of a cytosine to an uracil, the preparation of DNA fragments is treated with uracil DNA glycosylase (UDG) to selectively remove uracils. As deamination with a cytosine deaminase requires single stranded DNA (ssDNA), after deamination with a cytosine deaminase and prior to treatment with the uracil DNA glycosylase, a second complementary strand of DNA is synthesized, providing double stranded DNA (dsDNA) fragments. With the synthesis of these complementary strands, cytosines that have been converted to uracils (false positives) are copied as adenines in the second strand. In order to facilitate the downstream sequencing of only the original strand, this second strand of DNA, which serves as a scaffold for the enzymatic repair steps of the methods described herein, is marked in order to provide for its selective degradation in downstream steps, thereby facilitating the analysis of the original deaminase-treated single stranded DNA.

Double stranded DNA fragments are then treated with UDG, resulting in the removal of uracil bases from the first strand. Treatment with UDG is followed by treatment with an endonuclease. The endonuclease creates single nucleotide gaps at the sites of the removed uracil residues, resulting in double stranded DNA fragments with single nucleotide gaps at the sites of the removed uracil residues. This treatment with UDG and an endonuclease may be as separate steps or may be undertaken simultaneously in the same reaction mixture.

The preparation of double stranded DNA fragments with single nucleotide gaps at the sites of the removed uracil residues is then treated with a polymerase, a nucleotide (either dCTP, dITP, or other universal base), and a ligase, resulting in polymerase/ligase-mediated insertion of the nucleotide at the single nucleotide gaps at the sites of the removed uracil residues, providing repaired double stranded DNA fragments. After second strand synthesis and prior to polymerase/ligase-mediated insertion of dCTP, dITP, or other universal base, dNTPS may be removed from the preparation, so only that only the added nucleotides (either dCTP, dITP, or other universal base) are available for polymerase/ligase-mediated insertion into the repaired double stranded DNA fragments.

Subsequently, the second strand is selectively degraded or inactivated, allowing for the selective amplification of the original DNA strand, propagating the repaired/corrected DNA fragments, which may be followed by characterization by sequencing.

In some embodiments, not encompassed by the wording of the claims, for degradation of the second strand, second strand synthesis may be carried out using a 5'phosphorylated primer binding to the library adapter sequence at the 3' end. A variety of standard polymerases that tolerate uracil may be utilized in this step. The presence of a 5'phosphate on the second strand tags the second stand for its selective degradation later in the workflow using a lambda exonuclease. While lambda exonuclease can degrade DNA without a 5'phosphate, this occurs much slower than degrading DNA with a 5'phosphate. In order to improve selectivity of lambda exonuclease to degrade only the synthesized 2nd strand, during library prep, adapters with one or more phosphorothioate bonds can be utilized. This is shown in FIG. 6A.

In some embodiments not encompassed by the wording of the claims, an alternative strategy for the selective degradation of the second strand makes use of primers containing 8-oxoguanine and/or inosine residues to mark the second strand. One primer binds the 3' end of the library fragment and mediates polymerization of the library insert, while another primer binds the 5' end of the library fragment and is incorporated through ligation. A mixture of enzymes containing a polymerase (for example, an exonuclease-deficient Taq polymerase, which is uracil-tolerant) and a ligase (for example, Taq DNA ligase) may be used to generate the tagged second strand. The polymerase employed for this reaction is not required to have high fidelity, as the resulting strand is not sequenced. The presence of the 8-oxoguanine and/or inosine residues enable the selective cleavage of the adapter sequences later in the workflow using FPG/OGG, or Endonuclease V, respectively, rendering the second strand unamplifiable during PCR. This is shown in FIG. 6B.

With the methods described herein, the target nucleic acids (also referred to herein as "DNA fragments" or "a preparation of DNA fragments from an input sample") may be essentially any nucleic acid of known or unknown sequence.

Such target nucleic acids are typically derived from primary nucleic acids present in a sample, such as a biological sample. The primary nucleic acids may originate as DNA or RNA. DNA primary nucleic acids may originate in double-stranded DNA (dsDNA) form (e.g., genomic DNA, genomic DNA fragments, cell-free DNA, and the like) from a sample or may originate in single-stranded form from a sample. RNA primary nucleic acids may be mRNA or non-coding RNA, e.g., microRNA or small interfering RNA. A preparation of DNA fragments from an input sample may be single or double stranded DNA.

The primary nucleic acid molecules may represent the entire genetic complement of an organism, e.g., genomic DNA molecules which include both intron and exon sequences, as well as non-coding regulatory sequences such as promoter and enhancer sequences. The primary nucleic acid molecules may represent the entire genetic complement of specific cells of an organism, e.g., from tumor cells, where the genomic DNA molecules which include both intron and exon sequences, as well as non-coding regulatory sequences such as promoter and enhancer sequences. In one embodiment, particular subsets of genomic DNA can be used, such as, for example, particular chromosomes, DNA associated with open chromatin, DNA associated with closed chromatin, or one or more specific sequences such as a region of a specific gene (e.g., targeted sequencing). In one or more embodiments, the primary nucleic acid molecules may represent a particular subset of DNA, e.g., DNA having a specific sequence that anneals with a primer such as one used for targeted sequencing or target enrichment. In one embodiment, a particular subset of DNA can be used, such as cell-free DNA, which can include DNA of the subject including DNA from normal cells, DNA from diseased cells such as tumor cells, and/or DNA from fetal cells.

The primary nucleic acid molecules may represent the entire transcriptome of cells of an organism, e.g., mRNA molecules. The primary nucleic acid molecules may represent the entire transcriptome of specific cells of an organism, e.g., from tumor cells or for instance the cells of a tissue. In one embodiment, the primary nucleic acid molecules may represent a particular subset of mRNA, e.g., mRNA having a specific sequence that anneals with a primer such as one used for targeted sequencing or target enrichment.

A sample, such as a biological sample, can include nucleic acid molecules obtained from biopsies, tumors, scrapings, swabs, blood, mucus, urine, plasma, semen, hair, laser capture micro-dissections, surgical resections, and other clinical or laboratory obtained samples. In some embodiments, the sample can be an epidemiological, agricultural, forensic, or pathogenic sample. In some embodiments, the sample can include cultured cells. In some embodiments, the sample can include nucleic acid molecules obtained from an animal such as a human or mammalian source. In another embodiment, the sample can include nucleic acid molecules obtained from a non-mammalian source such as a plant, bacteria, virus, or fungus. In some embodiments, the source of the nucleic acid molecules may be an archived or extinct sample or species.

Additional non-limiting examples of sources of biological samples can include whole organisms as well as a sample obtained from a subject or a patient. The biological sample can be obtained from any biological fluid or tissue and can be in a variety of forms, including liquid fluid and tissue, solid tissue, and preserved forms such as dried, frozen, and fixed forms. The sample may be of any biological tissue, cells, or fluid. Such samples include, but are not limited to, sputum, blood, serum, plasma, blood cells (e.g., white cells), ascitic fluid, urine, saliva, tears, sputum, vaginal fluid (discharge), washings obtained during a medical procedure (e.g., pelvic or other washings obtained during biopsy, endoscopy or surgery), tissue, nipple aspirate, core or fine needle biopsy samples, cell-containing body fluids, peritoneal fluid, and pleural fluid, or cells therefrom, and free floating nucleic acids such as cell-free circulating DNA. Biological samples may also include sections of tissues such as frozen or fixed sections taken for histological purposes or micro-dissected cells or extracellular parts thereof. In some embodiments, the sample can be a blood sample, such as, for example, a whole blood sample. In another example, the sample is an unprocessed dried blood spot (DBS) sample. In yet another example, the sample is a formalin-fixed paraffin-embedded (FFPE) sample. In yet another example, the sample is a saliva sample. In yet another example, the sample is a dried saliva spot (DSS) sample.

Exemplary biological samples from which target nucleic acids can be derived include, for example, those from a eukaryote, for instance a mammal, such as a rodent, mouse, rat, rabbit, guinea pig, ungulate, horse, sheep, pig, goat, cow, cat, dog, primate, human or non-human primate; a plant, such as *Arabidopsis thaliana,* corn, sorghum, oat, wheat, rice, canola, or soybean; an algae, such as *Chlamydomonas reinhardtii;* a nematode such as *Caenorhabditis elegans;* an insect, such as *Drosophila melanogaster,* mosquito, fruit fly, honey bee or spider; a fish, such as zebrafish; a reptile; an amphibian, such as a frog or *Xenopus laevis; a Dictyostelium discoideum;* a fungi, such as *Pneumocystis carinii, Takifugu rubripes,* yeast, *Saccharamoyces cerevisiae,* or *Schizosaccharomyces pombe;* or a protozoan such as *Plasmodium falciparum.* Target nucleic acids can also be derived from a prokaryote such as a bacterium, *Escherichia coli, Staphylococcus* or *Mycoplasma pneumoniae;* an archaeon; a virus such as Hepatitis C virus or human immunodeficiency virus; or a viroid. Target nucleic acids can be derived from a homogeneous culture or population of organisms described herein or alternatively from a collection of several different organisms, for example, in a community or ecosystem.

In some embodiments, a biological sample includes tissue that is processed to obtain the desired primary nucleic acids. In some embodiments, cells are used obtain the desired primary nucleic acids. In some embodiments, nuclei are used to obtain the desired primary nucleic acids. The method can further include dissociating cells, and/or isolating nuclei from cells. Methods for isolating cells and nuclei from tissue are available (WO 2019/236599).

In some embodiments, nucleic acids present in tissue, in cells, or in isolated nuclei can be processed depending on the desired read-out. For instance, nucleic acids can be fixed during processing, and useful fixation methods are available (WO 2019/236599). Fixation can be useful to preserve a sample or maintain contiguity of analytes from a sample, a cell, or a nucleus. Fixation methods preserve and stabilize tissue, cell, and nucleus morphology and architecture, inactivates proteolytic enzymes, strengthens samples, cells, and nuclei so they can withstand further processing and staining, and protects against contamination. Examples of methods where fixation can be useful include, but are not limited to, whole genome sequencing of isolated nuclei and chromosome conformation capture methods such as Hi-C. Common methods of fixation include perfusion, immersion, freezing, and drying (Srinivasan et al., Am J Pathol. 2002 Dec; 161(6): 1961-1971.doi: 10.1016/S0002-9440(10)64472-0). In some embodiments such as whole genome sequencing, isolated nuclei can be processed to dissociate nucleosomes from DNA while leaving the nuclei intact, and methods for generating nucleosome-free nuclei are available (WO 2018/018008).

In some embodiments, primary nucleic acids in bulk, e.g., from a plurality of cells, can be used to produce a sequencing library as described herein. In other embodiments, individual cells or nuclei can be used as sources of primary nucleic acids to obtain sequence information from single cells and nuclei. Many different single cell library preparation methods are known in the art, including, but not limited to, Drop-seq, Seq-well, and single cell combinatorial indexing ("sci-") methods. Companies providing single cell products and related technologies include, but are not limited to, Illumina, 10X genomics, Takara Biosciences, BD biosciences, Biorad, 1cellbio, isoplexis, CellSee, nanoselect, and Dolomite bio. Sci-seq is a methodological framework that employs split-pool barcoding to uniquely label the nucleic acid contents of large numbers of single cells or nuclei. Typically, the number of nuclei or cells can be at least two. The upper limit is dependent on the practical limitations of equipment (e.g., multi-well plates, number of indexes) used in other steps of the methods as described herein. The number of nuclei or cells that can be used is not intended to be limiting and can number in the billions.

The target nucleic acids used in the methods and compositions of the present disclosure can be derived by fragmentation. Random fragmentation refers to the fragmentation of a polynucleotide molecule from a primary nucleic acid sample in a non-ordered fashion by enzymatic, chemical, or mechanical methods. Such fragmentation methods are known in the art and use standard methods (Sambrook and Russell, Molecular Cloning, A Laboratory Manual, third edition). Moreover, random fragmentation is designed to produce fragments irrespective of the sequence identity or position of nucleotides comprising and/or surrounding the break. In one or more embodiments, the random fragmentation is by mechanical means such as nebulization or sonication to produce fragments of about 50 base pairs in length to about 1500 base pairs in length, for example, about 50-700 base pairs in length, about 50-400 base pairs in length. In some preferred embodiments, fragments are about 100 to 200 base pairs in length.

In some embodiments, the DNA fragments are DNA library fragments. Any of the many library preparation protocols available are compatible with the methods described herein. A library may be a whole-genome library or a targeted library. A library includes, but is not limited to, a sequencing library. A multitude of sequencing library methods are known to a skilled person (see, for example, Sequencing Methods Review, available on the world wide web at illumina.com/content/dam/illumina-marketing/documents/products/research_ reviews/sequencing-methods-review.pdf). For example, library preparation may be for use with any of a variety of next generation sequencing platforms, such as for example, the sequencing by synthesis platform of ILLUMINA^{®} or the ion semiconductor sequencing platform of ION TORRENT^{™}. For example, established ligase-dependent methods or transposon-based methods may be used (see, for example, Head et al, 2014, Biotechniques; 56(2):61 and Bruinsma et al., 2019, BMC Genomics; 19:722) and numerous kits for making sequencing libraries by these methods are available commercially from a variety of vendors.

DNA fragments, including DNA library fragments, may be prepared from input sample material such that adapter sequences are ligated to fragments to facilitate downstream workflow steps, such as for example, degradation of the second strand, amplification, and/or sequencing. For example, universal amplification sequences, e.g., sequences present in a universal adaptor, may be placed at the ends of each nucleotide fragment to facilitate amplification. Methods for attaching adapters to a nucleic acid are known to the person skilled in the art. For example, the attachment can be through tagmentation using transposase complexes (WO 2016/130704), or through standard library preparation techniques using ligation (U.S. Pat. Pub. No. 2018/0305753). Addition of an adapter can occur before or after treatment of the target nucleic acid with a cytidine deaminase and/or an uracil de-glycosylase.

Adapter sequences may include 5' and/or 3' adapter sequences. An adapter may be attached to just one end of the DNA fragment, for example, 5' and/or 3' ends, or to both ends. As used herein, the term "adapter" and its derivatives, e.g., universal adapter, refers generally to any linear oligonucleotide which can be attached to a target nucleic acid. An adapter can be single-stranded or double-stranded DNA or can include both double-stranded and single-stranded regions. An adapter can include a universal sequence that is substantially identical, or substantially complementary, to at least a portion of a primer, for example a universal primer; an index (also referred to herein as a barcode or tag) to assist with downstream error correction, identification, or sequencing; and/or a unique molecular identifier. In some embodiments, the adapter is substantially non-complementary to the 3' end or the 5' end of any target sequence present in the sample. In some embodiments, adapter sequences may have one or more phosphorothioate bonds at the 5' end of the adapter sequences. In some embodiments, suitable adapter lengths are in the range of about 6-100 nucleotides, about 12-60 nucleotides, or about 15-50 nucleotides in length. For instance, The terms "adaptor" and "adapter" are used interchangeably. As used herein, the term "universal," when used to describe a nucleotide sequence, refers to a region of sequence that is common to two or more nucleic acid molecules where the molecules also have regions of sequence that differ from each other. Non-limiting examples of universal capture sequences include sequences that are identical to or complementary to P5 and P7 primers. The terms "P5" and "P7" may be used when referring to a universal capture sequence or a capture oligonucleotide. The terms "P5'" (P5 prime) and "P7''' (P7 prime) refer to the reverse complement of P5 and P7, respectively. It will be understood that any suitable universal capture sequence or a capture oligonucleotide can be used in the methods presented herein, and that the use of P5 and P7 are exemplary embodiments only. Uses of capture oligonucleotides such as P5 and P7 or their complements on flowcells are known in the art, as exemplified by the disclosures of WO 2007/010251, WO 2006/064199, WO 2005/065814, WO 2015/106941, WO 1998/044151, and WO 2000/018957. For example, any suitable forward amplification primer, whether immobilized or in solution, can be useful in the methods presented herein for hybridization to a complementary sequence and amplification of a sequence.
Similarly, any suitable reverse amplification primer, whether immobilized or in solution, can be useful in the methods presented herein for hybridization to a complementary sequence and amplification of a sequence. One of skill in the art will understand how to design and use primer sequences that are suitable for capture and/or amplification of nucleic acids as presented herein.

DNA fragments, including DNA library fragments, can have an average strand length that is desired or appropriate for a particular application of the methods, compositions, or kits set forth herein. For example, the average strand length can be less than about 100,000 nucleotides, 50,000 nucleotides, 10,000 nucleotides, 5,000 nucleotides, 1,000 nucleotides, 500 nucleotides, 200 nucleotides, 100 nucleotides, or 50 nucleotides. Alternatively, or additionally, the average strand length can be greater than about 10 nucleotides, 50 nucleotides, 100 nucleotides, 200 nucleotides, 500 nucleotides, 1,000 nucleotides, 5,000 nucleotides, 10,000 nucleotides, 50,000 nucleotides, or 100,000 nucleotides. The average strand length for a population of DNA fragments can be in a range between a maximum and minimum value set forth above.

In some embodiments, DNA fragments, including DNA library fragments, may be of a shorter length, for example, about 50 nucleotides to about 500 nucleotides in length, about 50 nucleotides to about 300 nucleotides in length, about 50 nucleotides to about 250 nucleotides in length, about 100 nucleotides to about 200 nucleotides in length, or about 100 nucleotides to about 250 nucleotides in length. In some embodiments, DNA fragments, including DNA library fragments, may be about 100 nucleotides to about 200 nucleotides in length. Shorter fragment length can be employed to maximize the overall performance of the enzymatic error-correction, by minimizing the number of potential false-positive uracils that may be present in any one individual DNA fragment. By minimizing the number of false positives in each fragment, the probability of successfully repairing library fragments and mediating their downstream amplification may be increased, thus preserving library quality and diversity.

### Cytosine Deaminase

The following passage relating to Cytosine Deaminase for the U-ERASE method is not encompassed in the wording of the claims.

With the methods described herein, a sample including single-stranded DNA (ssDNA) fragments is contacted with a cytosine deaminase to deaminate methylated cytosines. In some embodiments, a sample including single-stranded DNA (ssDNA) fragments is a preparation of denatured library fragments. In some embodiments, the library fragments may include 5' and/or 3' adapter sequences.

As used herein, a "cytidine deaminase enzyme" refers to an enzyme that deaminates cytosine and/or one or more cytosine derivatives. The deamination occurs at the amino group of the C4 position of the cytosine or cytosine derivative. For example, a cytidine deaminase enzyme may deaminate cytosine to form U, may deaminate mC to form T, and/or may deaminate hydroxymethylcytosine (hmC) to form hmU. A nonlimiting example of a cytidine deaminase enzyme that may deaminate cytosine to form U, may deaminate mC to form T, and/or may deaminate hmC to form hmU is apolipoprotein B mRNA editing enzyme, catalytic polypeptide-like (APOBEC). Nonlimiting examples of such APOBECs include APOBEC1, APOBEC2, APOBEC3A, APOBEC3B, APOBEC3C, APOBEC3E, APOBEC3F, APOBEC3G, APOBEC3H, and APOBEC4. As used herein, the term "methylcytosine" or "mC" refers to cytosine that includes a methyl group (-CH3 or -Me). The methyl group may be located at the 5 position of the cytosine, in which case the mC may be referred to as 5mC.

In some embodiments, a cytidine deaminase is an altered cytidine deaminase, recombinantly engineered to include a substitution mutation at one or more residues when compared to a reference cytidine deaminase. An altered cytidine deaminase can be based on a member of the AID subfamily, the APOBEC1 subfamily, the APOBEC2 subfamily, the APOBEC3 subfamily (e.g., the 3A subfamily, the 3B subfamily, the 3C subfamily, the 3D subfamily, the 3F subfamily, the 3G subfamily, or the 3H subfamily), or the APOBEC4 subfamily. The skilled person will readily appreciate that such an altered or engineered cytidine deaminase described herein is not naturally occurring. In some embodiments, such an altered or engineered cytidine deaminase demonstrates selective deamination for mC.

An altered cytidine deaminase may be one of three types of altered cytidine deaminases. One type of altered cytidine deaminase preferentially deaminates 5mC instead of C (i.e., converts 5mC to T at a greater rate than converting C to U) and is referred to herein as having "cytosine-defective deaminase activity." A second type of altered cytidine deaminase preferentially deaminates C instead of 5mC (i.e., converts C to U at a greater rate than converting 5mC to T) and is referred to herein as having "5mC-defective deaminase activity." A third type of altered cytidine deaminase preferentially deaminates C and 5mC to U and T, respectively, and has significantly reduced deamination of 5hmC, 5fC, and 5caC. The third type is referred to herein as having "5hmC-defective deaminase activity." Unless the context indicates otherwise, reference to an altered cytidine deaminase includes altered cytidine deaminases having cytosine-defective deaminase activity, altered cytidine deaminases having 5mC-defective deaminase activity, and altered cytidine deaminases having 5mC-defective deaminase activity.

Altered cytidine deaminases include apolipoprotein B mRNA editing enzymes, catalytic polypeptide-like (APOBEC) and activation induced cytidine deaminase (AID). Wild-type APOBEC and AID cytidine deaminases have the activity of deaminating cytidine (C) of DNA and/or RNA to form uridine (U). An altered cytidine deaminase of the present disclosure has an altered rate of deamination of C, 5mC, and/or 5hmC when compared to the wild-type enzyme. A cytidine deaminase of the present disclosure can be referred to herein as an "altered cytidine deaminase," "recombinant cytidine deaminase," "mutant cytosine deaminase," or "modified cytidine deaminases" and refers to any of the altered cytosine deaminases described herein that comprise one or more changes from the reference (i.e., wildtype) amino acid sequence that provide the unexpected property of an altered deamination profile, e.g., alters its ability to preferentially deaminate one form of cytosine over another.

Whether a protein has cytidine deaminase activity may be determined by *in vitro* assays. On example of an *in vitro* assay is based on digestion with the restriction enzyme *SwaI. A* protein that can deaminate 5mC to thymidine has cytidine deaminase activity.

An altered cytidine deaminase that preferentially deaminates 5mC instead of C (i.e., has cytosine-defective deaminase activity) can have a catalytic efficiency that is at least 10-fold, at least 50-fold, or at least 100-fold higher on 5mC than C substrates. In one embodiment, an altered cytidine deaminase that preferentially deaminates 5mC instead of C can have a catalytic efficiency that is no greater than 1500-fold higher on 5mC than C substrates.

An altered cytidine deaminase that preferentially deaminates C instead of 5mC (i.e., has 5mC-defective deaminase activity) can have a catalytic efficiency that is at least 10-fold, at least 50-fold, or at least 100-fold higher on C than 5mC substrates. In one embodiment, an altered cytidine deaminase that preferentially deaminates C instead of 5mC can have a catalytic efficiency that is no greater than 1500-fold higher on C than 5mC substrates.

When compared to a wild type cytidine deaminase, an altered cytidine deaminase that deaminates C and 5mC to U and T, respectively, and has significantly reduced deamination of 5hmC (i.e., has 5hmC-defective deaminase activity), the deamination of 5hmC by an altered cytidine deaminase disclosed herein is reduced by at least 80%, at least 90%, or at least 99% compared to the wild type cytidine deaminase. In one embodiment, the deamination of 5hmC by an altered cytidine deaminase disclosed herein is undetectable using an assay such as the *SwaI-*based assay.

In certain embodiments, an altered cytidine deaminase of the present disclosure is based on a member of the APOBEC protein family. An altered cytidine deaminase of the present disclosure that is "based on" a member of the APOBEC protein family means the altered cytidine deaminase is an APOBEC protein that includes one or more of the substitution mutations described herein as compared to a reference APOBEC sequence. An altered cytidine deaminase of the present disclosure that is "based on" a member of the APOBEC protein family can also include conservative and/or nonconservative mutations as described herein.

The APOBEC protein family includes subfamilies AID, APOBEC1, APOBEC2, APOBEC3 (including 3A, 3B, 3C, 3D, 3F, 3G, 3H), and APOBEC4. An altered cytidine deaminase of the present disclosure can be based on a member of the AID subfamily, the APOBEC1 subfamily, the APOBEC2 subfamily, the APOBEC3 subfamily (e.g., the 3A subfamily, the 3B subfamily, the 3C subfamily, the 3D subfamily, the 3F subfamily, the 3G subfamily, or the 3H subfamily), or the APOBEC4 subfamily. An altered cytidine deaminase of the present disclosure can be based on a member of the APOBEC protein family from a vertebrate, such as a mammal. Examples of mammals include, but are not limited to, rodents, primates, rabbit, bovine (e.g., cow), porcine (e.g., pig), and equine (e.g., horse). An example of a primate is a human and a chimpanzee.

The APOBEC protein family is a member of the large cytidine deaminase superfamily that contains a canonical zinc-dependent deaminase (ZDD) signature motif embedded within a core cytidine deaminase fold. This fold includes a five-stranded mixed beta (b)-sheet surrounded by six alpha (a)-helices with the order a1-b1-b2-a2-b3-a3-b4-a4-b5-a5-a6 (Salter et al., 2016, Trends Biochem Sci; 41(7):578-594. doi:10.1016/j.tibs.2016.05.001; Salter et al., 2018, Trends Biochem Sci; 43(8):606-622 doi.org/10.1016/j.tibs.2018.04.013). Each cytidine deaminase domain core structure of APOBEC proteins contains a highly conserved spatial arrangement of the catalytic center residues of a zinc-binding motif H-[P/A/V]-E-X_{[23-28]}-P-C-X_{[2-4]}-C (SEQ ID NO: 1) (referred to herein as the ZDD motif, where X is any amino acid, and the subscript range of numbers after X refers to the number of amino acids) (Salter et al., 2016, Trends Biochem Sci; 41(7):578-594. doi:10.1016/j.tibs.2016.05.001). Without intending to be limited by theory, the H and two C residues coordinate a Zn atom, and the E residue polarizes a water molecule near the Zn-atom for catalysis (Chen et al., 2021, Viruses; 13:497).

Some members of the APOBEC protein family, e.g., the AID subfamily, the APOBEC1 subfamily, the APOBEC2 subfamily, the APOBEC3A subfamily, the APOBEC3C subfamily, the APOBEC3H subfamily, and the APOBEC4 subfamily, include one copy of the ZDD motif. Other members of the APOBEC protein family, e.g., the APOBEC3B subfamily, the APOBEC3D subfamily, the APOBEC3F subfamily, and the APOBEC3G subfamily, include two copies of the ZDD motif, but often only the C-terminal copy is active (Salter et al., 2016, Trends Biochem Sci; 41(7):578-594. doi:10.1016/j.tibs.2016.05.001). Thus, an altered cytidine deaminase disclosed herein includes one or two ZDD motifs. In one embodiment, an altered cytidine deaminase based on a member of the APOBEC3A subfamily includes the following ZDD motif: HXEX₂₄SW(S/T)PCX_{[2-4]}CX₆FX₈LX₅R(L/I)YX_{[8-11]}LX₂LX_{[10]}M (SEQ ID NO: 2) (where X is any amino acid, and the subscript number or range of numbers after X refers to the number of amino acids) (Salter et al., 2016, Trends Biochem Sci; 41(7):578-594).

In one embodiment, an altered cytidine deaminase disclosed herein is a member of the following subfamilies, APOBEC3A, APOBEC3B, APOBEC3C, APOBEC3D, APOBEC3F, and APOBEC3G, and can include one or more highly conserved sites that are part of the active site and within the ZDD motif SEQ ID NO: 1. The sites include tryptophan at position 98 and serine or threonine at position 99 (Kouno et al., 2017, Nat. Comm; 8:15024).

In addition to the ZDD motif, a member of the APOBEC protein family also includes other highly conserved residues that are part of the active site but not present as part of the ZDD motif SEQ ID NO: 1. A member the APOBEC3A subfamily, APOBEC3B subfamily, APOBEC3C subfamily, APOBEC3D subfamily, APOBEC3F subfamily, and APOBEC3G subfamily typically includes one or more of the following highly conserved sites that are part of the active site: arginine at position 28; histidine, asparagine, or arginine at position 29; serine or threonine, preferably threonine, at position 31; asparagine or aspartic acid at position 57; tyrosine or phenylalanine at position 130; asparagine or tyrosine at position 131; asparagine, tyrosine, or phenylalanine, preferably tyrosine, at position 132; and arginine or lysine at position 189 (Kouno et al., 2017, Nat. Comm; 8:15024, DOI: 10.1038/ncomms15024).

An altered cytidine deaminase of the present disclosure includes a substitution mutation at one or more residues when compared to a reference cytidine deaminase. A substitution mutation can be at the same position or a functionally equivalent position compared to the reference cytidine deaminase. Reference cytidine deaminases and functionally equivalent positions are described in detail herein. The skilled person will readily appreciate that an altered cytidine deaminase described herein is not naturally occurring.

A reference cytidine deaminase can be a member of the APOBEC protein family. Essentially any known member of the APOBEC protein family can be a reference cytidine deaminase. The skilled person can easily identify members of each of the subfamilies by using a publicly available database such as the Protein database available at the National Center for Biotechnology Information (ncbi.nlm.nih.gov/protein) and searching for APOBEC1, APOBEC2, APOBEC3A, APOBEC3B, APOBEC3C, APOBEC3D, APOBEC3F, APOBEC3G, APOBEC3H, APOBEC4, or, when identifying members of the AID family, Activation-induced cytidine deaminase. A wild type reference cytidine deaminase has the activity of binding single-stranded DNA (ssDNA) and deaminating a cytosine present on the ssDNA to convert it to uracil. In one embodiment, a wild type reference cytidine deaminase has the activity of binding single-stranded RNA (ssRNA) and deaminating a cytosine present on the ssRNA to convert it to uracil. Methods for determining whether a protein binds ssDNA or ssRNA and deaminates a cytosine present are known to the skilled person.

In one embodiment, an altered cytidine deaminase has an amino acid sequence that is based on a reference sequence which is a member of the APOBEC protein family includes a ZDD motif H-[P/A/V]-E-X_{[23-28]}-P-C-X_{[2-4]}-C (SEQ ID NO: 1) and at least one substitution mutation disclosed herein. Optionally, an altered cytidine deaminase includes other active site residues disclosed herein. Non-limiting examples of reference cytidine deaminase proteins are shown in the following table.

In one embodiment, an altered cytidine deaminase has an amino acid sequence that is based on a reference sequence that is a member of the APOBEC3A subfamily, and includes a ZDD motif HXEX₂₄SW(S/T)PCX_{[2-4]}CX₆FX₈LX₅R(L/I)YX_{[8-11]}LX₂LX_{[10]}M (SEQ ID NO: 2) (where X is any amino acid, and the subscript number or range of numbers after X refers to the number of amino acids) and at least one substitution mutation disclosed herein. In one embodiment, the substitution mutation is a substitution mutation at the underlined tyrosine, such as a substitution mutation to alanine (A). Optionally, the altered cytidine deaminase includes other active site residues disclosed herein.

In one embodiment, the amino acid sequence of an altered cytidine deaminase includes the amino acids of a member of the APOBEC3A subfamily: X_{[16-26]}-GRXXTXLCYXV-X₁₅-GXXXN-X₁₂-HAEXXF-X₁₄-YXXTWXXSWSPC-X_{[2-4]}-CA-X₅-FL-X₇-LXIXXXR(L/I)Y-X₈-GLXXLXXXG-X₅-M-X₄-FXXCWXXFV-X₆-FXPW-X₁₃-LXXI- X_{[2-6]} (SEQ ID NO: 3) (where X is any amino acid, and the subscript number or range of numbers after X refers to the number of amino acids), or a subset thereof, and at least one substitution mutation disclosed herein. In one embodiment, the substitution mutation is a substitution mutation at the underlined tyrosine, such as a substitution mutation to alanine (A) or to tryptophan (W).

In one embodiment, the amino acid sequence of an altered cytidine deaminase includes the amino acids of a member of the APOBEC3A subfamily: X₂₆-GRXXTXLCYXV-X₁₅-G-X₁₆-HAEXXF-X₁₄-YXXTWXXSWSPC-X₄-CA-X₅-FL-X₇-LXIFXXR(L/I)Y-X₈-GLXXLXXXG-X₅-M-X₄-FXXCWXXFV-X₆-FXPW-X₁₃-LXXI-X₆ (SEQ ID NO: 4) (where X is any amino acid, and the subscript number after X refers to the number of amino acids present), or a subset thereof, and at least one substitution mutation disclosed herein. In one embodiment, the substitution mutation is a substitution mutation at the underlined tyrosine (Y), such as a substitution mutation to alanine (A) or to tryptophan (W).

A substitution mutation can be at the same position or a functionally equivalent position compared to a reference cytidine deaminase. By "functionally equivalent" it is meant that the altered cytidine deaminase has the amino acid substitution at the amino acid position in a reference cytidine deaminase that has the same functional role in both the reference cytidine deaminase and the altered cytidine deaminase.

In general, functionally equivalent substitution mutations in two or more different cytidine deaminases occur at homologous amino acid positions in the amino acid sequences of the cytidine deaminases. Hence, use herein of the term "functionally equivalent" also encompasses mutations that are "positionally equivalent" or "homologous" to a given mutation, regardless of whether or not the particular function of the mutated amino acid is known. It is possible to identify the locations of functionally equivalent and positionally equivalent amino acid residues in the amino acid sequences of two or more different cytidine deaminases on the basis of sequence alignment and/or molecular modelling. For example, the tyrosine at residue 130 of the APOBEC3A proteins of *Homo sapiens, Pongo pygmaeus, Nomascus leucogenys, Pan troglodytes,* and *Gorilla* and the tyrosine at residue 133 of the APOBEC3A protein from *Macaca fascicularis* are functionally equivalent and positionally equivalent. The skilled person can easily identify functionally equivalent residues in cytidine deaminases.

In one embodiment, an altered cytidine deaminase has an amino acid sequence that is structurally similar to a reference cytidine deaminase disclosed herein. In one embodiment, a reference cytidine deaminase is one that includes the amino acid sequence of a sequence listed in Table 1.

As used herein, an altered cytidine deaminase may be "structurally similar" to a reference cytidine deaminase if the amino acid sequence of the altered cytidine deaminase possesses a specified amount of sequence similarity and/or sequence identity compared to the reference cytidine deaminase.

Structural similarity of two amino acid sequences can be determined by aligning the residues of the two sequences (for example, a candidate altered cytidine deaminase and a reference cytidine deaminase described herein) to optimize the number of identical amino acids along the lengths of their sequences; gaps in either or both sequences are permitted in making the alignment in order to optimize the number of identical amino acids, although the amino acids in each sequence must nonetheless remain in their proper order. A candidate altered cytidine deaminase is the cytidine deaminase being compared to the reference cytidine deaminase. A candidate altered cytidine deaminase that has structural similarity with a reference cytidine deaminase and cytidine deaminase activity is an altered cytidine deaminase.

Unless modified as otherwise described herein, a pair-wise comparison analysis of amino acid sequences can be conducted, for instance, by the local homology algorithm of Smith and Waterman, 1981, Adv. Appl. Math; 2:482, by the homology alignment algorithm of Needleman and Wunsch, 1907, J Mol Biol; 48:443, by the search for similarity method of Pearson and Lipman, 1988, Proc Nat'l Acad Sci USA; 85:2444, by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Current Protocols in Molecular Biology, Ausubel et al., eds., Current Protocols, a joint venture between Greene Publishing Associates, Inc. and John Wiley & Sons, Inc., supplemented through 2004). One example of an algorithm that is suitable for determining structural similarity is the BLAST^{®} algorithm, which is described in Altschul et al., 1990, J Mol Biol; 215:403-410. The BLAST^{®} algorithm can be used to calculate percent sequence identity and percent sequence similarity between two sequences. Software for performing BLAST^{®} analyses is publicly available through the National Center for Biotechnology Information.

In the comparison of two amino acid sequences, structural similarity may be referred to by percent "identity" or may be referred to by percent "similarity." "Identity" refers to the presence of identical amino acids. "Similarity" refers to the presence of not only identical amino acids but also the presence of conservative substitutions. Thus, in one embodiment the amino acid sequence of a cytidine deaminase protein having sequence similarity to a reference sequence may include conservative substitutions of amino acids present in that reference sequence.

A conservative substitution for an amino acid in a protein may be selected from other members of the class to which the amino acid belongs. For example, it is well-known in the art of protein biochemistry that an amino acid belonging to a grouping of amino acids having a particular size or characteristic (such as charge, hydrophobicity, or hydrophilicity) can be substituted for another amino acid without altering the activity of a protein, particularly in regions of the protein that are not directly associated with biological activity. For example, amino acids having a non-polar side chain include alanine, glycine, isoleucine, leucine, methionine, phenylalanine, proline, tryptophan, and valine; amino acids having a hydrophobic side chain include glycine, alanine, valine, leucine, isoleucine, proline, phenylalanine, methionine, and tryptophan; amino acids having a polar side chain include arginine, asparagine, aspartic acid, glutamine, glutamic acid, histidine, lysine, serine, cysteine, tyrosine, and threonine; and amino acids having an uncharged side chain include glycine, serine, cysteine, asparagine, glutamine, tyrosine, and threonine.

Thus, as used herein, reference to a cytidine deaminase as described herein, such as reference to the amino acid sequence of one or more SEQ ID NOs described herein can include a protein with at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% amino acid sequence similarity to the reference cytidine deaminase. Examples of altered cytidine deaminases having similarity with a reference amino acid sequence includes those having, for instance, at least 80%, at least 85%, at least 90%, or at least 95% similarity with SEQ ID NO: 5 and having an alanine at amino acid 130. Other examples of altered cytidine deaminases having similarity with a reference amino acid sequence includes those having, for instance, at least 80%, at least 85%, at least 90%, or at least 95% similarity or identity with SEQ ID NO: 6 and having an alanine at amino acid 130 and a histidine at amino acid 132.

Alternatively, as used herein, reference to a cytidine deaminase as described herein, such as reference to the amino acid sequence of one or more SEQ ID NOs described herein can include a protein with at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% amino acid sequence identity to the reference cytidine deaminase. Examples of altered cytidine deaminases having identity with a reference amino acid sequence includes those having, for instance, at least 80%, at least 85%, at least 90%, or at least 95% similarity with SEQ ID NO: 5 and having an alanine (A) at amino acid 130. Other examples of altered cytidine deaminases having identity with a reference amino acid sequence includes those having, for instance, at least 80%, at least 85%, at least 90%, or at least 95% similarity or identity with SEQ ID NO: 6 and having an alanine (A) at amino acid 130 and a histidine (H) at amino acid 132.

An altered cytidine deaminase of the present disclosure may include a substitution mutation at a position functionally equivalent to tyrosine at position 130 (Y130) in a member of the APOBEC3A subfamily. Accordingly, an alignment can be produced using a member of the APOBEC3A subfamily and another candidate altered cytidine deaminase from the APOBEC3A subfamily or a different APOBEC subfamily. In one embodiment, the candidate is selected from APOPEC subfamilies APOBEC1 or AID. An example of an algorithm that can be used to produce an alignment is Clustal O. In some APOBEC family proteins, the wild type residue at a position functionally equivalent to Y130 is phenylalanine (F).

In another embodiment, an altered cytidine deaminase of the present disclosure includes a substitution mutation at a position functionally equivalent to the tyrosine (Y) of ZDD motif HXEX₂₄SW(S/T)PCX_{[2-4]}CX₆FX₈LX₅R(L/I)YX_{[8-11]}LX₂LX_{[10]}M (SEQ ID NO: 2) in a member of the APOBEC family, such as a member of the APOBEC3A subfamily. The underlined tyrosine (Y) of SEQ ID NO: 2 is the position functionally equivalent to the tyrosine amino acid 130 of the wild type APOBEC3A protein (SEQ ID NO: 12).

In one embodiment, the substitution mutation at a position functionally equivalent to Y130 increases cytidine deaminase activity and preferentially acts on 5mC compared to cytosine (i.e., has cytosine-defective deaminase activity). The substitution mutation can be a mutation to alanine (A), glycine (G), phenylalanine (F), histidine (H), glutamine (Q), methionine (M), asparagine (N), lysine (K), valine (V), aspartic acid (D), glutamic acid (E), serine (S), cysteine (C), proline (P), or threonine (T). For example, the altered cytidine deaminase can comprise SEQ ID NO: 9, wherein X is selected from A, G, F, H, Q, M, N, K, V, D, E, S, C, P or T (and is not Y), or can comprise SEQ ID NO: 10, wherein Z is selected from A, G, F, H, Q, M, N, K, V, D, E, S, C, P or T (and is not Y), preferably, in one embodiment, X or Z is A or L. In an exemplary aspect of this embodiment, the substitution mutation at a position functionally equivalent to Y130 is a mutation to alanine (A), (e.g., SEQ ID NO: 5). Specific examples of altered cytidine deaminases having increased activity and preferentially acting on 5mC compared to cytosine include SEQ ID NO: 5 or a sequence having at least 90%, at least 95%, at least 98%, at least 99% sequence identity to SEQ ID NO: 5 and comprising Y130A.

An altered cytidine deaminase of the present disclosure having cytosine-defective deaminase activity (i.e., converts 5mC to T at a greater rate than converting C to U) optionally includes a second substitution mutation at a position two, three, four, or five amino acids on the C-terminal side of the Y130 position, or functionally equivalent to the Y130 position. In one embodiment, the second mutation is a tyrosine (Y), tryptophan (W), cysteine (C), histidine (H), or phenylalanine (F) at a position two, three, four, or five amino acids on the C-terminal side of the Y130 position, or functionally equivalent to the Y130 position. In one embodiment, the second mutation is at a position functionally equivalent to tyrosine at position 132 (Y132) in a member of the APOBEC3A subfamily. An APOBEC protein, such as an APOBEC3A protein, containing substitution mutations at both the first site, a position functionally equivalent to Y130, and the second site, at a position two, three, four, or five amino acids on the C-terminal side of the Y130 position, increases the preferential activity to act on 5mC compared to the same APOBEC protein, such as an APOBEC3A protein, containing one substitution mutation at Y130. In one embodiment, the substitution mutation at the second position is an amino acid having a positively charged side chain and selected from arginine (R), histidine (H), lysine (L), or a polar side chain selected from glutamine (Q). In one embodiment, the substitution mutation at the second position is histidine (H), such as Y132 to histidine. The double mutant containing both first and second mutations can be any substitution mutation at a position functionally equivalent to Y130 described herein and any second substitution mutation at a position two, three, four, or five amino acids on the C-terminal side of the Y130 position described herein, in any combination. For example, the altered cytidine deaminase can be, for example, SEQ ID NO: 4 and have a substitution at Y130 and Y132, or the position functionally equivalent to Y130 and Y132 as described herein. One example of an altered cytidine deaminase is SEQ ID NO: 11 comprising Y130X and Y132Z, where X is selected from (A), (L), or (W) (preferably (A)), and Z is selected from (R), (H), (L), or (Q), preferably (H). This encompasses examples including, but not limited to, for example Y130A and Y132R, Y130A and Y132H, Y130A and Y132L, Y130A and Y132Q, Y130L and Y132R, Y130L and Y132H, Y130L and Y132L, Y130L and Y132Q, Y130W and Y132R, Y130W and Y132H, Y130W and Y132L, Y130W and Y130Q, or any suitable combinations therein. In one embodiment, the double mutant includes substitution mutations Y130A and Y132H. Specific examples of altered cytidine deaminases having both substitution mutations and preferentially acting on 5mC compared to the APOBEC protein having just the single substitution mutation at cytosine include SEQ ID NO: 6 or a sequence having at least 90%, at least 95%, at least 98%, at least 99% sequence identity to SEQ ID NO: 6 and comprising Y130A and Y132H.

The person of ordinary skill in the art can confirm the 5mC preferential deaminase activity of the arginine, glutamine, histidine, and lysine substitution mutations at the second position in the double mutants described above. For example, double mutants can be constructed to create an altered cytidine deaminase having a first substitution mutation at a position functionally equivalent to Y130 and a second arginine, glutamine, histidine, or lysine substitution mutation at the tyrosine position two amino acids on the C-terminal side of the Y130 position, and then evaluated for deamination of C residues in one assay and deamination of 5mC residues in a second assay. Using an assay such as the SwaI-based assay described herein, the ratio of 5mC deamination and C deamination can be compared to identify those double mutants that preferentially deaminate 5mC compared to C. One of ordinary skill in the art could similarly test double mutants having a tyrosine at a position three, four or five positions C-terminal to the position functionally equivalent to Y130 and confirm that a substitution mutation at that position to arginine, glutamine, histidine, or lysine, in combination with a mutation at the position functionally equivalent to Y130 (such as Y130A), as double mutants that preferentially deaminate 5mC compared to C.

Some embodiments presented herein relate to substitution mutations that result in 5mC-defective deaminase activity (i.e., converts C to U at a greater rate than converting 5mC to T). In one embodiment, the substitution mutation at a position functionally equivalent to Y130 increases cytidine deaminase activity and preferentially acts on cytosine compared to 5mC and is a mutation to an amino acid having a non-polar side chain or a hydrophobic side chain, such as leucine (L) or tryptophan (W). In an exemplary aspect of this embodiment, the substitution mutation at a position functionally equivalent to Y130 is a mutation to leucine. Other examples of mutations that result in increased preferential deamination activity on cytosine compared to 5mC include a single mutant with Y132P, and double mutants with a substitution mutation at Y130V and Y132H, or Y130W and Y132H. Specific examples of altered cytidine deaminases having increased cytidine deaminase activity and preferentially acts on cytosine compared to 5mC include SEQ ID NO: 7 or a sequence having at least 90%, at least 95%, at least 98%, at least 99% sequence identity to SEQ ID NO: 7 and comprising Y130L.

In one embodiment, the substitution mutation is at a position functionally equivalent to Y130 that results in 5hmC-defective deaminase activity (i.e., preferentially deaminates C and 5mC to U and T, respectively, and has significantly reduced deamination of 5hmC). In an exemplary aspect of this embodiment, the substitution mutation at a position functionally equivalent to Y130 is a mutation to an amino acid having a non-polar side chain or a hydrophobic side chain, such as tryptophan (W). Specific examples of altered cytidine deaminases having the ability to deaminate C and 5mC to U and T, respectively, but reduced ability to deaminate 5hmC, preferably no detectable ability to deaminate 5hmC include SEQ ID NO: 8 or a sequence having at least 90%, at least 95%, at least 98%, at least 99% sequence identity to SEQ ID NO: 8 and comprising Y130W.

In some embodiments, an altered cytidine deaminase includes a substitution mutation at a position functionally equivalent to tyrosine at position 130 (Y130) and/or tyrosine position 132 (Y132) in a member of the APOBEC3A subfamily. In some embodiments, such an altered cytidine deaminase demonstrates selective deamination for mC.

In some embodiments, an altered cytidine deaminase is an altered APOBEC3A cytidine deaminase, altered to include a substitution mutation at tyrosine at position 130 (Y130) and/or tyrosine position 132 (Y132). In some embodiments, such an altered APOBEC3A cytidine deaminase demonstrates selective deamination for mC.

In some embodiments, an altered cytidine deaminase is a double mutant of APOBEC3A, with substitution mutations Y130A/Y132H. In some embodiments, such an altered APOBEC3A cytidine deaminase demonstrates selective deamination for mC.

In some embodiments, an altered cytidine deaminase includes an altered cytidine deaminase having an amino acid of SEQ ID NO: 5, SEQ ID NO: 6, SEQ ID NO: 7, SEQ ID NO: 8, SEQ ID NO: 9, SEQ ID NO: 10, or SEQ ID NO: 11. In some embodiments, such an altered APOBEC3A cytidine deaminase demonstrates selective deamination for mC.

An altered cytidine deaminase described herein can include additional mutations. Typically, additional mutations do not unduly alter the activity of the altered cytidine deaminase. One or more additional mutations can be a conservative mutation.

An altered cytidine deaminase described herein can be a truncated protein. A truncated protein is a fragment of an altered cytidine deaminase of the present disclosure that retains the ability to deaminate 5mC to thymidine. A truncated altered cytidine deaminase can include a deletion of 1 to 13 amino acids on the N-terminal end of the protein, a deletion of 1 to 3 amino acids on the C-terminal end of the protein, or a combination thereof.

In some embodiments, an altered cytidine deaminase includes any of those described in International Patent Application No. PCT/US2023/017846 ("Altered Cytidine Deaminases and Methods of Use"), filed April 7, 2023, which is hereby incorporated by reference in its entirety.

In general, methods for using a cytidine deaminase include contacting target nucleic acids, e.g., DNA or RNA, with the enzyme, under conditions suitable for conversion of modified cytidines, such as 5mC, to thymidine, or for conversion of unmodified cytidine to uracil. Because amplification of DNA does not preserve the modification status of cytidine (e.g., the methylation status of 5mC is not retained), use of a cytidine deaminase typically occurs before amplification of target DNA. Target nucleic acids can be contacted with cytidine deaminase at essentially any time. For instance, target nucleic acids can be contacted with cytidine deaminase after isolation of genomic or cell free DNA or mRNA, before or after fragmentation, or before or after tagmentation. The skilled person will recognize that target nucleic acids can be contacted with a cytidine deaminase after addition of a universal sequence and/or an adapter, provided the universal sequence and/or an adapter is not added by amplification.

Reaction conditions suitable for conversion of modified cytidines, such as 5mC, to thymidine by a cytidine deaminase include, but are not limited to, a substrate of target nucleic acid suspected of including at least one modified cytidine, with appropriate pH, temperature of the reaction, time of the reaction, and concentration of the cytidine deaminase and/or DNA or RNA substrate. It is expected that a cytidine deaminase can function in essentially any buffer. Examples of useful buffers include, but are not limited to, a citrate buffer, such as the citrate buffer available from Thermo Fisher Scientific (Cat. No. #005000); sodium acetate buffer, Bis Tris-Propane HCl; and Tris-HCl Tris. Examples of other buffers include, but are not limited to, Bicine, DIPSO, glycylglycine, HEPES, imidazole, malonate, MES, MOPS, PB, phosphate, PIPES, SPG, succinate, TAPS, TAPSO, trincine. Cytidine deaminases typically function at near-neutral pH, e.g., pH 7. In some embodiments a reducing agent such as dithiothreitol (DTT) can be present. In some embodiments a divalent cation is not included. A deamination reaction can occur at a temperature of about 25°C to about 60°C, including but not limited to, at about 37°C, at about 45°C, at about 50°C, and at about 60°C.

Some cytidine deaminases preferentially deaminate a modified cytosine to thymidine at a faster rate than deamination of cytosine to uracil. Thus, in some embodiments the time of reaction can be used to allow the reaction to run to completion, to maximize the difference of deamination of modified cytosine versus deamination of cytosine. In some embodiments, the reaction can proceed for at least 15 minutes, at least 30 minutes, at least 45 minutes, at least 60 minutes, at least 90 minutes, at least 120 minutes, or at least 150 minutes, and for no greater than 15 minutes, no greater than 30 minutes, no greater than 45 minutes, no greater than 60 minutes, no greater than 90 minutes, no greater than 120 minutes, no greater than 150 minutes, or no greater than 180 minutes. In some embodiments, the reaction can run overnight.

In some embodiments, a deamination reaction can include a cytidine deaminase at a concentration from at least about 25 nanomolar (nM) to no greater than about 5 micromolar (µM). For instance, the concentration of the enzyme can be at least about 25 nM, at least about 0.5, at least about 1 µM, at least about 2µM, at least about 3 µM, at least about 4 µM, or at least about 5 µM, and/or no greater than 5 µM, no greater than 4 µM, no greater than 3µM, no greater than 2 µM, no greater than 1 µM, or 0.5 µM. In some embodiments, a deamination reaction can include about 1 ng to about 1 µg input nucleic acid. In some embodiments, a deamination reaction can include nucleic acids at a concentration of at least about 10 pM to at least about 200 nM.

### Second Strand Synthesis

The following passage relating to second strand synthesis is not encompassed by the wording of the claims.

After a preparation of single-stranded DNA (ssDNA) fragments has been treated with a cytidine deaminase, a second complementary strand of DNA is synthesized, providing double stranded DNA (dsDNA) fragments. With the synthesis of these complementary strands, cytosines that have been converted to uracils (false positives) are copied as adenines in the second strand. In order to facilitate the downstream sequencing of only the original strand, this second strand of DNA, which serves as a scaffold for the enzymatic repair steps of the methods as described in more detail below, is marked in order to provide for its selective degradation in downstream steps, thereby facilitating the analysis of the original deaminase-treated single stranded DNA.

Any of the many protocols available for the synthesis of a complementary second DNA strand are compatible with the methods described herein. A DNA polymerase and a mixture of all four deoxyribonucleoside 5'-triphosphates (dNTPs) are provided for the synthesis of the second complementary strand. These four types of dNTP include adenine (dATP), cytosine (dCTP), guanine (dGTP), and thymine (dTTP). Any of a variety of polymerases may be used, including, but not limited to, Taq, Phusion U, Klenow exo-, and Bsu. In some embodiments, a polymerase that tolerates uracil (dU tolerant), able to use templates containing uracil or capable of using dUTP during polymerization, may be utilized. Examples of dU tolerant polymerases include, but are not limited to, KAPA HiFi Uracil+ DNA Polymerase (Roche), Q5U^{®} Hot Start High-Fidelity DNA Polymerase (New England Biolabs), and VeraSeq Ultra DNA Polymerase (Enzymatics, Inc.).

### Double strand synthesis with 5' phosphorylated primer

The following passage relating to double strand synthesis 5' phosphorylated primer is not encompassed by the wording of the claims.

In some embodiments of the methods described herein not encompassed by the wording of the claims, during library prep, adapters with one or more phosphorothioate bonds are utilized. Such a primer contains one or more phosphorothioate bonds near the 5' end. With synthesis of the second strand, the presence of a 5'phosphate enables its selective degradation later in the workflow using lambda exonuclease. Lambda exonuclease can degrade DNA without a 5'phosphate, albeit this occurs much slower than degrading DNA with a 5'phosphate. This is shown in FIG. 6A. In order to improve selectivity of Lambda exonuclease to degrade only the synthesized 2nd strand, during library prep, adapters with one or more phosphorothioate bonds can be utilized.

### Double strand synthesis with 8-oxoguanine or inosine residues

The following passage relating to double strand synthesis with 8-oxoguanine or inosine residues is not encompassed by the wording of the claims.

In some alternative embodiments of the methods described herein not encompassed by the wording of the claims,, primers containing 8-oxoguanine or inosine residues are utilized for synthesis of the second complementary strand of DNA. This is shown in FIG. 6B. One primer may bind the 3' end of the library fragment and mediate polymerization of the library insert, while another primer may bind the 5' end of the library fragment and is incorporated through ligation. A mixture of enzymes containing a polymerase (including, for example, an exonuclease-deficient Taq polymerase which is uracil-tolerant) and a ligase (for example, Taq DNA ligase) can be used to generate the tagged second strand. The polymerase employed for this reaction is not required to have high fidelity, as the resulting strand is not sequenced. The presence of the 8-oxoguanine or inosine residues enables the selective cleavage of the adapter sequences later in the workflow using formamidopyrimidine-DNA glycosylase (FPG) or oxoguanine glycosylase (OGG) for the cleavage of adapters sequences containing 8-oxoguanine residues (see, for example, Murphy and George, 2005, Biochem Biophys Res Commun; 329(3):869-872; and Murphy and Guo, 2010, Biochem Biophys Res Commun; 392(3):335-339 ) and Endonuclease V for the cleavage of adapters sequences containing inosine residues (see, for example, Cao, 2013, Cell Mol Life Sci; 70(17):3145-56).

### Double strand synthesis with nested primers

The following passage relating to double strand synthesis with nested primers is not encompassed by the wording of the claims.

In some embodiments of the methods described herein not encompassed by the wording of the claims, nested primers are provided for synthesis of the second complementary strand of DNA. This is shown in FIG. 6C. With synthesis of the second strand, the truncated adapter sequence prevents its exponential amplification, as shown in FIG. 6D. Furthermore, for analysis via Sequencing-by-synthesis, the truncated adapter would prevent addition of the full adapter sequence needed for clustering on the flow cell. In contrast, the original library fragment maintains full adapter sequences and can be exponentially amplified during PCR, with full adapter sequences being added to facilitate clustering on an SBS flow cell.

### Uracil De-Glycosylation

The following passage on uracil de-glycosylation is not encompassed by the wording of the claims. With the methods described herein, after the preparation of double-stranded DNA (dsDNA) fragments, the ds DNA fragments are then contacted with an Uracil-DNA-glycosylase. Uracil-DNA-glycosylase (UDG), also known as Uracil-N-glycosylase (UNG), is a highly conserved repair enzyme that catalyzes the excision of uracil from uracil-containing single- and double-stranded DNA but is inactive to RNA. It is a monomeric protein with relatively stable physicochemical properties, a small molecular weight of 25KDa, and is widely present in various prokaryotic and eukaryotic organisms. See, for example, Hölz et al., 2019, Scientific Reports; 9:17822; Schormann et al., 2014, Protein Sci; 23:1667-1685; Zharkov et al., 2010, Mutation Research 685, 11-20; Stivers et al., 2001, Arch Biochem Biophys; 396, 1-9; Parikh et al., 2000, Proc Natl Acad Sci USA; 97:5083; Pearl, 2000, Mutat Res 460, 165-181; Lindahl, 1982, Annu Rev Biochem; 51:61-87; and Lindahl et al., 1977, J Biol Chem; 252:3286-3294.

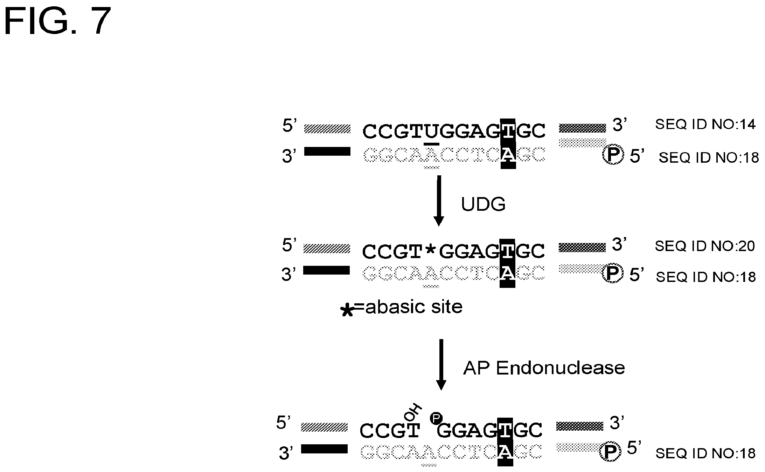

UDG excises uracil from DNA by hydrolyzing the N-glycoside bond between the uracil base and the sugar-phosphate backbone in single- and double-stranded DNA (Bellamy et al., 2007, Nucleic Acids Res; 35:1478-1487; Slupphaug et al., 1996, Nature 384, 87-92; Stivers et al., 1999, Biochemistry; 38:952-963; and Parikh et al., 2000, Mutat Res; 460:183-199), resulting in the formation of an abasic site (AP-site) having a hemiacetal formation. A schematic illustration of the UDG-mediated generation of single nucleotide gaps within double stranded DNA fragments is shown in FIG. 7.

A variety of UDG enzymes are commercially available, including, for example, *E. coli* Uracil-DNA Glycosylase (UDG) (New England Biolabs, Catalog # M0280S, see the worldwide web at neb.com/products/m0280-uracil-dna-glycosylase-udg#Product%20Information) and a heat-labile Uracil DNA Glycosylase (UDG/UNG) isolated from a psychrophilic marine bacteria (Yeasen Biotechnology (Shanghai) Co., Ltd., Catalog #10707ES, see the worldwide web at yeasenbiotech.com/solutiondetail/79?gelid=EAIaIQobChMI_Oie4unY-gIV3xCtBh0hRwGHEAAYASAAEgKsx_D_BwE). In some embodiments, the UDG is of commercial origin.

Reaction conditions suitable for the UDG-mediated excision of uracil from DNA include, but are not limited to, concentration of the single stranded DNA substrate, pH, temperature of the reaction, time of the reaction, and concentration of the UDG enzyme. It is expected that a UDG can function in essentially any buffer. An example of a useful buffer includes, but is not limited to, 1X UDG Reaction Buffer (New England Biolabs, Catalog # B0280S, see the worldwide web at neb.com/products/m0280-uracil-dna-glycosylase-udg#Product%20Information) which is 20 mM Tris-HCl, 1mM DTT, 1mM EDTA (pH 8 at 25°C). Uracil-DNA Glycosylase is active over a broad pH range, with an optimum at pH 8.0, does not require a divalent cation, and is inhibited by high ionic strength (> 200 µM). Uracil-DNA Glycosylase is active in a temperature of 25°C to 37°C and in some embodiments the reaction can proceed in a temperature of 25°C to 37°C. In some embodiments, the reaction can proceed at 37°C. In some embodiments, the reaction can proceed for about 5 minutes, about 10 minutes, about 15 minutes, about 20 minutes, about 30 minutes, about 45 minutes, about 60 minutes, about 90 minutes, about 120 minutes, or any range thereof. In some embodiments, a reaction can include about 0.001U/µl to about 1 U/ µl UDG enzyme, wherein one unit is defined as the amount of enzyme that catalyzes the release of 60 pmol of uracil per minute from double-stranded, uracil-containing DNA. Activity is measured by release of [³H]-uracil in a 50 µl reaction containing 0.2 µg DNA (10⁴-10⁵ cpm/µg) in 30 minutes at 37°C (see the worldwide web at neb.com/products/m0280-uracil-dna-glycosylase-udg#Product%20Information). In some embodiments, a reaction can include about 0.05 U/ µl UDG. In some embodiments, a reaction can include nucleic acids at a concentration of about 1ng to about 1ug of input nucleic acid. In some embodiments, a reaction can include nucleic acids at a concentration of about ~10pM to about 200nM. In some embodiments, a reaction can include nucleic acids at a concentration of about 200pM to about 20nM.

### Endonuclease treatment

The following passage on endonuclease treatment is not encompassed by the wording of the claims. In addition to treatment of dsDNA with an Uracil DNA glycosylase (UDG), which specifically recognizes and removes uracil residues, the ds DNA fragments are treated with an endonuclease, such as, for example, Endonuclease IV (Endo IV) or Apurinic/apyridminic Endonuclease 1 (APE1), which catalyzes the cleavage of the phosphodiester backbone at the abasic site and results in a 3' hydroxyl group, a one nucleotide gap, and a 5' phosphate group. The uracil repair module of these two enzymatic treatments is shown in FIG. 7. In some embodiments, treatment with an Uracil DNA glycosylase and treatment with an endonuclease are carried out as separate, sequential steps. In some embodiments, treatment with an Uracil DNA glycosylase and treatment with an endonuclease occur simultaneously, with both enzymatic processes occurring simultaneously in a single reaction mixture.

### Repair and ligation with alternative base

The following passage on repair and ligation with alternative base is not encompassed by the wording of the claims.

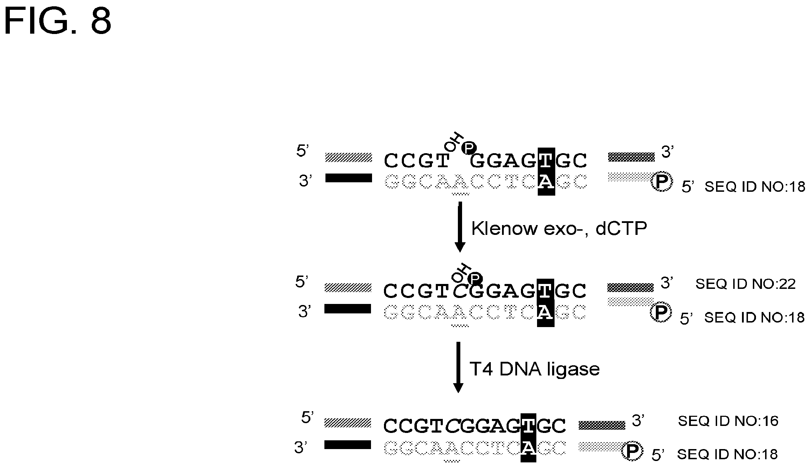

After treatment with an Uracil DNA glycosylase, which excises uracil residues to form abasic sites, and treatment with an endonuclease, which cleaves the phosphodiester backbone at the abasic site, resulting in a 3' hydroxyl group, a single nucleotide gap, and a 5' phosphate group, an alternative nucleotide is incorporated into the 1 nucleotide gap to "correct" the false positive. A representative workflow showing nucleobase replacement at the site of uracil excision is shown in FIG. 8. First, a polymerase is used to incorporate an alternative dNTP into the single nucleotide gap. Then, a ligase ligates across the mismatch to complete the repair of the DNA fragment.

In some embodiments, not encompassed by the wording of the claims, the alternative nucleotide for incorporation into the nick is deoxycytosine triphosphate (dCTP), which returns the DNA to its original sequence. Alternatively, in some embodiments, the alternative nucleotide is inosine triphosphate (dITP) or another universal base. These base options result in a mismatch with the adenine base on the complementary strand.

A polymerase is utilized to mediate the incorporation of the alternative dNTP. In some embodiments, an exonuclease deficient polymerase, such as Klenow exo- may be used to fill the gap with the desired dNTP. Klenow exo- has previously been used for similar applications (Riedl et al., 2015, Nature Communications; 6: 1-11) and has been demonstrated to have tolerance for incorporation of dCTP at an A template, albeit with reduced kinetic efficiency (Joyce et al., 1992, J Biol Chem; 267(34):24485-500; and Caroll et al., 1991, Biochemistry 30(3):804-13). Alternative embodiments include the use of T7 DNA polymerase, T4 DNA polymerase, or Sulfolobus DNA Polymerase IV (Boudsocq et al., 2001, Nucleic Acids Res; 29(22):4607-4616). In some embodiments, an exonuclease deficient polymerase without exonuclease activity, such as for example Klentaq may be used.

Then a ligase is utilized to mediate the incorporation of the dNTP. Any of a number of ligases may be used, including, for example, T4 DNA ligase. T4 DNA ligase is known to be particularly good at directing the ligation of DNA strands regardless of the presence of mismatches proximal to the ligation site (Alexander et al., 2003, Nucleic Acids Research; 31(12):3208-16). Second strand synthesis results in an adenine (A) across from any uracils in the template. Replacement of the U with a C reverses the false positive (FP) conversion, resulting in a nicked strand with a C:A mismatch on the 3' end. Ligation of C:A mismatches has been demonstrated to occur with high efficiency using multiple methods of detection (Alexander et al., 2003, Nucleic Acids Research; 31(12):3208-16; Kim and Mrksich, 2009, Nucleic Acids Research; 38(1):1-10). This would effectively take any FP conversions of C to U and reconvert them U to C. Alternatively, a universal base such as inosine could be used by the polymerase to synthesize an I:A base pair. Inosine amplifies by PCR as a G, which would selectively label any FP conversions with a C to G conversion. Bioinformatic tools could then correct C to G conversions back to C based on knowledge of the reference genome. Other DNA ligases that may be employed include T3 DNA ligase, T7 DNA ligase, or *E. coli* DNA ligase.

Inefficiency in the ligation step may result in loss of reads that contained FP conversions, as in order for PCR to occur, fragments need both adapters. In order to improve the efficiency of the ligation step, ligation reaction conditions may be optimized. In some embodiments, longer ligation reaction times, reduced temperature to promote increased stability of the mismatched end, the addition of DMSO, reduced ATP concentration, and/or low MgCl₂ concentration may be used to improve ligation efficiency. For example, in some embodiments, ligation reactions could be incubated for 16 hours or longer. In some embodiments, reaction mixture of 20% DMSO, reduced ATP concentration (10-100mM ATP), and low MgCl2 concentration (3-10mM MgCl2) can result in improved ligation efficiency in the presence of mismatches (Alexander et al., 2003, Nucleic Acids Research; 31(12):3208-16).

### Degradation of Second Strand

The following passage on degradation of second strand is not encompassed by the wording of the claims.

Following enzymatic repair of the original library fragment, the second strand is then selectively cleaved such that it cannot serve as a template for PCR, thus preventing propagation of the error through its adenine base.

For a second strand that was synthesized with a primer containing a 5' phosphate, lambda exonuclease is used for selective degradation. Degradation with lambda exonuclease is commonly employed to generate single-stranded DNA.

Alternatively, if the second strand was synthesized with primers containing oxoguanine, formamidopyrimidine-DNA glycosylase (FPG) and/or oxoguanine glycosylase (OGG) enzymes may be used to selectively cleave the adapter sequences off of the second strand, rendering it unamplifiable in the downstream PCR step. Likewise, if primers containing inosine were used, Endo V can be used for selective adapter cleavage. Formamidopyrimidine-DNA glycosylase (FPG), such as *Escherichia coli* FPG, is a 30-kDa globular monomer, is a combined DNA glycosylase-AP lyase that removes the damaged bases and cleaves phosphodiester bonds in the DNA backbone next to AP sites (He et al., 2022, *Am J Physiol Lung Cell Mol Physiol;* 282:L50-L55). FPG has a broad range of substrates, such as ring-opened guanine or adenine, oxidized guanine, cytidine, or uridine, and ring-opened and oxidized thymidine. Oxoguanine glycosylase (OGG), including human 8-oxoguanine-DNA glycosylase (hOGG1) possesses similar enzymatic activities to FPG.

Following degradation/cleavage of the second strand, the primary, repaired strands may be amplified using standard PCR. In some embodiments, U-intolerant polymerases may be employed to provide greater assay specificity. Optionally, U-intolerant polymerases may be used in the PCR to further increase the stringency of the assay against false positives.

It will be appreciated that any of the amplification methodologies described herein or generally known in the art may be used with universal or target-specific primers to amplify DNA fragments. Suitable methods for amplification include, but are not limited to, the polymerase chain reaction (PCR), strand displacement amplification (SDA), transcription mediated amplification (TMA) and nucleic acid sequence-based amplification (NASBA), as described in U.S. Pat. No. 8,003,354. The above amplification methods may be employed to amplify one or more nucleic acids of interest. For example, PCR, including multiplex PCR, SDA, TMA, NASBA and the like may be utilized to amplify DNA fragments. In some embodiments, primers directed specifically to the polynucleotide of interest are included in the amplification reaction.

As used herein, "amplify," "amplifying" or "amplification reaction" and their derivatives, refer generally to any action or process whereby at least a portion of a nucleic acid molecule is replicated or copied into at least one additional nucleic acid molecule. The additional nucleic acid molecule optionally includes sequence that is substantially identical or substantially complementary to at least some portion of the target nucleic acid molecule. The target nucleic acid molecule can be single-stranded or double-stranded and the additional nucleic acid molecule can independently be single-stranded or double-stranded. Amplification optionally includes linear or exponential replication of a nucleic acid molecule. In some embodiments, such amplification can be performed using isothermal conditions; in other embodiments, such amplification can include thermocycling. In some embodiments, the amplification is a multiplex amplification that includes the simultaneous amplification of a plurality of target sequences in a single amplification reaction. In some embodiments, "amplification" includes amplification of at least some portion of DNA and RNA based nucleic acids alone, or in combination. The amplification reaction can include any of the amplification processes known to one of ordinary skill in the art. In some embodiments, the amplification reaction includes polymerase chain reaction (PCR).

As used herein, "amplification conditions" and its derivatives, generally refers to conditions suitable for amplifying one or more nucleic acid sequences. Such amplification can be linear or exponential. In some embodiments, the amplification conditions can include isothermal conditions or alternatively can include thermocyling conditions, or a combination of isothermal and thermocycling conditions. In some embodiments, the conditions suitable for amplifying one or more nucleic acid sequences include polymerase chain reaction (PCR) conditions. Typically, the amplification conditions refer to a reaction mixture that is sufficient to amplify nucleic acids such as one or more target sequences, or to amplify an amplified target sequence ligated to one or more adapters, e.g., an adapter-ligated amplified target sequence.

Generally, the amplification conditions include a catalyst for amplification or for nucleic acid synthesis, for example a polymerase; a primer that possesses some degree of complementarity to the nucleic acid to be amplified; and nucleotides, such as deoxyribonucleotide triphosphates (dNTPs) to promote extension of the primer once hybridized to the nucleic acid. The amplification conditions can require hybridization or annealing of a primer to a nucleic acid, extension of the primer and a denaturing step in which the extended primer is separated from the nucleic acid sequence undergoing amplification. Typically, but not necessarily, amplification conditions can include thermocycling; in some embodiments, amplification conditions include a plurality of cycles where the steps of annealing, extending, and separating are repeated. Typically, the amplification conditions include cations such as Mg++ or Mn++ and can also include various modifiers of ionic strength.

As used herein, the term "polymerase chain reaction" (PCR) refers to the method of K. B. Mullis as described in U.S. Pat. Nos. 4,683,195 and 4,683,202, which describes a method for increasing the concentration of a segment of a polynucleotide of interest in a mixture of genomic DNA without cloning or purification. This process for amplifying the polynucleotide of interest consists of introducing a large excess of two oligonucleotide primers to the DNA mixture containing the desired polynucleotide of interest, followed by a series of thermal cycling in the presence of a DNA polymerase. The two primers are complementary to their respective strands of the double-stranded polynucleotide of interest. The mixture is denatured at a higher temperature first and the primers are then annealed to complementary sequences within the polynucleotide of interest molecule. Following annealing, the primers are extended with a polymerase to form a new pair of complementary strands. The steps of denaturation, primer annealing, and polymerase extension can be repeated many times (referred to as thermocycling) to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as the "polymerase chain reaction" (hereinafter "PCR"). Because the desired amplified segments of the polynucleotide of interest become the predominant nucleic acid sequences (in terms of concentration) in the mixture, they are said to be "PCR amplified." In a modification to the method discussed above, the target nucleic acid molecules can be PCR amplified using a plurality of different primer pairs, in some cases, one or more primer pairs per target nucleic acid molecule of interest, thereby forming a multiplex PCR reaction.

In some embodiments, following degradation/cleavage of the second strand, the primary, repaired strands are not subject to PCR amplification prior to sequencing. In some embodiments, a repaired library may be loaded directly onto the sequencer for sequencing without amplification, using procedures and reagents described, for example, on the worldwide at illumina.com/products/by-type/sequencing-kits/library-prep-kits/dna-pcr-free-prep.html.

In some embodiments, the double stranded corrected DNA fragments obtained with amplification may be sequenced. Sequencing may be by any of a variety of known methodologies, including, but not limited to any of a variety high-throughput, next generation sequencing (NGS) platforms, including, but not limited to, sequencing by synthesis, sequencing by ligation, nanopore sequencing, Sanger sequencing, and the like. In some embodiments, sequencing is performed using the sequencing by synthesis methodologies commercialized by ILLUMINA^{®} as described in U.S. Patent Application Publication No. 2007/0166705, U.S. Patent Application Publication No. 2006/0188901, U.S. Pat. No. 7,057,026, Beijing Genomics Institute (BG) as described in Carnevali et al., 2012, J Comput Biol; 9(3):279-92, or the ion semiconductor sequencing methodologies of ION TORRENT^{™} as described in US 2009/0026082 A1; US 2009/0127589 A1; US 2010/0137143 A1; or US 2010/0282617 A1.

Next Generation Sequencing (NGS) refers to sequencing methods that allow for massively parallel sequencing of clonally amplified molecules and of single nucleic acid molecules. Non-limiting examples of NGS include sequencing-by-synthesis using reversible dye terminators, and sequencing-by-ligation.

Preferred embodiments include sequencing-by-synthesis (SBS) techniques. SBS techniques generally involve the enzymatic extension of a nascent nucleic acid strand through the iterative addition of nucleotides against a template strand.

In some embodiments, rather than sequencing, the readout may be obtained by the use of an array, using for example, procedures as described on the worldwide web illumina.com/techniques/microarrays/methylation-arrays.html.

In addition to preventing false positive detection of 5-methylcytosine (5mC) and/or 5-hydroxymethylcytosine (5hmC) due to the deamination of unmethylated cytosines in an assay using a cytosine deaminase to selectively deaminate methylated cytosines, the Uracil Enzymatic Removal And Substitution at Errors (U-ERASE) methods described herein have additional applications.

The U-ERASE methods described herein replace uracil residues with cytosine residues (or other alternative residues) and can be applied to situations where efficient amplification of long DNA targets is required. PCR amplification of long targets (≥ 5 - 6 kbp) can be difficult due to various factors affecting the processivity of thermostable polymerases (Barnes, 1994, Proc Natl Acad Sci U S A; 91:2216-2220). A key impediment to long-range PCR is the prolonged heat exposure of template DNA and dNTPs to heat during thermal cycling conditions required for amplification of kilobase-long targets. Barnes (Barnes, 1994, Proc Natl Acad Sci U SA; 91:2216-2220) proposed single-stranded template DNA present in PCR is highly susceptible to depurination events, resulting in abasic sites which cannot be traversed by many polymerases. Hogrefe et al. (Hogrefe et al., 2022, Proc Natl Acad Sci U S A; 99:596-601) noted spontaneous deamination of dCTP to dUTP during PCR leads to mis-incorporation and accumulation of uracil into amplicons, which will then inhibit proofreading polymerases such as *Pfu* and other B-family polymerases (Greagg et al., 1999, Proc Natl Acad Sci U S A; 96:9045-9050). Additional thermal damage to the template, such as cytosine deamination to uracil, further inhibit PCR efficiency and introduce additional sequence errors (Pienaar et al., 2006, Comput Biol Chem; 30:102-111). To mitigate the accumulation of numerous cytosine to uracil lesions in template DNA during PCR of long DNA templates, the U-ERASE methods described herein can be employed to correct errors before resuming PCR and aiding the efficient generation of long and accurate PCR amplicons. In some embodiments, long a DNA template is at least about 5 to 6 kilobases (kbp) or greater in length.

The U-ERASE methods described herein can be used to provide for accurate and comprehensive genomic studies of formalin-fixed paraffin embedded (FFPE) material. A major source of sequencing error and noise in the study of FFPE tissues is cytosine deamination (Chen et al., 2014, Mol Diagn Ther; 18:587-593). While there are on-market solutions (including, but not limited to New England Biolabs NEBNext^{®} FFPE DNA Repair Mix and Oxford Gene Technology SureSeq FFPE DNA Repair Mix) and published solutions (Chen et al., 2017, Science; 355:752-756) for repairing cytosine deamination in FFPE, they rely on repair methods dependent on dsDNA duplexes to template correct replacement of U (deaminated cytosine) with C. However, FFPE DNA is commonly partially single-stranded, either due to DNA damage due to fixation and storage or the process by which it is extracted from paraffin (Stiller et al., 2016, Oncotarget; 7:59115-59128). The U-ERASE methods described herein may be used to selectively replace uracils due to the deamination of cytosines with cytosines in single-stranded DNA fragments obtained from formalin-fixed paraffin embedded (FFPE) genomic material.

The U-ERASE methods described herein can be applied to enable the efficient amplification with proofreading polymerases and accurate sequencing by NGS methods of ancient DNA samples and forensic DNA samples. Cytosine deamination is a contributor to sources of error and inefficiency in the amplification and study of ancient DNA (Gilbert et al., 2007, Nucleic Acids Research; 35: 1-10; and Hofreiter et al., 2001, Nucleic Acids Research; 29:4793-4799) and forensic DNA analysis (Gorden et al., 2018, Forensic Sci Int Genet; 34:257-264). Similar to the application of U-ERASE to aid efficient amplification of long targets in PCR, U-ERASE may be applied to replace uracil with cytosine and enable both efficient amplification with proofreading polymerases and accurate sequencing by NGS methods of ancient DNA and/or forensic DNA samples. Following these treatments, subjecting the sample to polymerase chain reaction (PCR) amplification results double stranded corrected fragments DNA.

The present disclosure also provides kits for undertaking a U-ERASE method not encompassed by the wording of the claims, for the enzymatic removal of uracil residues due to deamination of unmethylated cytosines in an assay using a cytosine deaminase to deaminate methylated cytosines.

In some embodiments not encompassed by the wording of the claims, a kit may include at least one or more of a cytosine deaminase, primers comprising a 5' phosphate group, a polymerase, dNTPs, an uracil DNA glycosylase (UDG), an endonuclease, a ligase, an alternative nucleotide (such as dCTP, dITP, or other universal base), and/or a lambda exonuclease in a suitable packaging material in an amount sufficient for at least one reaction. In some embodiments, the cytosine deaminase is an altered APOBEC.

In some embodiments not encompassed by the wording of the claims, a kit may include at least one or more of a cytosine deaminase, primers containing 8-oxoguanine or inosine residues, a polymerase, dNTPs, an uracil DNA glycosylase (UDG), an endonuclease, a ligase, an alternative nucleotide (such as dCTP, dITP, or other universal base), formamidopyrimidine-DNA glycosylase (FPG), oxoguanine glycosylase (OGG), and/or an Endonuclease V in a suitable packaging material in an amount sufficient for at least one reaction. In some embodiments, the cytosine deaminase is an altered APOBEC.

A kit may include one or more other components. Examples of other components include, for example, a positive control polynucleotide or a negative control polynucleotide. Optionally, other reagents such as buffers and solutions are also included. Instructions for use of the packaged components are also typically included.

As used herein, the phrase "packaging material" refers to one or more physical structures used to house the contents of the kit. The packaging material is constructed by known methods, preferably to provide a sterile, contaminant-free environment. The packaging material has a label which indicates that the components can be used for the removal of uracils due to deamination of unmethylated cytosines in an assay using a cytosine deaminase to deaminate methylated cytosines. In addition, the packaging material contains instructions indicating how the materials within the kit are employed to practice a U-ERASE method as described herein. As used herein, the term "package" refers to a solid matrix or material such as glass, plastic, paper, foil, and the like, capable of holding within fixed limits the polypeptides. "Instructions for use" typically include a tangible expression describing the reagent concentration or at least one assay method parameter, such as the relative amounts of reagent and sample to be admixed, maintenance time periods for reagent/sample admixtures, temperature, buffer conditions, and the like.

### EXAMPLES

### Example 1

### Chemoenzymatic Uracil Replacement of Nucleobases (ChURN)

Following the method detailed in FIG. 1, false positive conversion of Cs->Us in an APOBEC methylation detection assay will be corrected. After treatment of DNA libraries with the engineered cytidine deaminase, uracil DNA Glycosylase (UDG) will be used to catalyze the selective de-glycosylation of uracil residues, resulting in an abasic site. The formation of abasic sites results in hemiacetal formation which readily equilibrates with an aldehyde functional group. This aldehyde can subsequently be conjugated with a cytosine nucleobase analog bearing a reactive functional group, such as a hydroxylamine or a hydrazine. This results in a DNA library fragment in which false positive uracil residues have been corrected to cytosine, thus permitting downstream amplification and sequencing with improved methylation specificity.

Library fragments will be treated with an engineered cytidine deaminase enzyme that preferentially deaminates 5mC>T, with some off-target C>U activity. In order to correct these false positives, libraries will be first treated with uracil DNA glycosylase (UDG) to generate an abasic site. Libraries will then be treated with a reactive cytosine analog that selectively reacts with abasic sites to "repair" false positive sites. These libraries will then be amplified, propagating the corrected library sequence.

### Library preparation and APOBEC treatment

DNA libraries are first prepared from the input sample material such that adapter sequences are ligated to library fragments to facilitate downstream workflow steps. Any of many available library preparation protocols may be used. In order to maximize the overall performance of the enzymatic error-correction, libraries may be prepared targeting a shorter insert size, for example 100-200bp, in order to minimize the number of potential false-positive uracils that may be present in any individual library fragment. By minimizing the number of false positives in each fragment, the probability of successfully repairing library fragments and mediating their downstream amplification may be increased, thus preserving library quality and diversity. Once library fragments with adapter sequences have been prepared, libraries are denatured and subjected to ILMN cytidine deaminase mutants selective for mC deamination.

### Treatment with UDG and chemical treatment to install cytosine nucleobases

Because false positive (cytosine) deamination results in uracil bases, and true positive (methylcytosine) bases result in thymine bases, uracil DNA glycosylase (UDG) will be utilized to specifically recognize and remove uracil bases, thus removing the false positive signal and preventing its propagation as a "T" in downstream amplification and sequencing. APOBEC enzymes require ssDNA for recognition, and thus deaminated DNA will be single stranded. UDG recognizes both dsDNA and ssDNA (see the worldwide web at neb.com/products/m0280-uracil-dna-glycosylase-udg#Product%20Information).

The resulting abasic sites contain hemiacetal functional groups which readily equilibrate with the aldehyde form. Aldehyde functional groups are known to react readily with amine groups to form imine groups; typically hydroxylamine and hydrazine functional groups are more commonly employed, as the resulting oxime or hydrazone linkages have greater hydrolytic stability (see the worldwide web at thermofisher.com/us/en/home/references/molecular-probes-the-handbook/reagents-for-modifying-groups-other-thanthiols-or-amines/hydrazines-hydroxylamines-and-aromatic-amines-for-modifying-aldehydes-and-ketones.html; Kalia and Raines, 2008, Angew Chem Int Ed Engl; 47(39):7523-7526; and US 2022/0090179 A1). Therefore, treatment of the UDG-treated library fragments with hydroxylamine-cytosine derivatives or hydrazine-cytosine derivatives will result in the installation of a cytosine base (FIG. 2).

Treatment with an engineered APOBEC (specifically engineered to deaminate 5mC) may result in undesired cytosine to uracil deamination. As shown in FIG. 2, enzymatic treatment with Uracil DNA glycosylase first specifically deglycosylates uracil residues, forming an abasic site. This abasic site, bearing an aldehyde functional group, can then be treated with hydroxylamine-cytosine, resulting in a relatively hydrolytically stable oxime linkage to install a cytosine base. Alternatively, treatment with hydrazine-cytosine results in a hydrazone linkage to install a cytosine base.

Similar reactive cytosine derivatives have been previously reported to be successfully incorporated into DNA fragments, with an estimated efficiency of approximately 88% (Wang et al., 2021, ACS Central Science; 7(6):973-79). These cytosine derivatives are easily synthesized, with an example synthetic scheme for the generation of hydroxylamine-cytosine shown in FIG. 3. Various chemical reactions for the synthesis of reactive cytosines are shown in FIG. 4. Examples of hydroxylamine aldehyde reactive probes for abasic sites detection include, for example, Kubo et al., 1992, Biochemistry; 31(14):3703-3708; Bennett and Kitner, 2006, Nucleosides Nucleotides Nucleic Acids; 25(7):823-42; Wei et al., 2019, DNA Repair (Amst); 27:9-18; and Wilson and Kool, 2019, J Am Chem Soc; 141(49):19379-19388. Examples of hydrazide probes include, for example, Zhang et al., 2019, Mol Cell; 74:1304-1316.e8. Examples of hydrazine reactive probes include, for example, Melton et. al., 2014. Chem Res Toxicol; 27:2113-2118; and Gamboa Varela et. al., 2015, Angew Chem Int Ed Engl; 54(26): 7666-7669.

### Amplification of corrected library fragments

Following installation of the reactive cytosine compound, resulting repaired library fragments will be amplified for NGS analysis. Importantly, DNA molecules with oxime linkages have been previously reported to amplify successfully using standard PCR polymerases (Wang et al., 2021, ACS Central Science; 7(6):973-79).

### Example 2

### Chemoenzymatic Uracil Replacement of Nucleobases in Deaminated NA12878 Genomic DNA

NA12878 genomic DNA will be combined with fully unmethylated lambda control DNA and enzymatically CpG methylated pUC19 control DNA and mechanically sheared to give fragments of approximately ~300bp. This sheared DNA (50ng) will then be subjected to end-repair, A-tailing, and adapter ligation according to standard Illumina library preparation procedures. The adapter ligated DNA will be denatured via incubation in 0.02 N sodium hydroxide at 50°C for 10 minutes. Subsequently, ssDNA samples will be enzymatically deaminated in 50 mM Bis-Tris (pH 6.5), 1 mM DTT, 0.2 mg/mL BSA, 5 µg/mL RNAse A, 1M betaine with the 5mC-selective cytidine deaminase (200nM) for 30 minutes at 37°C, followed by a SPRI purification.

The libraries will then be subjected to treatment with Uracil DNA glycosylase (New England Biolabs) for 30 minutes at 37°C and subsequently SPRI purified. The purified DNA will be treated with 10 mM of the hydroxylamine cytosine derivative in MES buffer (pH 6.0) at 37 °C for 2-6 hours to yield repaired DNA fragments. Following a SPRI purification, libraries will be PCR amplified using unique-dual indexing primers (Q5 HiFi, New England Biolabs) using 12 cycles of PCR. Samples will be sequenced on a NovaSeq6000.

### Example 3

### Additional applications for CHURN

Chemoenzymatic Uracil Replacement of Nucleobases (CHURN) has additional applications. CHURN, which can replace uracil and/or abasic sites with cytosine (or another nucleobase functionalized with the correct hydroxylamine or hydrazine linkage), will be useful in applications beyond repairing non-desirable cytosine to uracil (C->U) activity by modified cytidine deaminases in methylation assays.

### Long-range PCR

PCR amplification of long targets (≥ 5 - 6 kbp) can be difficult due to various factors affecting the processivity of thermostable polymerases (Barnes, 1994, Proc Natl Acad Sci U S A; 91:2216-2220). A key impediment to long-range PCR is the prolonged heat exposure of template DNA and dNTPs to heat during thermal cycling conditions required for amplification of kilobase-long targets. Barnes (Barnes, 1994, Proc Natl Acad Sci U S A; 91:2216-2220) proposed single-stranded template DNA present in PCR is highly susceptible to depurination events, resulting in abasic sites which cannot be traversed by many polymerases. Hogrefe et al. (Hogrefe et al., 2022, Proc Natl Acad Sci U S A; 99:596-601) noted spontaneous deamination of dCTP to dUTP during PCR leads to mis-incorporation and accumulation of uracil into amplicons, which will then inhibit proofreading polymerases such as *Pfu* and other B-family polymerases (Greagg et al., 1999, Proc Natl Acad Sci U S A; 96:9045-9050). Additional thermal damage to the template, such as cytosine deamination to uracil, further inhibit PCR efficiency and introduce additional sequence errors (Pienaar et al., 2006, Comput Biol Chem; 30:102-111).

CHURN may be applied to situations where efficient amplification of long DNA targets is required. To mitigate the accumulation of numerous cytosine to uracil lesions in template DNA during long PCR, CHURN can be employed to correct errors before resuming PCR and aiding the efficient generation of long and accurate PCR amplicons.

### Formalin-Fixed Paraffin Embedded Tissues

A major source of sequencing error and noise in the study of formalin-fixed paraffin embedded (FFPE) tissues is cytosine deamination (Chen et al., 2014, Mol Diagn Ther; 18:587-593). While there are on-market solutions (including, but not limited to New England Biolabs NEBNext^{®} FFPE DNA Repair Mix and Oxford Gene Technology SureSeq FFPE DNA Repair Mix) and published solutions (Chen et al., 2017, Science; 355:752-756) for repairing cytosine deamination in FFPE, they rely on repair methods dependent on dsDNA duplexes to template correct replacement of U (deaminated cytosine) with C. However, FFPE DNA is commonly partially single-stranded, either due to DNA damage due to fixation and storage or the process by which it is extracted from paraffin (Stiller et al., 2016, Oncotarget; 7:59115-59128). CHURN is distinct from alternative solutions in its ability to replace uracil with cytosine in the absence of double-stranded DNA and will provide for more accurate and comprehensive genomic studies of FFPE material.

### Recovery and study of ancient DNA, forensic DNA

Cytosine deamination is a contributor to sources of error and inefficiency in the amplification and study of ancient DNA (Gilbert et al., 2007, Nucleic Acids Research; 35:1-10; and Hofreiter et al., 2001, Nucleic Acids Research; 29:4793-4799) and forensic DNA analysis (Gorden et al., 2018, Forensic Sci Int Genet; 34:257-264). Similar to the application of CHURN to aid efficient amplification of long targets in PCR, CHURN may be applied to replace uracil with cytosine and enable both efficient amplification with proofreading polymerases and accurate sequencing by NGS methods.

### Example 4

### Uracil Enzymatic Removal And Substitution at Errors (U-ERASE) with second strand synthesis with a primer containing a 5' phosphate and selective degradation with lambda exonuclease

APOBEC is a cytidine deaminase that recognizes single-stranded DNA and catalyzes the deamination of cytosine (C) to uracil (U), 5-methylcytosine (5mC) to thymine (T), and 5-hydroxymethylcytosine to 5-hydroxymethyluracil. Protein engineering of APOBEC3A has resulted in mutant APOBEC proteins with selectivity towards deamination of 5mC with reduced activity towards deamination of C, however residual activity for deamination of C remains. The Y130A/Y132H double mutant of the cytidine deaminase APOBEC3A demonstrates an ability to convert 5mCs to T in the human genome.

Smaller genomes with known methylation status of Cs in CpG sites were subjected to cytidine deamination with the Y130A/Y132H double mutant of APOBEC3A. Specifically, pUC19 plasmid, in which all Cs in CpG sites are methylated, and lambda DNA, in which all Cs in CpG sites are unmethylated, were treated with the Y130A/Y132H double mutant of APOBEC3A. In CpG methylated pUC19, ~80% of 5mCs in CpG sites were converted to T. In unmethylated lambda DNA, ~9% of Cs in CpG sites were converted to U, indicating a high level of false positive conversion of unmethylated Cs. Furthermore, when looking at individual reads for unmethylated lambda DNA, ~75% of reads contained at least 1 C to U false positive conversion when looking at all C contexts (CpG, CHG, and CHH).

Described in this example is a method for correcting false positive conversion of Cs->Us in a methylation detection assay that uses an engineered cytidine deaminase for selective mC deamination. As shown in FIG. 5, the method relies on creating a complement to each ssDNA library fragment, followed by removal of all uracil bases by Uracil DNA Glycosylase (UDG), gap formation using an AP endonuclease, and polymerase/ligase-mediated insertion of dCTP, dITP, or other universal base. Subsequently, the second strand is selectively degraded, and the repaired DNA strand is selectively amplified via PCR. First, as shown in FIG. 5, single stranded DNA libraries are subjected to an engineered cytidine deaminase, resulting in mC>T and some off-target C>U deamination. Then, a second strand of Uracil Enzymatic Removal And Substitution at Errors (U-ERASE) DNA is synthesized. Subsequently, libraries are treated with UDG and an AP endonuclease, resulting in the removal of uracil bases and 1 nt gaps at those sites. These gaps are repaired through treatment with a polymerase, dCTP, and a ligase, resulting in a repaired DNA strand. Subsequently, the second strand is selectively degraded, allowing for selective amplification of the original DNA strand via PCR

### Library preparation and APOBEC treatment

DNA libraries are first prepared from the input sample material such that adapter sequences are ligated to library fragments to facilitate downstream workflow steps. Many possible library preparation protocols are compatible with the method. Adapters may contain phosphorothioate bonds near the 5' end. Failure to efficiently repair uracil within a fragment will result in the loss of that molecule, reducing overall library complexity. In order to maximize the overall performance of the enzymatic error-correction, libraries may be prepared targeting a shorter insert size, for example 100-200bp, in order to minimize the number of potential false-positive uracils that may be present in any individual library fragment. By minimizing the number of false positives in each fragment, the probability of successfully repairing all uracils present in library fragments and mediating their downstream amplification may be increased, thus preserving library quality and diversity. Once library fragments with adapter sequences have been prepared, libraries are denatured and subjected to a cytidine deaminase selective for mC deamination.

### Synthesis of Second strand

APOBEC-mediated deamination of DNA libraries is typically carried out on single-stranded DNA (ssDNA). In order to mediate enzymatic repair of undesired uracil residues, a second strand of DNA is first synthesized. When synthesizing the complement strand, all cytosines that have been converted to uracils (false positives) will be copied as adenines in the 2nd strand. In order to facilitate the sequencing of only the original strand, this 2nd strand of DNA, which serves as a scaffold for enzymatic repair, is marked in order to allow for selective degradation in downstream steps.

With this example, second strand synthesis can be carried out using a primer binding to the library adapter sequence at the 3' end. Specifically, this primer contains a 5'phosphate group to tag the second strand (FIG. 6A). A variety of standard polymerases that tolerate uracil may be utilized in this step. The presence of a 5'phosphate on the second strand enables its selective degradation later in the workflow using lambda exonuclease. Lambda exonuclease can degrade DNA without a 5'phosphate, albeit this occurs much slower than degrading DNA with a 5'phosphate. In order to improve selectivity of Lambda exonuclease to degrade only the synthesized 2nd strand, during library prep, adapters with one or more phosphorothioate bonds can be utilized.

As shown in Fig. 6A, the second strand is synthesized using a 5'phosphorylated primer. After the uracil repair module, lambda exonuclease, which is specific for DNA strands with a 5' phosphate, is used to selectively degrade the second strand. Fig. 6B shows an alternative strategy for second strand synthesis, described in more detail in Example 2, that leverages extension-ligation and adapter sequences with modified bases (either 8-oxoguanine or inosine). After the uracil repair module, FPG or OGG (for 8-oxoG) or Endo V (inosine), is used to cleave the adapter sequences, rendering the second strand unamplifiable during PCR.

### Cleavage of uracils

Once dsDNA library fragments have been generated, the libraries are treated with an Uracil DNA glycosylase (UDG), which specifically recognizes and removes uracil residues, resulting in an abasic site. The DNA is also treated with an endonuclease, such as Endonuclease IV (Endo IV) or Apurinic/apyridminic Endonuclease 1 (APE1), which catalyzes the cleavage of the phosphodiester backbone at the abasic site and results in a 3' hydroxyl group, 1 nucleotide gap, and 5' phosphate group (FIG. 7). Optionally, both enzymatic processes may occur in a single reaction.

FIG. 7 shows the processes of uracil excision from library fragments. Uracil DNA glycosylase (UDG) first removes the uracil base from the DNA polynucleotide. Then, an AP endonuclease cleaves the phosphodiester backbone, resulting in a 3'hydroxyl group, 1 nt gap, and 5' phosphate. The two possible endonucleases that may be used include AP Endonuclease 1 or Endonuclease IV.

### Repair and ligation of an alternative base

After excision of the uracil, a targeted nucleotide is incorporated into the 1 nucleotide gap to "correct" the false positive. FIG. 8 shows a representative workflow showing nucleobase replacement at the site of uracil excision. First, Klenow exo- incorporates dCTP into the 1 nucleotide gap. Then, T4 DNA ligase ligates across the mismatch to complete repair of the library fragment.

Cytosine triphosphate (dCTP) is the preferred substrate for incorporation into the nick, as it would return the DNA to its original sequence. Alternatively, inosine triphosphate (dITP) or another universal base may be utilized. These base options result in a mismatch with the adenine base on the complementary strand. In order to mediate the incorporation of the dNTP, an exonuclease deficient polymerase, such as Klenow exo- may be utilized. In one iteration, Klenow exo- can be used to fill the gap with the desired dNTP. Klenow exo- has previously been used for similar applications (Riedl et al., 2015, Nature Communications; 6:1-11) and has been demonstrated to have tolerance for incorporation of dCTP at an A template, albeit with reduced kinetic efficiency (Joyce et al., 1992, Journal of Biological Chemistry; 267(34):24485-500; and Caroll et al., 1991, Biochemistry 30(3):804-13). Alternative polymerases may include T7 DNA polymerase, T4 DNA polymerase, or Sulfolobus DNA Polymerase IV.

Efficiency of ligation containing a mismatch at the 3' end is dependent on the ligase identity. While a number of ligases may work for this step, in one iteration, T4 DNA ligase may be used to ligate the repaired DNA. T4 DNA ligase is known to be particularly good at directing the ligation of DNA strands regardless of the presence of mismatches proximal to the ligation site (Alexander et al., 2003, Nucleic Acids Research; 31(12):3208-16).

Second strand synthesis results in an A across from any U in the template. Replacement of the U with a C reverses the FP conversion, resulting in a nicked strand with a C:A mismatch on the 3' end. Ligation of C:A mismatches was demonstrated to occur with high efficiency using multiple methods of detection (Alexander et al., 2003, Nucleic Acids Research; 31(12):3208-16; Kim and Mrksich, 2009, Nucleic Acids Research; 38(1):1-10). This would effectively take any FP conversions C->U and reconvert them U->C. Alternatively, a universal base such as inosine could be used by the polymerase to synthesize an I:A base pair. Inosine amplifies by PCR as a G, which would selectively label any FP conversions with a C->G conversion. Bioinformatic tools could then correct C->G conversions back to C based on knowledge of the reference genome. Other DNA ligases that may be employed include T3 DNA ligase, T7 DNA ligase, or *E. coli* DNA ligase.

Any inefficiency in the ligation step would result in loss of reads that contained FP conversions, as in order for PCR to occur, fragments need both adapters. In order to improve the efficiency of the ligation step, ligation reaction conditions can be optimized. Longer ligation reaction times have been shown to improve ligation efficiency. Ligation reactions could be incubated for 16 hours or longer to improve this step. Additionally, temperature can be reduced to promote increased stability of the mismatched end. A report optimizing ligation of at a mismatched end found that 20% DMSO, reduced ATP concentration (10-100mM ATP), and low MgCl2 concentration (3-10mM MgCl2) resulted in improved ligation efficiency in the presence of mismatches (Alexander et al., 2003, Nucleic Acids Research; 31(12):3208-16).

### Cleavage of 2nd strand and amplification of repaired DNA

Following enzymatic repair of the original library fragment, the second strand is selectively cleaved such that it cannot serve as a template for PCR, thus preventing propagation of the error through its adenine base. For a second strand that was synthesized with a primer containing a 5' phosphate, lambda exonuclease is used for selective degradation. This strategy is commonly employed to generate single stranded DNA (Avci-Adali et al., 2010, Molecules; 15(1): 1-11). The presence of phosphorothioate bonds on the original library fragments may improve the selectivity of degradation. Alternatively, if the second strand was synthesized with primers containing oxoguanine as described in more detail in Example 2, FPG/OGG enzymes can be used to selectively cleave the adapter sequences off of the second strand, rendering it unamplifiable in the downstream PCR step. Likewise, if primers containing inosine were used, Endo V can be used for selective adapter cleavage.

Following degradation/cleavage of the second strand, the primary, repaired strands can be amplified using standard PCR. Optionally, as shown in FIG. 6, U-intolerant polymerases may be used in the PCR to further increase the stringency of the assay against false positives.

### Secondary Considerations

Existing methylation sequencing assays utilize APOBEC proteins to carry out deamination of unmethylated cytosine residues (for example, NEB EM-Seq). While wild-type APOBEC3A is known to deaminate both cytosine and 5-methylcytosine (Schutsky et al., 2017, Nucleic Acids Research; 45(13):7655-65), no existing methods have been established that leverage selective APOBEC enzymes along with an uracil-specific error correction strategy in order to facilitate methylation sequencing. Furthermore, because enzymatic error correction typically requires dsDNA, application of such methods to ssDNA, the required substrate for APOBEC deamination, is non-obvious. To facilitate such enzymatic repair, the method described in this example includes the following innovations: (1) Synthesis of a tagged second strand that will facilitate its downstream degradation, (2) enzymatic removal and replacement of the uracil base with a mismatched base, such as cytosine, and (3) selective degradation or cleavage of the second strand used to facilitate enzymatic repair.

### Example 5

### Uracil Enzymatic Removal And Substitution at Errors (U-ERASE) with second strand synthesis with oligonucleotides

Alternatively, second strand synthesis in the method described in the example above may be undertaken with the annealing of oligonucleotides to the ssDNA to facilitate enzymatic repair. Primers containing 8-oxoguanine or inosine residues may be used to mark the second strand for degradation. As shown in FIG. 6B, one primer binds the 3' end of the library fragment and mediates polymerization of the library insert, while another primer binds the 5' end of the library fragment and is incorporated through ligation. A mixture of enzymes containing a polymerase (for example, an exonuclease-deficient Taq polymerase, which is uracil-tolerant) and a ligase (for example, Taq DNA ligase) can be used to generate the tagged 2nd strand. The polymerase employed for this reaction is not required to have high fidelity, as the resulting strand is not sequenced. The presence of the 8-oxoguanine or inosine residues enables the selective cleavage of the adapter sequences later in the workflow using FPG/OGG, or Endonuclease V, respectively. As shown in Fig. 6B, this alternative strategy for second strand synthesis leverages extension-ligation and adapter sequences with modified bases (either 8-oxoguanine or inosine). After the uracil repair module, FPG/OGG or Endo V, for 8-oxoG and inosine, respectively, is used to cleave the adapter sequences off of the second strand, rendering the second strand unamplifiable the downstream PCR step.

### SEQUENCE INFORMATION

SEQ ID NO: 1
   zinc-binding motif H-[P/A/V]-E-X[23-28]-P-C-X[2-4]-C
SEQ ID NO: 2
   ZDD motif: HXEX₂₄SW(S/T)PCX_{[2-4]}CX₆FX₈LX₅R(L/I)YX_{[8-11]}LX₂LX_{[10]}M
SEQ ID NO: 3
   altered cytidine deaminase includes the amino acids of a member of the APOBEC3A subfamily: 
X[16-26]-GRXXTXLCYXV-X15-GXXXN-X12-HAEXXF-X14-YXXTWXXSWSPC- X[2-4]-CA-
X5-FL-X7-LXIXXXR(L/I)Y-X8-GLXXLXXXG-X5-M-X4-FXXCWXXFV-X6-FXPW-X13-
LXXI- X[2-6]
SEQ ID NO: 4
   altered cytidine deaminase includes the amino acids of a member of the APOBEC3A subfamily: 
X26-GRXXTXLCYXV-X15-G-X16-HAEXXF-X14-YXXTWXXSWSPC-X4-CA-X5-FL-X7-
LXIFXXR(L/I)Y-X8-GLXXLXXXG-X5-M-X4-FXXCWXXFV-X6-FXPW-X13-LXXI-X6
SEQ ID NO: 5
   Altered cytosine deaminase (SG1) - synthetic construct: 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARIADYDPLYKEALQMLRDAGAQVSIMTYDEFKHCWDTFVDHQGCPFQP
WDGLDEHSQALSGRLRAILQNQGN
SEQ ID NO: 6
   Altered cytosine deaminase (SG2) - synthetic construct: 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARIADHDPLYKEALQMLRDAGAQVSIMTYDEFKHCWDTFVDHQGCPFQP
WDGLDEHSQALSGRLRAILQNQGN
SEQ ID NO: 7
   Altered cytosine deaminase- synthetic construct
   APOBECC3A with (Y130L) 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARILDYDPLYKEALQMLRDAGAQVSIMTYDEFKHCWDTFVDHQGCPFQP
WDGLDEHSQALSGRLRAILQNQGN
SEQ ID NO: 8
   Altered cytosine deaminase - synthetic construct
   APOBEC3A with (Y130W) 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARIWDYDPLYKEALQMLRDAGAQVSIMTYDEFKHCWDTFVDHQGCPFQP
WDGLDEHSQALSGRLRAILQNQGN
SEQ **ID** NO: 9
   Altered cytosine deaminase (SG1 with Y130X) 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARIXDYDPLYKEALQMLRDAGAQVSIMTYDEFKHCWDTFVDHQGCPFQP
WDGLDEHSQALSGRLRAILQNQGN (wherein X can be A, G, F, H, Q, M, N, K, V, D, E, S,
C, P, or T, preferably A)
SEQ ID NO: 10
   Altered cytosine deaminase - synthetic construct 
X26-GRXXTXLCYXV-X15-G-X16-HAEXXF-X14-YXXTWXXSWSPC-X4-CA-X5-FL-X7-
LXIFXXR(L/I)Z-X8-GLXXLXXXG-X5-M-X4-FXXCWXXFV-X6-FXPW-X13-LXXI-X6
 (wherein Z is A, G, F, H, Q, M, N, K, V, D, E, S, C, P, or T, and the number after an X refers to the number of amino acids present)
SEQ ID NO: 11
   Altered cytosine deaminase - synthetic construct 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARIXDZDPLYKEALQMLRDAGAQVSIMTYDEFKHCWDTFVDHQGCPFQP
WDGLDEHSQALSGRLRAILQNQGN, (wherein X can be A, L, or W, preferably A; and Z is
selected from R, H, L, or Q, preferably H).
SEQ ID NO: 12
   Wild Type human APOBEC3A protein (UniProt: P31941) 
MEASPASGPRHLMDPHIFTSNFNNGIGRHKTYLCYEVERLDNGTSVKMDQHRGFLHNQ
AKNLLCGFYGRHAELRFLDLVPSLQLDPAQIYRVTWFISWSPCFSWGCAGEVRAFLQEN
THVRLRIFAARIYDYDPLYKEALQMLRDAGAQVSIMTYDEFKHCWDTFVDHQGCPFQP
WDGLDEHSQALSGRLRAILQNQGN
SEQ ID NO: 13
   CCGTCGGAGCGC
   (wherein the bold C is 5-methylcytosine)
SEQ ID NO: 14
   CCGTUGGAGTGC
SEQ ID NO:15
   CCGTNGGAGTGC
   (wherein N is an abasic site)
SEQ ID NO: 16
   CCGTCGGAGTGC
SEQ ID NO: 17
   CGACACCGACGG
SEQ ID NO: 18
   GGCAACCTCAGC
SEQ ID NO: 19
   GGCAGCCTCAGC
SEQ ID NO: 20
   CCGTTGGAGTGC
SEQ ID NO: 21
   CCGTNGGAGTGC
   (wherein N is an abasic site)

## Claims

1. A method of preventing false positive detection of 5-methylcytosine (5mC) and/or 5-hydroxymethylcytosine (5hmC) due to deamination of unmethylated cytosines in an assay using a cytosine deaminase to selectively deaminate methylated cytosines, the method comprising:
providing a sample comprising single stranded DNA library fragments in which a cytosine deaminase has deaminated methylated cytosines;
contacting the sample with an uracil DNA glycosylase (UDG) to deglycosylate uracil residues to form abasic sites having a hemiacetal formation within the single stranded DNA library fragments; and
contacting the sample with a reactive cytosine nucleobase analog to install a cytosine at abasic sites thru a noncanonical linkage resulting in double stranded DNA corrected library fragments.

2. The method of claim 1, further comprising subjecting the sample to polymerase chain reaction (PCR) amplification.

3. The method of claim 1 or 2, wherein the DNA library fragments are about 100bp to about 200bp in length, and wherein preferably, the DNA library fragments comprise 5' and/or 3' adapter sequences.

4. The method of any one of claims 1 to 3 further comprising sequencing the corrected library fragments.

5. The method of any one of claims 1 to 4, wherein the cytosine deaminase comprises an altered cytosine deaminase,
wherein preferably the altered cytosine deaminase is a member of the AID subfamily, the APOBEC1 subfamily, the APOBEC2 subfamily, the APOBEC3A subfamily, the APOBEC3B subfamily, the APOBEC3C subfamily, the APOBEC3D subfamily, the APOBEC3F subfamily, the APOBEC3G subfamily, the APOBEC3G subfamily, the APOBEC3H subfamily, or the APOBEC4 subfamily, or an alteration thereof, wherein preferably the altered cytosine deaminase comprises an altered APOBEC3A,
wherein preferably the altered cytidine deaminase comprises an amino acid substitution mutation at a position functionally equivalent to (Tyr/Phe)130 in a wild-type APOBEC3A protein and/or an amino acid substitution mutation at a position functionally equivalent to Tyr132 in a wild-type APOBEC3A protein, wherein preferably the altered cytidine deaminase comprises amino acid substitution mutations at positions functionally equivalent to (Tyr/Phe)130 and Tyr132 in a wild-type APOBEC3A protein,
wherein preferably the altered cytidine deaminase comprises an amino acid substitution mutation at a position functionally equivalent to (Tyr/Phe)130 in a wild-type APOBEC3A protein, wherein the substitution mutation is (Tyr/Phe)130Trp, and wherein preferably, the (Tyr/Phe)130 is Tyr130, and the wild-type APOBEC3A protein is SEQ ID NO: 12.

6. The method of claim 5, wherein the substitution mutation at the position functionally equivalent to Tyr130 comprises a mutation to alanine, glycine, phenylalanine, histidine, glutamine, methionine, asparagine, lysine, valine, aspartic acid, glutamic acid, serine, cysteine, proline, arginine, or threonine,
wherein preferably the substitution mutation at the position functionally equivalent to Tyr130 comprises a mutation to Ala, Val, or Trp,
wherein preferably the substitution mutation at the position functionally equivalent to Tyr132 comprises a mutation to His, Arg, Gln, or Lys, and
wherein preferably the altered cytidine deaminase converts 5-methyl cytosine (5mC) to thymidine (T) by deamination at a greater rate than conversion of cytosine (C) to uracil (U) by deamination.

7. The method of claim 6, wherein the rate is at least 100-fold greater, wherein preferably the altered cytidine deaminase converts cytosine (C) to uracil (U) by deamination and 5-methyl cytosine (5mC) to thymidine (T) by deamination at a greater rate than conversion of 5-hydroxymethyl cytosine (5hmC) to 5-hydroxymethyl uracil (5hmU) by deamination, and wherein preferably conversion of 5hmC to 5hmU by deamination is undetectable.

8. The method of any one of claims 5 to 7, wherein the altered cytidine deaminase comprises a ZDD motif H- [P/A/V]-E-X[23-28]-P-C-X[2-4]-C (SEQ ID NO: 1).

9. The method of any one of claims 5 to 8, wherein the altered cytidine deaminase is a member of the APOBEC3A subfamily and comprises a ZDD motif HXEX24SW(S/T)PCX[2-4]CX6FX8LX5R(L/I)YX[8-11]LX2LX[10]M (SEQ ID NO: 2), wherein the amino acid substitution mutation at the position functionally equivalent to (Tyr/Phe)130 of the wild-type APOBEC3A protein is the Tyr (Y) amino acid of the ZDD motif.

10. The method of any one of claims 5 to 9, wherein the altered cytidine deaminase is a member of the APOBEC3A subfamily and comprises X[16-26]-GRXXTXLCYXV-X15-GXXXN-X12-HAEXXF-X14-YXXTWXXSWSPC- X[2-4]-CA-X5-FL-X7-LXIXXXR(L/I)Y-X8-GLXXLXXXG-XS-M-X4-FXXCWXXFV-X6-FXPW-X13-LXXI- X[2-6] (SEQ ID NO: 3).

11. The method of any one of claims 5 to 10, wherein the altered cytidine deaminase is a member of the APOBEC3A family and comprises SEQ ID NO: 5, SEQ ID NO: 6, SEQ ID NO: 7, SEQ ID NO: 8, SEQ ID NO: 9, SEQ ID NO: 10, or SEQ ID NO: 11.

12. The method of any one of claims 1 to 11, wherein the reactive cytosine nucleobase analog comprises a hydroxylamine-cytosine derivative, a hydrazine-cytosine derivative, or a hydrazide-cytosine derivative.

13. The method of any one of claims 1 to 12, wherein amplification comprises a standard PCR polymerase or a U-intolerant polymerase.

14. A kit comprising:
a cytosine deaminase;
an uracil DNA glycosylase (UDG); and
a reactive cytosine nucleobase analog.

15. The kit of claim 14, wherein the cytosine deaminase is an altered APOBEC.

## Patentansprüche

1. Verfahren zur Vermeidung falsch positiver Nachweise von 5-Methylcytosin (5mC) und/oder 5-Hydroxymethylcytosin (5hmC) aufgrund einer Desaminierung unmethylierter Cytosine in einem Test unter Verwendung einer Cytosin-Desaminase zur selektiven Desaminierung methylierter Cytosine, wobei das Verfahren Folgendes umfasst:
Bereitstellen einer Probe, die einzelsträngige DNA-Bibliotheksfragmente umfasst, in denen eine Cytosin-Deaminase methylierte Cytosine desaminiert hat;
Kontaktieren der Probe mit einer Uracil-DNA-Glycosylase (UDG) zur Deglycosylierung von Uracilresten, um abasische Stellen, die eine Halbacetalbildung aufweisen, innerhalb der einzelsträngigen DNA-Bibliotheksfragmente zu erzeugen; und
Kontaktieren der Probe mit einem reaktiven Cytosin-Nukleobasen-Analogon, um über eine nichtkanonische Verknüpfung ein Cytosin an abasischen Stellen einzuführen, was zu doppelsträngigen DNA-korrigierten Bibliothekfragmenten führt.

2. Verfahren nach Anspruch 1, weiter umfassend Unterziehen der Probe einer Polymerase-Kettenreaktion (PCR) zur Amplifikation.

3. Verfahren nach Anspruch 1 oder 2, wobei die DNA-Bibliotheksfragmente eine Länge von etwa 100 bp bis etwa 200 bp aufweisen und wobei die DNA-Bibliotheksfragmente vorzugsweise 5'- und/oder 3'-Adaptersequenzen umfassen.

4. Verfahren nach einem der Ansprüche 1 bis 3, das weiter die Sequenzierung der korrigierten Bibliothekfragmente umfasst.

5. Verfahren nach einem der Ansprüche 1 bis 4, wobei die Cytosin-Deaminase eine veränderte Cytosin-Deaminase umfasst,
wobei die veränderte Cytosin-Deaminase vorzugsweise ein Mitglied der AID-Unterfamilie, der APOBEC1-Unterfamilie, der APOBEC2-Unterfamilie, der APOBEC3A-Unterfamilie, der APOBEC3B-Unterfamilie, der APOBEC3C-Unterfamilie, der APOBEC3D-Unterfamilie, der APOBEC3F-Unterfamilie, der APOBEC3G-Unterfamilie, der APOBEC3H-Unterfamilie oder der APOBEC4-Unterfamilie oder eine Abwandlung davon ist, wobei die veränderte Cytosin-Deaminase vorzugsweise eine veränderte APOBEC3A umfasst,
wobei die veränderte Cytidin-Deaminase vorzugsweise eine Aminosäuresubstitutionsmutation an einer Position umfasst, die funktionell äquivalent ist zu (Tyr/Phe)130 in einem Wildtyp-APOBEC3A-Protein und/oder eine Aminosäuresubstitutionsmutation an einer Position, die funktionell äquivalent ist zu Tyr132 in einem Wildtyp-APOBEC3A-Protein, wobei die veränderte Cytidin-Deaminase vorzugsweise Aminosäuresubstitutionsmutationen an Positionen umfasst, die funktionell äquivalent sind zu (Tyr/Phe)130 und Tyr132 in einem APOBEC3A-Wildtypprotein,
wobei die veränderte Cytidin-Deaminase vorzugsweise eine Aminosäuresubstitutionsmutation an einer Position umfasst, die funktionell äquivalent ist zu (Tyr/Phe)130 in einem Wildtyp-APOBEC3A-Protein, wobei die Substitutionsmutation (Tyr/Phe)130Trp ist, und wobei (Tyr/Phe)130 vorzugsweise Tyr130 ist und das Wildtyp-APOBEC3A-Protein SEQ ID NO: 12 ist.

6. Verfahren nach Anspruch 5, wobei die Substitutionsmutation an der Position, die funktionell äquivalent ist zu Tyr130, eine Mutation zu Alanin, Glycin, Phenylalanin, Histidin, Glutamin, Methionin, Asparagin, Lysin, Valin, Asparaginsäure, Glutaminsäure, Serin, Cystein, Prolin, Arginin oder Threonin umfasst,
wobei die Substitutionsmutation an der Position, die funktionell äquivalent ist zu Tyr130, vorzugsweise eine Mutation zu Ala, Val oder Trp umfasst,
wobei die Substitutionsmutation an der Position, die funktionell äquivalent ist zu Tyr132, vorzugsweise eine Mutation zu His, Arg, Gin oder Lys umfasst, und
wobei die veränderte Cytidin-Deaminase vorzugsweise 5-Methylcytosin (5mC) durch Desaminierung mit einer höheren Reaktionsgeschwindigkeit in Thymidin (T) umwandelt als Cytosin (C) durch Desaminierung in Uracil (U) umgewandelt wird.

7. Verfahren nach Anspruch 6, wobei die Reaktionsgeschwindigkeit mindestens 100-fach höher ist, wobei die veränderte Cytidin-Deaminase Cytosin (C) vorzugsweise durch Desaminierung in Uracil (U) und 5-Methylcytosin (5mC) durch Desaminierung in Thymidin (T) mit einer höheren Reaktionsgeschwindigkeit umwandelt als 5-Hydroxymethylcytosin (5hmC) durch Desaminierung in 5-Hydroxymethyluracil (ShmU) umgewandelt wird, und wobei die Umwandlung von 5hmC in 5hmU durch Desaminierung vorzugsweise nicht nachweisbar ist.

8. Verfahren nach einem der Ansprüche 5 bis 7, wobei die veränderte Cytidin-Deaminase ein ZDD-Motiv H-[P/A/V]-E-X[23-28]-P-C-X[2-4]-C (SEQ ID NO: 1) umfasst.

9. Verfahren nach einem der Ansprüche 5 bis 8, wobei die veränderte Cytidin-Deaminase ein Mitglied der APOBEC3A-Unterfamilie ist und ein ZDD-Motiv HXEX24SW(S/T)PCX[2-4]CX6FX8LX5R(L/I)YX[8-11]LX2LX[10]M (SEQ ID NO: 2) umfasst, wobei die Aminosäuresubstitutionsmutation an der Position, die funktionell äquivalent ist zu (Tyr/Phe)130 des Wildtyp-APOBEC3A-Proteins, die Aminosäure Tyr (Y) des ZDD-Motivs ist.

10. Verfahren nach einem der Ansprüche 5 bis 9, wobei die veränderte Cytidin-Deaminase ein Mitglied der APOBEC3A-Unterfamilie ist und X[16-26]-GRXXTXLCYXV-X15-GXXXN-X12-HAEXXF-X14-YXXTWXXSWSPC- X[2-4]-CA-X5-FL-X7-LXIXXXR(L/I)Y- X8-GLXXLXXXG-X5-M-X4-FXXCWXXFV-X6-FXPW-X13-LXXI- X[2-6] (SEQ ID NO: 3) umfasst.

11. Verfahren nach einem der Ansprüche 5 bis 10, wobei die veränderte Cytidin-Deaminase ein Mitglied der APOBEC3A-Familie ist und SEQ ID NO: 5, SEQ ID NO: 6, SEQ ID NO: 7, SEQ ID NO: 8, SEQ ID NO: 9, SEQ ID NO: 10 oder SEQ ID NO: 11 umfasst.

12. Verfahren nach einem der Ansprüche 1 bis 11, wobei das reaktive Cytosin-Nukleobasen-Analogon ein Hydroxylamin-Cytosin-Derivat, ein Hydrazin-Cytosin-Derivat oder ein Hydrazid-Cytosin-Derivat umfasst.

13. Verfahren nach einem der Ansprüche 1 bis 12, wobei die Amplifikation eine Standard-PCR-Polymerase oder eine U-intolerante Polymerase umfasst.

14. Kit, umfassend:
eine Cytosin-Deaminase;
eine Uracil-DNA-Glycosylase (UDG); und
ein reaktives Cytosin-Nukleobasen-Analogon.

15. Kit nach Anspruch 14, wobei es sich bei der Cytosin-Deaminase um eine veränderte APOBEC handelt.

## Revendications

1. Procédé de prévention de faux positifs dans la détection de la 5-méthylcytosine (5mC) et/ou de la 5-hydroxyméthylcytosine (5hmC) due à la désamination des cytosines non méthylées dans un dosage utilisant une cytosine désaminase pour désaminer sélectivement les cytosines méthylées, le procédé comprenant :
la fourniture d'un échantillon comprenant des fragments de banque d'ADN simple brin dans lesquels une cytosine désaminase a désaminé des cytosines méthylées ;
la mise en contact de l'échantillon avec un uracile ADN glycosylase (UDG) pour déglycosyler les résidus d'uracile afin de former des sites abasiques présentant une formation d'hémiacétal au sein des fragments de banque d'ADN simple brin ; et
la mise en contact de l'échantillon avec un analogue de cytosine nucléobase réactif pour installer une cytosine sur des sites abasiques via une liaison non canonique, ce qui donne des fragments de banque d'ADN double brin corrigés.

2. Procédé selon la revendication 1, comprenant en outre la soumission de l'échantillon à une amplification par réaction en chaîne par polymérase (PCR).

3. Procédé selon la revendication 1 ou 2, dans lequel les fragments de banque d'ADN présentent une longueur d'environ 100 pb à environ 200 pb, et dans lequel, de préférence, les fragments de banque d'ADN comprennent des séquences d'adaptateurs 5' et/ou 3'.

4. Procédé selon l'une quelconque des revendications 1 à 3, comprenant en outre le séquençage des fragments de banque corrigés.

5. Procédé selon l'une quelconque des revendications 1 à 4, dans lequel la cytosine désaminase comprend une cytosine désaminase modifiée,
dans lequel, de préférence, la cytosine désaminase modifiée est un membre de la sous-famille AID, de la sous-famille APOBEC1, de la sous-famille APOBEC2, de la sous-famille APOBEC3A, de la sous-famille APOBEC3B, de la sous-famille APOBEC3C, de la sous-famille APOBEC3D, de la sous-famille APOBEC3F, de la sous-famille APOBEC3G, de la sous-famille APOBEC3H ou de la sous-famille APOBEC4, ou une modification de celles-ci, dans lequel de préférence la cytosine désaminase modifiée comprend une APOBEC3A modifiée,
dans lequel de préférence la cytidine désaminase modifiée comprend une mutation de substitution d'acide aminé à une position fonctionnellement équivalente à (Tyr/Phe)130 dans une protéine APOBEC3A de type sauvage et/ou une mutation de substitution d'acide aminé à une position fonctionnellement équivalente à Tyr132 dans une protéine APOBEC3A de type sauvage, dans lequel de préférence la cytidine désaminase modifiée comprend des mutations de substitution d'acide aminé à des positions fonctionnellement équivalentes à (Tyr/Phe)130 et Tyr132 dans une protéine APOBEC3A de type sauvage,
dans lequel de préférence la cytidine désaminase modifiée comprend une mutation de substitution d'acide aminé à une position fonctionnellement équivalente à (Tyr/Phe)130 dans une protéine APOBEC3A de type sauvage, dans lequel la mutation de substitution est (Tyr/Phe)130Trp, et dans lequel de préférence, le (Tyr/Phe)130 est Tyr130, et la protéine APOBEC3A de type sauvage est SEQ ID NO: 12.

6. Procédé selon la revendication 5, dans lequel la mutation de substitution à la position fonctionnellement équivalente à Tyr130 comprend une mutation en alanine, glycine, phénylalanine, histidine, glutamine, méthionine, asparagine, lysine, valine, acide aspartique, acide glutamique, sérine, cystéine, proline, arginine ou thréonine,
dans lequel de préférence la mutation de substitution à la position fonctionnellement équivalente à Tyr130 comprend une mutation en Ala, Val ou Trp,
dans lequel, de préférence, la mutation de substitution à la position fonctionnellement équivalente à Tyr132 comprend une mutation en His, Arg, Gin ou Lys, et
dans lequel de préférence la cytidine désaminase modifiée convertit la 5-méthyl cytosine (5mC) en thymidine (T) par désamination à un taux supérieur à celui de la conversion de la cytosine (C) en uracile (U) par désamination.

7. Procédé selon la revendication 6, dans lequel le taux est au moins 100 fois supérieur, dans lequel de préférence la cytidine désaminase modifiée convertit la cytosine (C) en uracile (U) par désamination et la 5-méthylcytosine (5mC) en thymidine (T) par désamination à un taux supérieur à celui de la conversion de la 5-hydroxyméthylcytosine (5hmC) en 5-hydroxyméthyluracile (5hmU) par désamination, et dans lequel de préférence la conversion de la 5hmC en ShmU par désamination est indétectable.

8. Procédé selon l'une quelconque des revendications 5 à 7, dans lequel la cytidine désaminase modifiée comprend un motif ZDD H- [P/A/V]-E-X[23-28]-P-C-X[2-4]-C (SEQ ID NO: 1).

9. Procédé selon l'une quelconque des revendications 5 à 8, dans lequel la cytidine désaminase modifiée est un membre de la sous-famille APOBEC3A et comprend un motif ZDD HXEX24SW(S/T)PCX[2-4]CX6FX8LX5R(L/I)YX[8-11]LX2LX[10]M (SEQ ID NO: 2), dans lequel la mutation de substitution d'acide aminé à la position fonctionnellement équivalente à (Tyr/Phe)130 de la protéine APOBEC3A de type sauvage est l'acide aminé Tyr (Y) du motif ZDD.

10. Procédé selon l'une quelconque des revendications 5 à 9, dans lequel la cytidine désaminase modifiée est un membre de la sous-famille APOBEC3A et comprend X[16-26]-GRXXTXLCYXV-X15-GXXXN-X12-HAEXXF-X14-YXXTWXXSWSPC- X[2-4]-CA-X5-FL-X7-LXIXXXR(L/I)Y-X8-GLXXLXXXG-X5-M-X4-FXXCWXXFV-X6-FXPW-X13-LXXI- X[2-6] (SEQ ID NO: 3).

11. Procédé selon l'une quelconque des revendications 5 à 10, dans lequel la cytidine désaminase modifiée est un membre de la famille APOBEC3A et comprend SEQ ID NO: 5, SEQ ID NO: 6, SEQ ID NO: 7, SEQ ID NO: 8, SEQ ID NO: 9, SEQ ID NO: 10 ou SEQ ID NO: 11.

12. Procédé selon l'une quelconque des revendications 1 à 11, dans lequel l'analogue de cytosine nucléobase réactif comprend un dérivé d'hydroxylamine-cytosine, un dérivé d'hydrazine-cytosine ou un dérivé d'hydrazide-cytosine.

13. Procédé selon l'une quelconque des revendications 1 à 12, dans lequel une amplification comprend une polymérase PCR standard ou une polymérase intolérante à U.

14. Kit, comprenant :
une cytosine désaminase ;
un uracile ADN glycosylase (UDG) ; et
un analogue de cytosine nucléobase réactif.

15. Kit selon la revendication 14, dans lequel la cytosine désaminase est une APOBEC modifiée.
